# EUROPEAN PATENT APPLICATION

(11) **EP 4 296 286 A1**
(43) Date of publication of application: **27.12.2023**
(21) Application number: 22755586.9
(22) Date of filing: 18.02.2022
(51) Int. Cl.: C07K 19/00, C07K 16/28, C07K 16/30

(54) **ANTI-GPRC5D×BCMA×CD3 TRISPECIFIC ANTIBODY AND USE THEREOF**

(30) Priority: 19.02.2021 CN 202110189359
(71) Applicant: Innovent Biologics (Suzhou) Co., Ltd., Suzhou, Jiangsu 215123 (CN)
(72) Inventor: XU, Wei, Suzhou, Jiangsu 215123 (CN); SHEN, Wanwan, Suzhou, Jiangsu 215123 (CN); LI, Li, Suzhou, Jiangsu 215123 (CN); WANG, Xuan, Suzhou, Jiangsu 215123 (CN); ZHU, Chenjuan, Suzhou, Jiangsu 215123 (CN)
(74) Representative: Sagittarius IP
(86) International application number: PCT/CN2022/076832
(87) International publication number: WO 2022/174813

(57) **Abstract**

The present invention relates to a trispecific antigen-binding protein, and more specifically to a trispecific antibody specifically binding to two tumor antigens of GPRC5D and BCMA and T cell surface antigen CD3, and a pharmaceutical composition comprising same, a preparation method and the use.

## Description

### TECHNICAL FIELD

The present invention relates to a trispecific antigen-binding protein, and more particularly to a trispecific antibody specifically binding to two tumor antigens of GPRC5D and BCMA and a T cell surface antigen CD3, a pharmaceutical composition comprising same, a preparation method therefor and use thereof.

### BACKGROUND

T cell bispecific antibodies (BsAbs) have been used for tumor treatment. Such antibodies can form synapses between cytotoxic T lymphocytes and tumor cells, thereby inducing tumor cell destruction. The BsAbs generally involve dual targeting against tumor-associated antigens (TAAs) and T-cell surface antigens (also known as T-cell engaging antigens (TEAs)). However, therapeutic strategies based on the BsAbs typically rely on the distribution of the tumor-associated antigens on the tumor cells to be treated. This leads to the selectivity of treatment for the patient population and the limitation of treatment. In addition, studies have also shown that therapeutic modalities targeting a single TAA site may cause the recurrence of the disease due to the escape mechanism of the tumor, thereby limiting the effectiveness of the treatment.

The development of trispecific and/or tetraspecific antibodies has been proposed to effectively recruit immune cells to tumor sites and improve the efficacy of the treatment. For this reason, in recent years, various multivalent and multispecific antibody formats have been described. However, the diversity of requirements for therapeutic functionality and therapeutic behavior of different therapeutic products has determined that there is no "optimal form" that can be applied to most different desired molecular combinations. Accordingly, in achieving multispecific properties, a variety of factors need to be considered, including, for example, the spatial distribution or size of the different target antigens, as well as the tumor-associated antigen expression density at the surface of the tumor cells. In many cases, the antibody format that is effective for a particular combination of target antigens must be identified by generating and comparing the functionality of the different antibody formats.

### SUMMARY

Since the expressions of GPRC5D and BCMA in tumors are irrelevant, the inventor proposes that a multispecific antibody for combined targeting of GPRC5D and BCMA can cover both patients with GPRC5D-positive tumors and patients with BCMA-positive tumors, and moreover, can avoid recurrence caused by loss of a single antigen, thereby achieving the purposes of improving the coverage rate of patients and improving the treatment effect.

To achieve this goal, the inventor proposes a trispecific antibody targeting GPRC5D/BCMA/CD3 and intensively studies various GPRC5D × BCMA × CD3 formats. By adjusting the combination mode and distance between two TAA antigens (GPRC5D and BCMA) and a T-cell surface antigen (CD3) and regulating the effect of a T-cell engager antibody on mediating the activation and killing effect of T cells, the inventor successfully achieves multi-target combination, reducing the requirement for combined use of multiple drugs. On this basis, heavy chain mispairing is further reduced using a Knob-in-Hole technology at Fc fragment; and the problem of light chain mispairing is solved by extracellular redox. The antibody of the present invention also has good preparability and good druggability.

Therefore, in one aspect, the present invention provides a trispecific Y-type antibody molecule having the above-described advantages, comprising an antigen-binding site that specifically binds to GPRC5D, BCMA, and CD3, and comprising two antibody arms, stems located at the C-termini of the antibody arms, and conjugate components, wherein the conjugate components are conjugated to the C-termini of the antibody arms and/or the stems.

In yet another aspect, the present invention provides an antibody molecule that specifically binds to GPRC5D, comprising a combination of CDR sequences selected from the followings: HCDR1, HCDR2, HCDR3, LCDR1, LCDR2, and LCDR3, which are set forth in SEQ ID NOs: 1-6, 7-12, 13-18, or 19-24 in the above order. The antibody molecule of the present invention binds to GPRC5D with high affinity and blocks GPRC5D-mediated signaling in the cell.

In yet another aspect, the present invention further provides a nucleic acid encoding the antibody molecule of the present invention, a vector, a host cell, and use of the antibody molecule of the present invention, particularly a method and use for treating GPRC5D-positive and/or BCMA-positive tumors such as multiple myeloma.

### BRIEF DESCRIPTION OF THE DRAWINGS

The preferred embodiments of the present invention described in detail below will be better understood when read in conjunction with the following drawings. For the purpose of illustrating the present invention, currently, preferred embodiments are shown in the drawings. However, it should be understood that the present invention is not limited to an accurate arrangement and means of the embodiments shown in the drawings.
FIG. 1 schematically shows structures of designed trispecific antibody molecules of Format_1, Format_2, Format_6, and Format_7.
FIG. 2 schematically shows a structure of the control bispecific antibody molecule used in the examples.
FIG. 3 shows that activation of T cells mediated by exemplary antibodies TS-F2-1 and TS-F2-2 is detected by a Jurkat-NFAT-Luc reporter system.
FIG. 4 shows that activation of T cells mediated by exemplary antibodies TS-F2-3 and TS-F6 is detected by a Jurkat-NFAT-Luc reporter system.
FIG. 5 shows that activation of T cells mediated by exemplary antibodies TS-F2-4 and TS-F2-5 is detected by a Jurkat-NFAT-Luc reporter system (A-D); and activation of T cells mediated by exemplary antibodies TS-F2-4, TS-F2-5, and TS-F2-6 is detected by a Jurkat-NFAT-Luc reporter system (E-F).
FIG. 6 shows that activation of T cells mediated by exemplary antibodies TS-F7-1 and TS-F7-2 is detected by a Jurkat-NFAT-Luc reporter system.
FIG. 7 shows that activation of T cells mediated by exemplary antibodies TS-F7-3 and TS-F7-4 is detected by a Jurkat-NFAT-Luc reporter system.
FIG. 8 shows the activation of CD4 + T cells in PBMCs mediated by exemplary antibodies.
FIG. 9 shows activation (B) of CD8 + T cells in PBMCs mediated by exemplary antibodies.
FIG. 10 shows the killing effects of PBMCs mediated by exemplary antibodies on H929 cells.
FIG. 11 shows the cytokine release amount accompanied by the killing process of PBMCs mediated by exemplary antibodies on NCl-H929 cells.
FIG. 12 shows the tumor inhibitory effect of exemplary antibodies in an H929 tumor-bearing humanized mouse model.
FIG. 13 shows expression levels of GPRC5D and BCMA on the surfaces of different multiple myeloma cells.

### DETAILED DESCRIPTION

Unless otherwise defined, all technical and scientific terms used herein have the same meaning as commonly understood by those of ordinary skill in the art to which the present invention belongs. All publications, patent applications, patents, and other references mentioned herein are incorporated by reference in their entireties. In addition, the materials, methods, and examples described herein are illustrative only and are not intended to be limiting. Other features, objectives, and advantages of the present invention will be apparent from the specification and drawings, and from the appended claims.

### I. Definitions

The term "about" used in combination with a numerical value is intended to encompass the numerical values in a range from a lower limit less than the specified numerical value by 5% to an upper limit greater than the specified numerical value by 5%.

As used herein, the term "comprise" or "include" is intended to mean that the elements, integers, or steps are included, but not to the exclusion of any other elements, integers, or steps.

The term "antibody" is used herein in the broadest sense, refers to a protein comprising an antigen-binding site, and encompasses natural and artificial antibodies with various structures, including but not limited to monoclonal antibodies, polyclonal antibodies, multispecific antibodies (e.g., bispecific antibodies), single-chain antibodies, intact antibodies, and antibody fragments.

The terms "whole antibody", "full-length antibody", "complete antibody" and "intact antibody" are used interchangeably herein to refer to a naturally occurring glycoprotein comprising at least two heavy (H) chains and two light (L) chains interconnected by disulfide bonds. Each heavy chain consists of a heavy chain variable region (abbreviated herein as VH) and a heavy chain constant region. Each heavy chain constant region consists of 3 domains CH1, CH2, and CH3. Each light chain consists of a light chain variable region (abbreviated herein as VL) and a light chain constant region. The light chain constant region consists of one domain CL. The VH region and the VL region can be further divided into hypervariable regions (complementarity-determining regions, or CDRs), with relatively conservative regions (framework regions, or FRs) inserted therebetween. Each VH or VL consists of three CDRs and four FRs, arranged from amino-terminus to carboxyl-terminus in the following order: FR1, CDR1, FR2, CDR2, FR3, CDR3, and FR4. The constant regions are not directly involved in the binding of antibodies to antigens, but exhibit a variety of effector functions.

The term "antigen-binding fragment" is a portion or segment of an intact or a complete antibody that has fewer amino acid residues than an intact or a complete antibody, which can bind to an antigen or compete with an intact antibody (i.e., an intact antibody from which the antigen-binding fragment is derived) for binding to an antigen. The antigen-binding fragment may be prepared by recombinant DNA techniques, or by enzymatic or chemical cleavage of an intact antibody. The antigen-binding fragments include, but are not limited to, Fab, Fab', F(ab')2, Fv, single-chain Fv, diabodies, and single-domain antibodies (sdAbs). The Fab fragment is a monovalent fragment consisting of VL, VH, CL, and CH1 domains and can be obtained, for example, by papain digestion of a complete antibody. In addition, the F(ab')2, a dimer of the Fab', is a bivalent antibody fragment produced by pepsin digestion of a portion below disulfide bonds in a hinge region of a complete antibody. The F(ab')2 can be reduced by disrupting the disulfide bonds in the hinge region under neutral conditions, and the F(ab')2 dimer is thus converted into Fab' monomers. The Fab' monomer is substantially a Fab fragment with a hinge region (for more detailed descriptions of the antibody fragment, see Fundamental Immunology, W. E. Paul (ed.), Raven Press, N.Y. (1993)). The Fv fragment consists of the VL and VH domains of a single arm of an antibody. In addition, although the two domains VL and VH of the Fv fragment are encoded by separate genes, using the recombinant method, the domains can be linked by a synthetic linker peptide capable of making these two domains produced as single protein chains in which the VL and VH regions are paired to form a single-chain Fv. The antibody fragment can be obtained by a chemical method, a recombinant DNA method, or a protease digestion method.

The terms "antigen-binding site" and "antigen-binding domain" as used herein are used interchangeably and refer to a region in an antibody molecule that actually binds to an antigen. Preferably, the antigen-binding site used in the antibody molecule of the present invention comprises a VH/VL pair consisting of a light chain variable domain (VL) and a heavy chain variable domain (VH) of the antibody, and the VH/VL pair may be contained in a single polypeptide chain or in two separate polypeptide chains. In a preferred embodiment, the antibody molecule of the present invention comprises at least one antigen-binding site that specifically binds to GPRC5D, at least one antigen-binding site that specifically binds to BCMA, and at least one antigen-binding site that specifically binds to CD3. In one embodiment, the antibody of the present invention is a trivalent trispecific antibody, a tetravalent trispecific antibody, or a hexavalent trispecific antibody.

As used herein, the term "monospecific antibody" refers to an antibody having one or more binding sites, each of which binds to the same epitope of the same antigen. As used herein, the term "multispecific antibody" refers to an antibody having at least two antigen-binding sites, each of which binds to a different epitope of the same antigen or a different epitope of a different antigen. The antibody provided herein is a trispecific antibody against GPRC5D, BCMA, and CD3.

The term "immunoglobulin molecule" refers to a protein having a structure of a naturally existing antibody. For example, an IgG is a heterotetrameric glycoprotein of about 150,000 Daltons consisting of two light chains and two heavy chains which are disulfide-bonded. Each immunoglobulin heavy chain has a heavy chain variable region (VH), also called a heavy chain variable domain, followed by three heavy chain constant domains (CH1, CH2, and CH3), from N-terminus to C-terminus. Similarly, each immunoglobulin light chain has a light chain variable region (VL), also called a light chain variable domain, followed by a light chain constant domain (CL) from N-terminus to C-terminus. The heavy chains of an immunoglobulin can be assigned to one of five classes, α (IgA), δ (IgD), ε (IgE), γ (IgG), or µ (IgM), in which some classes can be further divided into subclasses such as γ1 (IgG1), y2 (IgG2), y3 (IgG3), y4 (IgG4), α1 (IgA1), and α2 (IgA2). The light chains of an immunoglobulin can be divided into one of the two classes, κ or λ, based on the amino acid sequence of constant domains thereof. The IgG immunoglobulin essentially consists of two Fab molecules and two dimerized Fc regions linked by an immunoglobulin hinge region.

The term "variable region" or "variable domain" refers to a domain of a heavy chain or light chain of an antibody involved in the binding of the antibody to an antigen. Variable domains of heavy and light chains of natural antibodies generally have similar structures, wherein each domain comprises four conserved framework regions (FRs) and three complementarity-determining regions. In some cases, a single VH or VL domain can be sufficient to provide antigen-binding specificity.

"Complementarity-determining region" or "CDR region" or "CDR" or "highly variable region" is a region in an antibody variable domain that is highly variable in sequence, forms a structurally defined loop ("a hypervariable loop") and/or comprises antigen-contacting residues ("antigen-contacting sites"). CDRs are primarily responsible for binding to antigen epitopes. The CDRs of heavy and light chains are numbered sequentially from the N-terminus and are generally referred to as CDR1, CDR2, and CDR3. The CDRs located in a heavy chain variable domain of an antibody are also referred to as HCDR1, HCDR2, and HCDR3, whereas the CDRs located in a light chain variable domain of an antibody are referred to as LCDR1, LCDR2, and LCDR3. In a given amino acid sequence of a light chain variable region or heavy chain variable region, the CDR sequences may be determined using a variety of schemes well known in the art, e.g., Chothia based on the three-dimensional structure of the antibody and the topology of CDR loops, Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (the international ImMunoGeneTics information system, imgt.cines.fr/ on the World Wide Web), and North CDR definition based on the affinity propagation clustering using a large number of crystal structures (North et al., A New Clustering of Antibody CDR Loop Conformations, Journal of Molecular Biology, 406, 228-256(2011)).

For example, Kabat- and Chothia-numbered CDR regions have different definition ranges.

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L52 |
| LCDR3 (Kabat and Chothia numbering systems) | L89-L97 | L89-L97 | L91-L96 | L89-L96 | L89-L96 |
| HCDR1 (Kabat numbering system) | H31-H35B | H26-H35B | H26-H32 | H30-H35B | H26-H35B |
| HCDR1 (Chothia numbering system) | H31-H35 | H26-H35 | H26-H32 | H30-H35 | H26-H35 |
| HCDR2 (Kabat and Chothia numbering systems) | H50-H65 | H50-H58 | H53-H55 | H47-H58 | H51-H57 |
| HCDR3 (Kabat and Chothia numbering systems) | H95-H102 | H95-H102 | H96-H101 | H93-H101 | H93-H102 |

Unless otherwise stated, the term "CDR" or "CDR sequence" used herein encompasses CDR sequences determined by any one of the schemes above.

CDRs can also be determined based on having the same Kabat numbering positions as the reference CDR sequences. Unless otherwise stated, residue positions of an antibody variable region (including heavy chain variable region residues and light chain variable region residues) in the present invention are positions numbered according to the Kabat numbering system (Kabat et al., Sequences of Proteins of Immunological Interest, 5th Ed. Public Health Service, National Institutes of Health, Bethesda, Md. (1991)).

The term "Fc domain" or "Fc region" is used herein to define a C-terminus region of an immunoglobulin heavy chain, which comprises at least a portion of a constant region. The "Fc region" includes Fc regions of native sequences and variant Fc regions. A native immunoglobulin "Fc domain" comprises two or three constant domains, i.e., a CH2 domain, a CH3 domain, and an optional CH4 domain. For example, in native antibodies, an immunoglobulin Fc domain comprises the second and the third constant domains (CH2 domain and CH3 domain) derived from two heavy chains of IgG, IgA, and IgD antibodies; or comprises the second, the third, and the fourth constant domains (CH2 domain, CH3 domain, and CH4 domain) derived from two heavy chains of IgM and IgE antibodies. Unless otherwise stated herein, amino acid residues in the Fc region or heavy chain constant region are numbered according to the EU numbering system (also known as the EU Index) as described in, for example, Kabat et al., Sequences of Proteins of Immunological Interes, 5th Ed., Public Health Service, National Institutes of Health, Bethesda, MD, 1991.

The term "effector function" refers to bioactivities attributed to an immunoglobulin Fc region that vary with immunoglobulin isotype. Examples of immunoglobulin effector functions include: C1q binding and complement-dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake in antigen-presenting cells, down regulation of cell surface receptors (such as B-cell receptors), and B-cell activation.

The term "chimeric antibody" is an antibody molecule in which: (a) a constant region or a portion thereof is modified, substituted, or exchanged such that antigen-binding sites are linked to constant regions of different or modified classes, effector functions, and/or species, or disparate molecules imparting new properties (e.g., enzymes, toxins, hormones, growth factors, and drugs) to chimeric antibodies, etc.; or (b) a constant region or a portion thereof is modified, substituted, or exchanged by variable regions with different or modified antigen-binding specificities. For example, a mouse antibody can be modified by substituting its constant region with a constant region from a human immunoglobulin. Due to the substitution with a human constant region, the chimeric antibody can retain its specificity for recognizing antigens, while having reduced antigenicity in humans as compared to the original mouse antibody.

"Humanized antibody" is an antibody that retains the antigen-specific reactivity of a non-human antibody (such as a mouse monoclonal antibody) and has lower immunogenicity when administered to humans as a therapeutic agent. This can be achieved, for example, by retaining non-human antigen-binding sites and substituting the remainder of the antibodies with their human counterparts (i.e., the portions of the constant and variable regions not involved in binding are the corresponding parts of human antibodies). See, e.g., Padlan, Anatomy of the antibody specimen, Mol. Immun., 1994, 31:169-217. Other examples of human antibody engineering techniques include, but are not limited to, the Xoma technology disclosed in US 5,766,886.

As used herein, the term "binding" or "specific binding" means that the binding effect is selective for antigens and can be distinguished from unwanted or non-specific interactions. The ability of an antigen-binding site to bind to a particular antigen can be determined by an enzyme-linked immunosorbent assay (ELISA) or a conventional binding assay known in the art.

"Affinity" or "binding affinity" refers to the inherent binding affinity that reflects the interaction between members of a binding pair. The affinity of molecule X for its partner Y can be generally represented by a dissociation constant (KD), which is a ratio of the dissociation rate constant to the association rate constant (kdis and kon, respectively). Affinity can be measured by common methods known in the art. One specific method for measuring affinity is the ForteBio kinetic binding assay described herein.

The "percent identity (%)" of an amino acid sequence refers to the percentage of amino acid residues in a candidate sequence that are the same as those of a specific amino acid sequence shown in this specification when aligning the candidate sequence with the specific amino acid sequence shown in this specification, with gaps introduced if necessary to achieve maximum percent sequence identity and without considering any conservative replacements as part of sequence identity. In some embodiments, the present invention considers variants of the antibody molecule of the present invention that have a considerable degree of identity to the antibody molecule and sequence thereof specifically disclosed herein, and for example, the identity is at least 80%, 85%, 90%, 95%, 97%, 98%, 99% or higher. The variants may comprise conservative modifications.

For polypeptide sequences, "conservative modifications" include replacements of, deletions of, or additions to a polypeptide sequence that result in the replacement of a certain amino acid with a chemically similar amino acid. Conservative replacement tables providing functionally similar amino acids are well known in the art. Such conservatively modified variants are additional to polymorphic variants, interspecies homologs, and alleles of the present invention and do not exclude them. The following 8 groups comprise amino acids that are conservatively substituted with each other: 1) alanine (A) and glycine (G); 2) aspartic acid (D) and glutamic acid (E); 3) asparagine (N) and glutamine (Q); 4) arginine (R) and lysine (K); 5) isoleucine (I), leucine (L), methionine (M) and valine (V); 6) phenylalanine (F), tyrosine (Y), and tryptophan (W); 7) serine (S) and threonine (T); and 8) cysteine (C) and methionine (M) (see, for example, Creighton, Proteins (1984)). In some embodiments, the term "conservative sequence modification" is used to refer to an amino acid modification that does not significantly affect or alter the binding characteristics of an antibody comprising the amino acid sequence.

The term "host cell" refers to a cell into which an exogenous polynucleotide has been introduced, including the progeny of such cells. Host cells include "transformants" and "transformed cells", which include primary transformed cells and progeny derived therefrom. Host cells are any type of cell system that can be used to produce the antibody molecule of the present invention, including eukaryotic cells, e.g., mammalian cells, insect cells, and yeast cells; and prokaryotic cells, e.g., *E*. *coli* cells. Host cells include cultured cells, as well as cells within a transgenic animal, a transgenic plant, or cultured plant tissue or animal tissue.

The term "expression vector" refers to a vector comprising a recombinant polynucleotide, which comprises an expression control sequence operably linked to a nucleotide sequence to be expressed. Expression vectors contain sufficient cis-regulatory elements for expression, and other elements for expression can be provided by a host cell or in an *in vitro* expression system. Expression vectors include all those known in the art, including cosmids, plasmids (e.g., naked or contained in liposomes), and viruses (e.g., lentiviruses, retroviruses, adenoviruses, and adeno-associated viruses) incorporated into recombinant polynucleotides. The terms "individual" and "subject" can be used interchangeably and refer to a mammal. The mammals include, but are not limited to, domesticated animals (e.g., cows, sheep, cats, dogs, and horses), primates (e.g., human and non-human primates such as monkeys), rabbits and rodents (e.g., mice and rats). In particular, individuals are humans.

The term "treatment" refers to a clinical intervention intending to alter the natural progress of a disease in an individual being treated. Desired therapeutic effects include, but are not limited to, preventing the occurrence or recurrence of diseases, alleviating symptoms, reducing any direct or indirect pathological outcomes of diseases, preventing metastasis, delaying disease progression, improving or alleviating conditions, and alleviating or improving prognosis. In some embodiments, the antibody molecule of the present invention is used to delay the progression of a disease or to slow the progression of a disease.

The term "anti-tumor effect" or the tumor inhibitory effect refers to a biological effect that can be demonstrated by a variety of means, including but not limited to, for example, decrease in tumor volume, decrease in the number of tumor cells, decrease in tumor cell proliferation, or decrease in tumor cell viability. The terms "tumor" and "cancer" are used interchangeably herein and encompass solid and liquid tumors.

The term "GPRC5D" refers to a tumor-associated antigen, G protein-coupled receptor family C group 5 member D (e.g., human GPRC5D protein under UniProt accession No. Q9NZD1). Herein, "antigen-binding specificity against GPRC5D" refers to an antibody or an antibody fragment, such as scFv or Fab, that specifically binds to GPRC5D. In one embodiment, the antigen-binding site of the antibody molecule of the present invention that binds to GPRC5D may have high-affinity binding activity for GPRC5D-expressing cells, and have an EC50 value of, for example, 1-100 nM, such as 20-60 nM, as measured by flow cytometry. In one embodiment, the antigen-binding specificity is cross-reactive to human and monkey GPRC5D.

The term "BCMA" refers to a tumor-associated antigen, B-cell maturation antigen, also known as BCMA, TR17_HUMAN, and TNFRSF17 (e.g., human BCMA protein under UniProt accession No. Q02223). Herein, "antigen-binding specificity against BCMA" refers to an antibody or an antibody fragment, such as scFv or Fab, that specifically binds to BCMA. In one embodiment, the antigen-binding site of the antibody molecule of the present invention that binds to BCMA may have high-affinity binding activity for BCMA, and have a KD value of, for example, 0.1-10 nM, such as 0.1-5 nM, as measured by bio-layer interferometry. In one embodiment, the antigen-binding specificity is cross-reactive to human and monkey BCMA.

The term "CD3" refers to a T-cell engaging antigen, T-cell surface glycoprotein CD3 (e.g., human CD3 protein under UniProt accession No. P07766). Herein, "antigen-binding specificity against CD3" refers to an antibody or an antibody fragment, such as scFv or Fab, that specifically binds to CD3. In one embodiment, the antigen-binding site of the antibody molecule of the present invention that binds to CD3 may have high-affinity binding activity for a CD3 epsilon chain, and have a KD value of, for example, 1-50 nM, such as 0.1-5 nM, as measured by bio-layer interferometry. Preferably, in the trispecific antibody molecule of the present invention, a high-affinity CD3 antigen-binding site is used, e.g., with a KD value of less than 50 nM. In one embodiment, the antigen-binding specificity is cross-reactive to human and monkey CD3.

When describing the structure of the antibody of the present invention, the term "N-terminus" refers to the last amino acid at the N-terminus, and the term "C-terminus" refers to the last amino acid at the C-terminus. A "knob-in-hole" mutation is used herein to refer to the introduction of mutations in a first Fc polypeptide and a second Fc polypeptide, respectively, using the "knob-in-hole" technique to form a protuberance ("knob") and a complementary cavity ("hole") at the interface of the first Fc polypeptide and the interface of the second Fc polypeptide. It is known in the art that the "knob-in-hole" technique enables the engineering of the interface between different chains of an antibody molecule to promote the correct association of the chains of the antibody molecule. Generally, this technique involves introducing a "protuberance" at the interface of one chain, and introducing a corresponding "cavity" at the interface of the other chain to be paired with, such that the protuberance can be placed at the cavity. A preferred interface comprises the CH3 domain from the heavy chain constant domains of one chain and the CH3 domain from the heavy chain constant domains of the other chain to be paired with. The protuberance can be constructed by replacing small amino acid side chains at an interface of the CH3 domain from the heavy chain constant domains of one chain with large side chains, such as tyrosine or tryptophan. The compensating cavity of the same size as, or a similar size to, the protuberance is constructed at an interface of the CH3 domain from the heavy chain constant domains of the other chain to be paired with, by replacing large amino acid side chains with small side chains, such as alanine or threonine. Another optional interface comprises a light chain CL domain and a heavy chain CH1 domain of the Fab fragment described above, and the correct heterodimerization between the two chains of the Fab fragment is promoted by constructing a protuberance-cavity interaction. "Single chain variable fragment" or "scFv" is used herein to refer to a single-chain antibody fragment comprising a heavy chain variable domain VH and a light chain variable domain VL linked by a linker, wherein the VH and the VL are paired to form an antigen-binding site. The "disulfide-stabilized single chain variable fragment" or "dsscFv" is used herein to refer to a disulfide-stabilized single chain variable fragment, wherein the scFv fragment forms a disulfide bond linkage between the VH and VL domains by artificially introducing cysteine mutations into the VH and VL domains.

The "Fv fragment" is used herein to refer to an antibody fragment comprising a heavy chain variable domain VH and a light chain variable domain VL. The "disulfide stabilized variable fragment" or "dsFv" is used herein to refer to an Fv fragment stabilized by an artificially introduced disulfide bond between the VH and VL domains.

The "single-domain antibody" or "sdAb" is used herein to refer to an antibody fragment consisting of a single variable antibody domain, e.g., consisting of VH or VL, such as a heavy chain variable domain derived from a camelidae heavy chain antibody, and a VH-like single domain (v-NAR) derived from fish IgNAR. The single variable domain of the single-domain antibody does not require the interaction with other variable domains to recognize a target antigen. Examples of single-domain antibodies include single-domain antibodies derived from Camelidae (Llama and camel) and cartilaginous fishes (e.g., nurse sharks).

The "Fab fragment" or "Fab" is used herein to refer to an immunoglobulin fragment consisting of two polypeptide chains and comprising an immunoglobulin heavy chain variable domain VH, a heavy chain constant domain CH1, a light chain variable domain VL, and a light chain constant domain CL, wherein one polypeptide chain comprises, from N-terminus to C-terminus, a VH and one constant region selected from CH1 and CL, and the other polypeptide chain comprises, from N-terminus to C-terminus, a VL and the other constant region selected from CL and CH1, wherein the VH and VL domains are paired to form an antigen-binding site. As used herein, Fab is also referred to as crossFab when one polypeptide chain of Fab comprises VH linked to CL and the other polypeptide chain comprises VL linked to CH1.

As used herein, "single-chain Fab" or "scFab" is used to refer to a single-chain polypeptide formed by linking two chains of a Fab fragment via a linker.

In some embodiments of the present invention, the antibody of the present invention may comprise a Fab fragment selected from the followings: (i) Fab consisting of one chain comprising VH-CH1 and one chain comprising VL-CL; and (ii) a crossFab consisting of one chain comprising VH-CL and one chain comprising VL-CH1. In other embodiments of the present invention, the antibody of the present invention may comprise a single-chain Fab fragment selected from the followings: (i) single-chain Fab comprising VH-CH1-linker-VL-CL; and (iii) single-chain Fab comprising VH-CL-linker-VL-CH1.

As used herein, the immunoglobulin constant domain may be selected according to the expected function of the antibody molecule. For example, the constant domain may be an IgA, IgD, IgE, IgG, or IgM domain, particularly an immunoglobulin constant domain of human IgG, such as a constant domain of human IgG1, IgG2, IgG3, or IgG4, preferably a constant domain of human IgG1. As an example, a Fab fragment of the antibody may comprise CH and CL constant domains derived from IgG1. As yet another example, an Fc region of the antibody may comprise CH2 and CH3 domains derived from IgG1.

As used herein, the term "flexible linker peptide" or "linker peptide" or "linker" refers to a short amino acid sequence consisting of amino acids, such as glycine (G) and/or serine (S) and/or threonine residues (T) used alone or in combination, or a hinge region derived from immunoglobulin. In one embodiment, the linker peptide has a length of 5-50 amino acids, such as 10, 15, 20, 25, or 30 amino acids. In one embodiment, the linker peptide comprises amino acid sequence (G4S)n, wherein n is an integer equal to or greater than 1, and for example, n is an integer of 2, 3, 4, 5, 6, or 7. In one embodiment, the linker peptide comprises the amino acid sequence TS(G4S)n, wherein n is an integer equal to or greater than 1, and for example, n is an integer of 2, 3, 4, 5, 6, or 7. In yet another embodiment, the linker peptide is a hinge region derived from immunoglobulin, e.g., a hinge region amino acid sequence with "CPPC", such as an amino acid sequence "EPKSCDKTHTCPPCP" (SEQ ID NO: 114) or "EPKSSDKTHTCPPCP" (SEQ ID NO: 115). The linker peptide that may be used to link the domains of the antibody molecule of the present invention may also be, for example, but not limited to, the following amino acid sequences: GGG (SEQ ID NO: 116), DGGGS (SEQ ID NO: 117), TGEKP (SEQ ID NO: 118), GGRR (SEQ ID NO: 119), EGKSSGSGSESKVD (SEQ ID NO: 120), KESGSVSSEQLAQFRSLD (SEQ ID NO: 121), GGRRGGGS (SEQ ID NO: 122), LRQRDGERP (SEQ ID NO: 123), LRQKDGGGSERP (SEQ ID NO: 124), and GSTSGSGKPGSGEGSTKG (SEQ ID NO: 125). Alternatively, a computer program can be used to simulate three-dimensional structures of proteins and peptides, or a suitable flexible linker peptide is rationally designed by a phage display method.

### II. Trispecific antibody molecule of the present invention

The trispecific antibody molecule of the present invention is a Y-type antibody molecule. As used herein, the term "Y-type antibody molecule" refers to an antibody molecule of a Y-type structure comprising two antibody arms and a stem, wherein the two antibody arms form two branches of the Y-type structure and are each linked to the stem of the Y-type structure formed by the pairing of two antibody Fc domains. A typical Y-type antibody molecule is an immunoglobulin IgG molecule. The Y-type structure of the immunoglobulin IgG molecule consists of three regions: two antibody arms (Fab) and a stem (Fc), wherein hinge regions rich in elasticity are used to link the stem (Fc) to the antibody arms (Fab) of the antibody. In an IgG molecule, two arms can be used for specific binding of the antigen, whereas the stem determines the type and functional properties of the antibody. Herein, the Y-type antibody molecule also encompasses a molecule having the same or similar Y-type structure as the IgG molecule, such as a Y-type molecule with a conjugate component on the antibody arms and/or the stem of the antibody. Preferably, the antibody molecule of the present invention is a trispecific antibody molecule with a conjugate component.

In the Y-type antibody molecule of the present invention, the antibody arms may be composed of antigen-binding domains such as Fab, scFv, and sdAB, and the antigen-binding domains constituting the two antibody arms may target the same or different antigens and/or epitopes, with the same or different binding specificities. In the Y-type antibody molecule of the present invention, the stem of the antibody molecule may consist of two immunoglobulin Fc domains in pair, wherein the Fc domains may or may not each independently comprise a mutation. When the antibody molecule is an asymmetric IgG-like molecule, preferably the two Fc domains of the stem comprise mutations that facilitate their pairing and dimerization, such as a complementary "knob-in-hole" mutation. In addition, the Fc domain of the stem may also comprise other mutations, such as a cysteine mutation (to facilitate a disulfide bond linkage between the first Fc and the second Fc) and a charge pairing mutation; and/or a mutation that reduces or eliminates effector functions (e.g., a mutation that reduces or eliminates ADCC activity, such as an L234A/L235A mutation combination). The two antibody arms of the Y-type antibody molecule of the present invention may be covalently linked to each Fc domain of the stem, respectively, either directly or via a linker peptide, preferably the arms are linked to the Fc domains of the stem via the hinge regions of immunoglobulin.

In addition to the arms and the stem, the Y-type antibody molecule of the present invention also preferably comprises a conjugate component conjugated to the arms and/or the stem, the conjugate component comprising an additional antigen-binding domain, such as Fab, scFv, and sdAB, preferably scFv. The antigen-binding domain in the conjugate component may impart different antigen-binding specificities from those of the antibody arms. For example, the two antibody arms of the Y-type molecule may comprise antigen-binding domains providing first and second antigen-binding specificities, respectively, whereas the conjugate component of the Y-type molecule comprises an antigen-binding domain providing a third antigen-binding specificity, and the first, the second, and the third antigen-binding specificities are different from each other. Alternatively, both antibody arms of the Y-type molecule may comprise an antigen-binding domain providing a first antigen-binding specificity, whereas the conjugate component of the Y-type molecule comprises an antigen-binding domain providing second and third antigen-binding specificities, and the first, the second, and the third antigen-binding specificities are different from each other.

In the Y-type antibody molecule of the present invention, the first, the second, and the third antigen-binding specificities are different antigen-binding specificities directed against antigens selected from CD3, BCMA, and GPRC5D, respectively. For example, the first and the second antigen-binding specificities may be directed against different tumor-associated antigens (TAAs) selected from GPRC5D and BCMA, respectively; and the third antigen-binding specificity may be directed against T cell surface antigen (TEA) CD3. Alternatively, the first (or the second) and the third antigen-binding specificities may be directed against different tumor-associated antigens (TAAs) selected from GPRC5D and BCMA, respectively, whereas the second (or the first) antigen-binding specificity may be directed against a T cell surface antigen (TEA).

According to the structures of the arms, stem, and conjugate component, the Y-type antibody molecule of the present invention may comprise at least 2 polypeptide chains, such as 2 or 4 polypeptide chains. Herein, a polypeptide chain comprising an Fc domain of the stem is referred to as a heavy chain, and correspondingly, a polypeptide chain comprising no Fc domain is referred to as a light chain. Therefore, in some embodiments, the Y-type molecule of the present invention is a two-chain molecule comprising a first heavy chain polypeptide chain and a second heavy chain polypeptide chain, and in other embodiments, the Y-type molecule of the present invention is a four-chain molecule comprising two heavy chain polypeptide chains and two light chain polypeptide chains. In the Y-type molecule comprising a conjugate component of the present invention, the conjugate component may be conjugated to the heavy chain polypeptide chain of the antibody molecule (e.g. the C-terminus of the Fc domain of the stem) or may be conjugated to the light chain polypeptide chain of the antibody molecule (e.g. the C-terminus of the Fab light chain of the antibody arm that comprises the Fab antigen-binding site). Therefore, for example, the antibody of the present invention may comprise two heavy chain polypeptide chains and two light chain polypeptide chains, wherein a first heavy chain and a first light chain are paired to form one antibody arm of the antibody, a second heavy chain and a second light chain are paired to form the other antibody arm of the antibody, and an Fc domain of the first heavy chain and an Fc domain of the second heavy chain are paired to form the stem of the antibody, wherein the first heavy chain or the second heavy chain further comprises a single-chain antibody fragment (such as scFv) which forms an antibody conjugate component at the C-terminus, or the first light chain and the second light chain further comprise a single-chain antibody fragment (such as an scFv) which forms an antibody conjugate component at the C-terminus.

In one aspect, therefore, the present invention provides a trispecific Y-type antibody molecule. The Y-type antibody molecule of the present invention has a structure of the following formula:

(M1:M2-(X1)p)-Fc::Fc-(X2)q, (formula I)

wherein,
M1 and M2 represent a first antibody arm and a second antibody arm of the Y-type antibody molecule that comprise antigen-binding sites, respectively, and the antigen-binding sites of the first antibody arm and the second antibody arm may be the same or different;
Fc::Fc represents the stem of the Y-type antibody molecule that is located at the C-termini of the first and the second antibody arms, and the stem consists of a first Fc domain and a second Fc domain which are paired and dimerized;
X1 represents a conjugate component conjugated to the C-terminus of each antibody arm, and X2 represents a conjugate component conjugated to the C-terminus of the stem, wherein the conjugate component comprises an antigen-binding site and is conjugated to the antibody molecule either directly or via a linker peptide;
p and q each represent an integer of 0, 1, or 2, and p and q are not 0 simultaneously; and the antibody specifically binds to GPRC5D, BCMA, and CD3 via the antibody arms and the conjugate component.

Preferably, when the first and second antibody arms bind to different antigens, one of p and q is 0, wherein the antibody arms M1 and M2 provide the first and second antigen-binding specificities and the conjugate component provides the third antigen-binding specificity; or preferably, when the first and second antibody arms bind to the same antigen, neither p nor q is 0, wherein the antibody arms M1 and M2 provide the first antigen-binding specificity and the conjugate components X1 and X2 provide the second and the third antigen-binding specificities, respectively.

In some embodiments, the antibody arms (M1 and M2) of the antibody molecule comprise an antigen-binding site selected from Fab or scFab (preferably Fab) and one of p and q is 0, wherein the antibody arms M1 and M2 bind to a first antigen and a second antigen, respectively and the conjugate component (X1 or X2) binds to a third antigen, and the first, the second, and the third antigens are different from each other and are independently selected from GPRC5D, BCMA, and CD3, preferably, when p = 0, q = 1, and the conjugate component X2 is conjugated to one of the two Fc domains of the stem of the antibody molecule; or preferably, when q = 0, p = 2, the conjugate component X1 is conjugated to the Fab light chains of both antibody arms M1 and M2 of the antibody molecule.

In some embodiments, the antibody arms (M1 and M2) of the antibody molecule comprise antigen-binding sites selected from Fab or scFab (preferably Fab), and neither p nor q is 0, wherein the antibody arms M1 and M2 bind to the same first antigen, the conjugate component X1 and X2 bind to a second antigen and a third antigen, respectively, and the first, the second and, the third antigens are different from each other and are independently selected from GPRC5D, BCMA, and CD3,

Preferably, p = 2 and q = 2, wherein the conjugate component X1 is conjugated to the Fab light chains of both antibody arms M1 and M2 of the antibody molecule, and the conjugate component X2 is conjugated to the two Fc domains of the stem of the antibody molecule.

In still other embodiments, the antibody arms (M1 and M2) of the antibody molecule comprise antigen-binding sites selected from scFv (preferably dsscFv), and p = 0, q =1 or 2, wherein the antibody arms M1 and M2 bind to a first antigen and a second antigen, respectively, the conjugate component X2 binds to a third antigen, and the first, the second, and the third antigens are different from each other and are independently selected from GPRC5D, BCMA, and CD3, preferably, when p = 0, q = 1, the conjugate component X2 is conjugated to the C-terminus of one of the two Fc domains of the stem of the antibody molecule.

In any one of the above-mentioned embodiments, preferably, the conjugate component comprises an antigen-binding site selected from scFv, dsFv, and dsAb, preferably scFv, more preferably dsscFv.

In any one of the above-mentioned embodiments, preferably, the first Fc domain and the second Fc domain comprise hinge regions having "CPPC" amino acid residues, and therefore the antibody molecule of the present invention forms an interchain disulfide bond between the first and the second Fc domains to facilitate proper pairing of the polypeptide chains of the antibody molecule of the present invention. Preferably, in the antibody molecule of the present invention, the antibody arms are fused at the N-termini of the Fc regions via the hinge regions of the Fc regions.

In any one of the above-mentioned embodiments, preferably the conjugate component is conjugated to the stem and/or arms of the antibody molecule via a flexible linker peptide. Preferably, the linker peptide has a length of 5-15 amino acids, such as 10, 12, or 15 amino acids. Preferably, the linker peptide comprises an amino acid sequence TS(G4S)n or (G4S)n, wherein n = 1, 2, or 3, preferably n = 2.

Therefore, in some preferred embodiments, when one of p and q of formula I is 0, the present invention provides an IgG-like antibody molecule having a structure of the following formula:

(M1:M2-(X1)p)-Fc::Fc-(X2)q, (formula I)

wherein,
M1 and M2 comprise Fab or scFab, preferably Fab, that binds to a first antigen and a second antigen, respectively,
   wherein when p = 0, X2 comprises scFv that binds to a third antigen, or
   when q =0, X1 comprises scFv that binds to a third antigen, and
the first, the second, and the third antigens are different from each other and are independently selected from GPRC5D, BCMA, and CD3.

In still other preferred embodiments, when p = 0 of formula I, the present invention provides an IgG-like antibody molecule having a structure of the following formula:

(M1:M2)-Fc::Fc-(X2)q, (Format_2)

wherein,
q = 1,
M1 and M2 comprise Fab or scFab, preferably Fab, that binds to a first antigen and a second antigen, respectively, and
X2 comprises scFv that binds to a third antigen,
the first, the second, and the third antigens are different from each other and are independently selected from GPRC5D, BCMA, and CD3.

In still other preferred embodiments, when q = 0 of formula I, the present invention provides an IgG-like antibody molecule having a structure of the following formula:

(M1:M2-(X1)p)-Fc::Fc, (Format_7)

wherein,
P = 2,
M1 and M2 comprise Fab that binds to a first antigen and a second antigen, respectively, and
X2 comprises scFv that binds to a third antigen, the first, the second, and the third antigens are different from each other and are independently selected from GPRC5D, BCMA, and CD3.

In still other embodiments, when p = 2 and q = 2 of formula I, the present invention provides an IgG-like antibody molecule having a structure of the following formula:

(M1:M2-(X1)p)-Fc::Fc-(X2)q, (Format_6)

wherein,
M1 and M2 comprise Fab or scFab, preferably Fab, that binds to the same first antigen, and X1 and X2 comprise scFv that binds to a second antigen and a third antigen, respectively,
the first, the second, and the third antigens are different from each other and are independently selected from GPRC5D, BCMA, and CD3.

In still other embodiments, when p = 0 and q = 1 of formula I, the present invention provides an IgG-like antibody molecule having a structure of the following formula:

(M1:M2)-Fc::Fc-X2, (Format_1)

wherein,
M1, M2, and X2 comprise scFv that binds to first, second, and third antigens, respectively,
the first, the second, and the third antigens are different from each other and are independently selected from GPRC5D, BCMA, and CD3.

The antibody arms, conjugate components, and stem, that constitute the trispecific antibody molecule of the present invention, are described in further detail below, respectively. It will be understood by those of skill in the art that these descriptions will be used for any trispecific antibody molecule of the present invention described above, such as a molecule of formula I, and molecules having the structures of Format_1, 2, 6, and 7.

Antibody arms and conjugate components of the antibody molecule of the present invention

The antibody molecule of the present invention provides antigen-binding sites that specifically bind to the first, the second, and the third antigens via the antigen-binding sites of the antibody arms and conjugate components, wherein the first, the second, and the third antigens are different from each other and independently selected from tumor-associated antigen GPRC5D, tumor-associated antigen BCMA, and T cell engagement antigen CD3.

In one embodiment, the present invention provides an antibody molecule of formula I, wherein when p = 0 or q = 0, the first and the second antibody arms of the antibody provide the first and the second antigen-binding sites, respectively, and the conjugate components provide a third antigen-binding site.

In another embodiment, the present invention provides an antibody molecule of formula I, wherein when neither p nor q is 0, the first and the second antibody arms of the antibody provide a first antigen-binding site, the conjugate components conjugated to the C-termini of the antibody arms provide a second antigen-binding site, and the conjugate component conjugated to the stem of the antibody provides a third antigen-binding site.

The antigen-binding sites that can be used for the trispecific antibody molecule of the present invention (including the antigen-binding sites that specifically bind to GPRC5D, BCMA, and CD3) may be any antibody or antibody fragment that can bind to a target antigen. For example, in the antibody molecule of formula I of the present invention, the antigen-binding sites in the antibody arms and the conjugate components may independently be scFv, dsFv, Fab, scFab, or sdAb. Preferably, in some embodiments, the antigen-binding sites of the antibody arms are selected from Fab and scFab, preferably Fab. Preferably, in other embodiments, the antigen-binding sites of the conjugate components are selected from scFv, dsFv, and dsAb, preferably scFv, and more preferably disulfide-stabilized scFv (i.e., dsscFv). In some more preferred embodiments, the antigen-binding sites of the antibody arms are Fab, and the antigen-binding sites of the conjugate components are scFv, particularly dsscFv.

Herein, Fab_GPRC5D, Fab_BCMA, and Fab_CD3 are used to represent antigen-binding sites in Fab forms that bind to GPRC5D, BCMA, and CD3, respectively. Herein, ScFv_GPRC5D, ScFv_BCMA, and ScFv_CD3 are used to represent antigen-binding sites in scFv formats that bind to GPRC5D, BCMA, and CD3, respectively.

In some embodiments, the Fab antigen-binding site contained in the antibody molecule of the present invention consists of two polypeptide chains comprising VH, CH1, VL, and CL domains of immunoglobulin, wherein the VH and the VL are paired and the CH1 and the CL are paired to form the antigen-binding sites. In some embodiments, in the Fab, one chain comprises, from N-terminus to C-terminus, VH and CH1 (i.e., VH-CH1), and the other chain comprises, from N-terminus to C-terminus, VL and CL (i.e., VL-CL). In other embodiments, in Fab, one chain comprises, from N-terminus to C-terminus, VH and CL (i.e., VH-CL), and the other chain comprises, from N-terminus to C-terminus, VL and CH1 (i.e., VL-CH1). In some embodiments, the Fab constitutes the antigen-binding sites of the antibody arms of the antibody molecule of the present invention. In the embodiments, the Fab may be fused to the N-terminus of the Fc domain of the stem of the antibody stem via the C-terminus of the chain comprising VH, or the Fab is fused to the N-terminus of the Fc domain of the stem of the antibody via the C-terminus of the chain comprising VL. Preferably, the Fab comprises a VH-CH1 chain and a VL-CL chain and binds to the Fc region of the stem of the antibody via the C-terminus of the VH-CH1 chain. Preferably, when the antibody molecule of the present invention comprises a conjugate component conjugated to the antibody arm, the conjugate component is conjugated to the C-terminus of the Fab chain that is not linked to the stem of the antibody. Herein, the Fab chain that is not linked to the stem of the antibody is also referred to as the light chain of the Fab. Therefore, in some embodiments, the antibody molecule of the present invention has conjugate components conjugated to the Fab light chains of the antibody arms.

In other embodiments, the scFab antigen-binding site contained in the antibody molecule of the present invention consists of one polypeptide chain comprising VH, CH1, VL, and CL domains of immunoglobulin, wherein the VH and the VL are paired and the CH1 and the CL are paired to form the antigen-binding sites. In some embodiments, the scFab comprises: VH-CH1, linker, and VL-CL, from N-terminus to C-terminus. In other embodiments, the scFab comprises: VL-CL, linker, and VH-CH1, from N-terminus to C-terminus. In yet other embodiments, the scFab comprises: VH-CL, linker, and VL-CH1, from N-terminus to C-terminus. In other embodiments, the scFab comprises: VL-CH1, linker, and VH-CL, from N-terminus to C-terminus. In some embodiments, scFab constitutes the antigen-binding sites of the antibody arms of the antibody molecule of the present invention. In some embodiments, the scFab is fused to the N-terminus of the Fc domain of the stem of the antibody via the C-terminus of a polypeptide chain thereof. Preferably, the scFab comprises: VL-CL, linker, and VH-CH1, from N-terminus to C-terminus, and binds to the Fc region of the stem of the antibody via the C-terminus of the CH1 domain. Preferably, when the antibody molecule of the present invention comprises a scFab antibody arm, the conjugate component of the antibody molecule is conjugated only to the C-terminus of the Fc domain of the stem of the antibody.

In some embodiments, the Fab or scFab contained in the antibody molecule of the present invention comprises a CH1 domain from IgG immunoglobulin, such as a CH1 domain of IgG1, preferably a CH1 domain of human IgG1. In some preferred embodiments, the CH1 domain comprises an amino acid sequence of SEQ ID NO: 104 or an amino acid sequence having at least 90%, 95%, 97%, 98%, or 99% sequence identity thereto. In some embodiments, the Fab or scFab contained in the antibody molecule of the present invention comprises a kappa light chain constant domain from IgG immunoglobulin, such as a CLκ domain of IgG1, preferably a CLκ domain of human IgG1. For example, the Fab or scFab comprises a kappa light chain constant domain having an amino acid sequence of SEQ ID NO: 105 or an amino acid sequence having at least 90%, 95%, 97%, 98%, or 99% sequence identity thereto. In other embodiments, the Fab or scFab contained in the antibody molecule of the present invention comprises a lamda light chain constant region from IgG immunoglobulin, such as a CL_{λ} domain of IgG1, preferably a CL_{λ} lambda domain of human IgG1. For example, the Fab comprises a lamda light chain constant domain having an amino acid sequence of SEQ ID NO: 106 or an amino acid sequence having at least 90%, 95%, 97%, 98%, or 99% sequence identity thereto. In some embodiments, when the antibody arms M1 and M2 of the antibody molecule of the present invention comprise Fab or scFab (preferably Fab) that bind to different antigens, the light chain constant domains of the Fab or scFab of the antibody arms M1 and M2 are different from each other. For example, in some embodiments, the Fab of the antibody arm M1 comprises a kappa light chain constant domain and the Fab of the antibody arm M2 comprises a lamda light chain variable domain; or the Fab of the antibody arm M1 comprises a lamda light chain constant domain and the Fab of the antibody arm M2 comprises a kappa light chain variable domain.

In some embodiments, the scFv antigen-binding site contained in the antibody molecule of the present invention consists of one polypeptide chain comprising VH and VL domains of immunoglobulin, wherein the VH and the VL are linked via a linker to be paired to form the antigen-binding site. In some embodiments, the scFv is a trans-configuration comprising: VH, linker, and VL (VH-linker-VL) from N-terminus to C-terminus. In other embodiments, the scFv is a cis-configuration comprising: VL, linker, and VH (VH-linker-VL) from N-terminus to C-terminus. In some embodiments, the linker is a peptide linker consisting of amino acid residues. Suitable peptide linkers are known to those skilled in the art. In one embodiment, the linker has a length of 10-50 amino acids, such as 5-30 amino acids, for example, 15 amino acids or 20 amino acids. In one embodiment, the linker comprises amino acid sequence (G4S) n, wherein n = 1, 2, 3, 4, or 5, preferably n = 3 or 4, more preferably n = 4.

In some embodiments, the scFv constitutes the antigen-binding site of the antibody arm of the antibody molecule of the present invention and is fused to the N-terminus of the Fc domain of the stem of the antibody via the C-terminus of a polypeptide chain thereof.

In other embodiments, the scFv constitutes the antigen-binding site of the conjugate component of the antibody molecule of the present invention. In some embodiments, the scFv is fused to the C-terminus of the stem of the antibody and/or the C-terminus of the antibody arm via the N-terminus of a polypeptide chain thereof. Preferably, in the antibody molecule of the present invention, the antibody arm comprises Fab, and the conjugate component comprises scFv, wherein the scFv is fused to the C-terminus of the Fc of the stem of the antibody and/or the C-terminus of the Fab light chain of the antibody arm via the N-terminus of a polypeptide chain thereof.

In some preferred embodiments, the scFv antigen-binding site contained in the antibody molecule of the present invention is disulfide-stabilized scFv, i.e., dsscFv. When the antigen-binding site is dsscFv, the disulfide bond introduced between the VH and VL variable domains of the scFv via mutation may be located between the following residue pairs (the following positions are determined according to Kabat numbering): residue 37 of VH + residue 95 of VL; residue 44 of VH + residue 100 of VL; residue 44 of VH + residue 105 of VL; residue 45 of VH + residue 87 of VL; residue 55 of VH + residue 101 of VL; residue 100 of VH + residue 50 of VL; residue 100b of VH + residue 49 of VL; residue 98 of VH + residue 46 of VL; residue 105 of VH + residue 43 of VL; and residue 106 of VH + residue 57 of VL. Preferably, in order to form dsscFv, cysteine substitutions can be introduced into residue 44 of VH and residue 100 of VL, thereby a disulfide bond linkage can be formed between the two residues when the VH and the VL are paired.

### Antigen-binding site that binds to GPRC5D

The GPRC5D antigen-binding site that may be used in the antibody molecule of the present invention may be any antibody or antibody fragment that can bind to GPRC5D. For example, in the antibody molecule of the present invention, the GPRC5D antigen-binding site contained in the antibody arm or the conjugate component may independently be scFv, dsFv, Fab, scFab or sdAb, and is preferably an antigen-binding site having an scFv or Fab structure described above. Preferably, the GPRC5D antigen-binding site of the antibody arm is selected from: Fab and scFab, preferably Fab. Preferably, the GPRC5D antigen-binding site of the conjugate component is selected from: scFv, dsFv and dsAb, preferably scFv, and more preferably disulfide-stabilized scFv (i.e., dsscFv). In a more preferred embodiment, the antibody comprises an antibody arm that binds to GPRC5D, and the GPRC5D antigen-binding site of the antibody arm is Fab. In another more preferred embodiment, the antibody comprises a conjugate component that binds to GPRC5D, and the GPRC5D antigen-binding site of the conjugate component is scFv, particularly dsscFv.

In some preferred embodiments, the antigen-binding site that binds to GPRC5D, e.g., GPRC5D antigen-binding site having the scFv or Fab structure described above, comprises:
(i) HCDR1, 2, and 3 sequences of a heavy chain variable domain set forth in SEQ ID NO: 25, and LCDR1, 2, and 3 sequences of a light chain variable domain set forth in SEQ ID NO: 26, or
(ii) HCDR1, 2, and 3 sequences of a heavy chain variable domain set forth in SEQ ID NO: 27, and LCDR1, 2, and 3 sequences of a light chain variable domain set forth in SEQ ID NO: 28, or
(iii) HCDR1, 2, and 3 sequences of a heavy chain variable domain set forth in SEQ ID NO: 29, and LCDR1, 2, and 3 sequences of a light chain variable domain set forth in SEQ ID NO: 30, or
(iv) HCDR1, 2, and 3 sequences of a heavy chain variable domain set forth in SEQ ID NO: 31, and LCDR1, 2, and 3 sequences of a light chain variable domain set forth in SEQ ID NO: 32, or
(v) HCDR1, 2, and 3 sequences of a heavy chain variable domain set forth in SEQ ID NO: 98, and LCDR1, 2, and 3 sequences of a light chain variable domain set forth in SEQ ID NO: 99, or
(vi) HCDR1, 2, and 3 sequences of a heavy chain variable domain set forth in SEQ ID NO: 100, and LCDR1, 2, and 3 sequences of a light chain variable domain set forth in SEQ ID NO: 101.

In some preferred embodiments, the antigen-binding site that binds to GPRC5D, e.g., GPRC5D antigen-binding site having the scFv or Fab structure described above, comprises a combination of CDR sequences selected from the followings:
(i) HCDR1, 2, and 3 sequences of a heavy chain variable domain and LCDR1, 2, and 3 sequences of a light chain variable domain set forth in SEQ ID NOs: 1-6, respectively, or
(ii) HCDR1, 2, and 3 sequences of a heavy chain variable domain and LCDR1, 2, and 3 sequences of a light chain variable domain set forth in SEQ ID NOs: 7-12, respectively, or
(iii) HCDR1, 2, and 3 sequences of a heavy chain variable domain and LCDR1, 2, and 3 sequences of a light chain variable domain set forth in SEQ ID NOs: 13-18, respectively, or
(iv) HCDR1, 2, and 3 sequences of a heavy chain variable domain and LCDR1, 2, and 3 sequences of a light chain variable domain set forth in SEQ ID NOs: 19-24, respectively, or
(v) HCDR1, 2, and 3 sequences of a heavy chain variable domain and LCDR1, 2, and 3 sequences of a light chain variable domain set forth in SEQ ID NOs: 13, 110, and 15-18, respectively.

Alternatively, the antigen-binding site that binds to GPRC5D, e.g., scFv or Fab, comprises a variant of one of the combinations of CDR sequences, wherein the variant comprises at least one and no more than 5, 4, 3, 2, or 1 amino acid alteration (preferably amino acid substitution, preferably conservative substitution) in total on 1, 2, 3, 4, 5, or preferably 6 CDR regions, and preferably the heavy chain CDR3 remains unchanged. In some preferred embodiments, the antigen-binding site that binds to GPRC5D, e.g., GPRC5D antigen-binding site having the scFv or Fab structure described above, comprises a heavy chain variable domain VH selected from the followings:
(a) a heavy chain variable domain comprising an amino acid sequence set forth in SEQ ID NO: 25 or an amino acid sequence having at least 80%, 85%, 90%, 95%, 97%, 98%, or 99% sequence identity thereto;
(b) a heavy chain variable domain comprising an amino acid sequence set forth in SEQ ID NO: 27 or an amino acid sequence having at least 80%, 85%, 90%, 95%, 97%, 98%, or 99% sequence identity thereto;
(c) a heavy chain variable domain comprising an amino acid sequence set forth in SEQ ID NO: 29 or an amino acid sequence having at least 80%, 85%, 90%, 95%, 97%, 98%, or 99% sequence identity thereto;
(d) a heavy chain variable domain comprising an amino acid sequence set forth in SEQ ID NO: 31 or an amino acid sequence having at least 80%, 85%, 90%, 95%, 97%, 98%, or 99% sequence identity thereto;
(e) a heavy chain variable domain comprising an amino acid sequence set forth in SEQ ID NO: 98 or an amino acid sequence having at least 80%, 85%, 90%, 95%, 97%, 98%, or 99% sequence identity thereto; and
(f) a heavy chain variable domain comprising an amino acid sequence set forth in SEQ ID NO: 100 or an amino acid sequence having at least 80%, 85%, 90%, 95%, 97%, 98%, or 99% sequence identity thereto.

In some preferred embodiments, the antigen-binding site that binds to GPRC5D, e.g., GPRC5D antigen-binding site having the scFv or Fab structure described above, comprises a light chain variable domain VL selected from the followings:
(a) a light chain variable domain comprising an amino acid sequence set forth in SEQ ID NO: 26 or an amino acid sequence having at least 80%, 85%, 90%, 95%, 97%, 98%, or 99% sequence identity thereto;
(b) a light chain variable domain comprising an amino acid sequence set forth in SEQ ID NO: 28 or an amino acid sequence having at least 80%, 85%, 90%, 95%, 97%, 98%, or 99% sequence identity thereto;
(c) a light chain variable domain comprising an amino acid sequence set forth in SEQ ID NO: 30 or an amino acid sequence having at least 80%, 85%, 90%, 95%, 97%, 98%, or 99% sequence identity thereto;
(d) a light chain variable domain comprising an amino acid sequence set forth in SEQ ID NO: 32 or an amino acid sequence having at least 80%, 85%, 90%, 95%, 97%, 98%, or 99% sequence identity thereto;
(e) a light chain variable domain comprising the amino acid sequence set forth in SEQ ID NO: 99 or an amino acid sequence having at least 80%, 85%, 90%, 95%, 97%, 98%, or 99% sequence identity thereto; and
(f) a light chain variable domain comprising an amino acid sequence set forth in SEQ ID NO: 101 or an amino acid sequence having at least 80%, 85%, 90%, 95%, 97%, 98%, or 99% sequence identity thereto.

In some preferred embodiments, the antigen-binding site that binds to GPRC5D, e.g., GPRC5D antigen-binding site having the scFv or Fab structure described above, comprises a combination of VH and VL amino acid sequences selected from the followings:
(a) a heavy chain variable domain comprising an amino acid sequence set forth in SEQ ID NO: 25 or an amino acid sequence having at least 80%, 85%, 90%, 95%, 97%, 98%, or 99% sequence identity thereto; and a light chain variable domain comprising an amino acid sequence set forth in SEQ ID NO: 26 or an amino acid sequence having at least 80%, 85%, 90%, 95%, 97%, 98%, or 99% sequence identity thereto; or
(b) a heavy chain variable domain comprising an amino acid sequence set forth in SEQ ID NO: 27 or an amino acid sequence having at least 80%, 85%, 90%, 95%, 97%, 98%, or 99% sequence identity thereto; and a light chain variable domain comprising an amino acid sequence set forth in SEQ ID NO: 28 or an amino acid sequence having at least 80%, 85%, 90%, 95%, 97%, 98%, or 99% sequence identity thereto; or
(c) a heavy chain variable domain comprising an amino acid sequence set forth in SEQ ID NO: 29 or an amino acid sequence having at least 80%, 85%, 90%, 95%, 97%, 98%, or 99% sequence identity thereto; and a light chain variable domain comprising an amino acid sequence set forth in SEQ ID NO: 30 or an amino acid sequence having at least 80%, 85%, 90%, 95%, 97%, 98%, or 99% sequence identity thereto; or
(d) a heavy chain variable domain comprising an amino acid sequence set forth in SEQ ID NO: 31 or an amino acid sequence having at least 80%, 85%, 90%, 95%, 97%, 98%, or 99% sequence identity thereto; and a light chain variable domain comprising an amino acid sequence set forth in SEQ ID NO: 32 or an amino acid sequence having at least 80%, 85%, 90%, 95%, 97%, 98%, or 99% sequence identity thereto;
(e) a heavy chain variable domain comprising an amino acid sequence set forth in SEQ ID NO: 98 or an amino acid sequence having at least 80%, 85%, 90%, 95%, 97%, 98%, or 99% sequence identity thereto; and a light chain variable domain comprising an amino acid sequence set forth in SEQ ID NO: 99 or an amino acid sequence having at least 80%, 85%, 90%, 95%, 97%, 98%, or 99% sequence identity thereto; or
(f) a heavy chain variable domain comprising an amino acid sequence set forth in SEQ ID NO: 100 or an amino acid sequence having at least 80%, 85%, 90%, 95%, 97%, 98%, or 99% sequence identity thereto; and a light chain variable domain comprising an amino acid sequence set forth in SEQ ID NO: 101 or an amino acid sequence having at least 80%, 85%, 90%, 95%, 97%, 98%, or 99% sequence identity thereto.

In some preferred embodiments, the antigen-binding site that binds to GPRC5D, e.g., GPRC5D antigen-binding site having the scFv or Fab structure described above, comprises a combination of VH and VL sequences selected from the followings:
(i) a heavy chain variable domain comprising an amino acid sequence set forth in SEQ ID NO: 25 and a light chain variable domain comprising an amino acid sequence set forth in SEQ ID NO: 26, or
(ii) a heavy chain variable domain comprising an amino acid sequence set forth in SEQ ID NO: 27 and a light chain variable domain comprising an amino acid sequence set forth in SEQ ID NO: 28, or
(iii) a heavy chain variable domain comprising an amino acid sequence set forth in SEQ ID NO: 29, and a light chain variable domain comprising an amino acid sequence set forth in SEQ ID NO: 30, or
(iv) a heavy chain variable domain comprising an amino acid sequence set forth in SEQ ID NO: 31 and a light chain variable domain comprising an amino acid sequence set forth in SEQ ID NO: 32;
(v) a heavy chain variable domain comprising an amino acid sequence set forth in SEQ ID NO: 98 and a light chain variable domain comprising an amino acid sequence set forth in SEQ ID NO: 99, or
(vi) a heavy chain variable domain comprising an amino acid sequence set forth in SEQ ID NO: 100, and a light chain variable domain comprising an amino acid sequence set forth in SEQ ID NO: 101.

In some preferred embodiments, when the antigen-binding site that binds to GPRC5D is disulfide-stabilized scFv, the scFv further comprises a cysteine substitution at position 44 of the VH domain and a cysteine substitution at position 100 of the VL domain.

### Antigen-binding site that binds to BCMA

The BCMA antigen-binding site that may be used for the antibody molecule of the present invention may be any antibody or antibody fragment that can bind to BCMA. For example, in the antibody molecule of the present invention, the BCMA antigen-binding site contained in the antibody arm or the conjugate component may independently be scFv, dsFv, Fab, scFab, or sdAb, and is preferably an antigen-binding site having the scFv or Fab structure described above. Preferably, the BCMA antigen-binding site of the antibody arm is selected from: Fab and scFab, preferably Fab. Preferably, the BCMA antigen-binding site of the conjugate component is selected from: scFv, dsFv and dsAb, preferably scFv, and more preferably disulfide-stabilized scFv (i.e., dsscFv). In a more preferred embodiment, the antibody comprises an antibody arm that binds to BCMA, and the BCMA antigen-binding site of the antibody arm is Fab. In another more preferred embodiment, the antibody comprises a conjugate component that binds to BCMA, and the BCMA antigen-binding site of the conjugate component is scFv, particularly dsscFv.

In some preferred embodiments, the antigen-binding site that binds to BCMA, e.g., BCMA antigen-binding site having the scFv or Fab structure described above, comprises: HCDR1, 2 and, 3 sequences of a heavy chain variable domain set forth in SEQ ID NO: 39, and LCDR1, 2, and 3 sequences of a light chain variable domain set forth in SEQ ID NO: 40.

In some preferred embodiments, the antigen-binding site that binds to BCMA, e.g., BCMA antigen-binding site having the scFv or Fab structure described above, comprises a combination of CDR sequences selected from the followings:
- HCDR1 comprising or consisting of an amino acid sequence set forth in SEQ ID NO: 33;
- HCDR2 comprising or consisting of an amino acid sequence set forth in SEQ ID NO: 34;
- HCDR3 comprising or consisting of an amino acid sequence set forth in SEQ ID NO: 35;
- LCDR1 comprising or consisting of an amino acid sequence set forth in SEQ ID NO: 36;
- LCDR2 comprising or consisting of an amino acid sequence set forth in SEQ ID NO: 37; and
- LCDR3 comprising or consisting of an amino acid sequence set forth in SEQ ID NO: 38,
alternatively, the antigen-binding site that binds to BCMA comprises a variant of one of the combinations of CDR sequences, wherein the variant comprises at least one and no more than 5, 4, 3, 2, or 1 amino acid alteration (preferably amino acid substitution, preferably conservative substitution) in total on 1, 2, 3, 4, 5, or preferably 6 CDR regions, and preferably the heavy chain CDR3 remains unchanged.

In some preferred embodiments, the antigen-binding site that binds to BCMA, e.g., BCMA antigen-binding site having the scFv or Fab structure described above, comprises a heavy chain variable domain VH having an amino acid sequence set forth in SEQ ID NO: 39 or an amino acid sequence having at least 80%, 85%, 90%, 95%, 97%, 98%, or 99% sequence identity thereto.

In some preferred embodiments, the antigen-binding site that binds to BCMA, e.g., BCMA antigen-binding site having the scFv or Fab structure described above, comprises a light chain variable domain VL having an amino acid sequence set forth in SEQ ID NO: 40 or an amino acid sequence having at least 80%, 85%, 90%, 95%, 97%, 98%, or 99% sequence identity thereto.

In some preferred embodiments, the antigen-binding site that binds to BCMA, e.g. BCMA antigen-binding site having the scFv or Fab structure described above, comprises a heavy chain variable domain VH having an amino acid sequence set forth in SEQ ID NO: 39 or an amino acid sequence having at least 80%, 85%, 90%, 95%, 97%, 98%, or 99% sequence identity thereto, and comprises a light chain variable domain VL having an amino acid sequence set forth in SEQ ID NO: 40 or an amino acid sequence having at least 80%, 85%, 90%, 95%, 97%, 98%, or 99% sequence identity thereto.

Preferably, the antigen-binding site that binds to BCMA, e.g., the BCMA antigen-binding site having an scFv or Fab structure described above, comprises a VH domain having an amino acid sequence set forth in SEQ ID NO: 39 and a VL domain having an amino acid sequence set forth in SEQ ID NO: 40. In some preferred embodiments, when the antigen-binding site that binds to BCMA is a disulfide-stabilized scFv, the scFv further comprises a cysteine substitution at position 44 of the VH domain and a cysteine substitution at position 100 of the VL domain.

### Antigen-binding site that binds to CD3

The CD3 antigen-binding site that may be used for the antibody molecule of the present invention may be any antibody or antibody fragment that can bind to CD3. For example, in the antibody molecule of the present invention, the CD3 antigen-binding site contained in the antibody arm or the conjugate component may independently be scFv, dsFv, Fab, scFab, or sdAb, and is preferably an antigen-binding site having the scFv or Fab structure described above. Preferably, the CD3 antigen-binding site of the antibody arm is selected from: Fab and scFab, preferably Fab. Preferably, the CD3 antigen-binding site of the conjugate component is selected from: scFv, dsFv and dsAb, preferably scFv, and more preferably disulfide-stabilized scFv (i.e., dsscFv). In a more preferred embodiment, the antibody comprises an antibody arm that binds to CD3, and the CD3 antigen-binding site of the antibody arm is Fab. In another more preferred embodiment, the antibody comprises a conjugate component that binds to CD3, and the CD3 antigen-binding site of the conjugate component is scFv, particularly dsscFv.

In some preferred embodiments, the antigen-binding site that binds to CD3, e.g., CD3 antigen-binding site having the scFv or Fab structure described above, comprises: HCDR1, 2, and 3 sequences of a heavy chain variable domain set forth in SEQ ID NO: 48, and LCDR1, 2, and 3 sequences of a light chain variable domain set forth in SEQ ID NO: 49.

In other preferred embodiments, the antigen-binding site that binds to CD3, e.g., CD3 antigen-binding site having the scFv or Fab structure described above, comprises: HCDR1, 2, and 3 sequences of a heavy chain variable domain set forth in SEQ ID NO: 50, and LCDR1, 2, and 3 sequences of a light chain variable domain set forth in SEQ ID NO: 49.

In some preferred embodiments, the antigen-binding site that binds to CD3, e.g., CD3 antigen-binding site having the scFv or Fab structure described above, comprises a combination of CDR sequences selected from the followings:
- HCDR1 comprising or consisting of an amino acid sequence set forth in SEQ ID NO: 41;
- HCDR2 comprising or consisting of an amino acid sequence set forth in SEQ ID NO: 42 or 47;
- HCDR3 comprising or consisting of an amino acid sequence set forth in SEQ ID NO: 43;
- LCDR1 comprising or consisting of an amino acid sequence set forth in SEQ ID NO: 44;
- LCDR2 comprising or consisting of an amino acid sequence set forth in SEQ ID NO: 45; and
- LCDR3 comprising or consisting of an amino acid sequence set forth in SEQ ID NO: 46,
alternatively, the antigen-binding site that binds to CD3 comprises a variant of one of the combinations of CDR sequences, wherein the variant comprises at least one and no more than 5, 4, 3, 2, or 1 amino acid alteration (preferably amino acid substitution, preferably conservative substitution) in total on 1, 2, 3, 4, 5, or preferably 6 CDR regions, and preferably the heavy chain CDR3 remains unchanged.

In some preferred embodiments, the antigen-binding site that binds to CD3, e.g., CD3 antigen-binding site having the scFv or Fab structure described above, comprises a heavy chain variable domain VH having an amino acid sequence set forth in SEQ ID NO: 48 or 50, or an amino acid sequence having at least 80%, 85%, 90%, 95%, 97%, 98%, or 99% sequence identity thereto.

In some preferred embodiments, the antigen-binding site that binds to CD3, e.g., CD3 antigen-binding site having the scFv or Fab structure described above, comprises a light chain variable domain VL having an amino acid sequence set forth in SEQ ID NO: 49 or an amino acid sequence having at least 80%, 85%, 90%, 95%, 97%, 98%, or 99% sequence identity thereto.

In some preferred embodiments, the antigen-binding site that binds to CD3, e.g., CD3 antigen-binding site having the scFv or Fab structure described above, comprises a heavy chain variable domain VH having an amino acid sequence set forth in SEQ ID NO: 48 or 50 or an amino acid sequence having at least 80%, 85%, 90%, 95%, 97%, 98%, or 99% sequence identity thereto; and a light chain variable domain VL comprising an amino acid sequence set forth in SEQ ID NO: 49 or an amino acid sequence having at least 80%, 85%, 90%, 95%, 97%, 98%, or 99% sequence identity thereto.

Preferably, the antigen-binding site that binds to CD3, e.g., CD3 antigen-binding site having the scFv or Fab structure described above, comprises a VH domain having an amino acid sequence set forth in SEQ ID NO: 48 or 50, and a VL domain having an amino acid sequence set forth in SEQ ID NO: 49. More preferably, the antigen-binding site that binds to CD3, e.g., the CD3 antigen-binding site having an scFv or Fab structure described above, comprises a VH domain having an amino acid sequence set forth in SEQ ID NO: 48, and a VL domain having an amino acid sequence set forth in SEQ ID NO: 49. In some preferred embodiments, when the antigen-binding site that binds to CD3 is a disulfide-stabilized scFv, the scFv further comprises a cysteine substitution at position 44 of the VH domain and a cysteine substitution at position 100 of the VL domain.

### Stem of antibody molecule of the present invention

The antibody molecule of the present invention comprises a stem located at the C-terminus of the antibody arm and formed by a first Fc domain and a second Fc domain. In some embodiments, the first and second Fc domains are the same. In other embodiments, the first Fc domain and the second Fc domain are different, and the two are paired and heterodimerized.

The Fc domain fragments suitable for the antibody molecules of the present invention may be any antibody Fc domain. For example, the Fc domain of the antibody of the present invention may comprise two or three constant domains, i.e., a CH2 domain, a CH3 domain, and optionally a CH4 domain. Preferably, the Fc domain of the antibody of the present invention comprises CH2-CH3 from N-terminus to C-terminus, more preferably hinge region-CH2-CH3 from N-terminus to C-terminus.

In the antibody molecule of the present invention, the Fc domain may comprise the second and the third constant domains (CH2 domain and CH3 domain) derived from IgG, IgA, and IgD antibodies, or comprise the second, the third, and the fourth constant domains (CH2 domain, CH3 domain, and CH4 domain) derived from IgM and IgE antibodies. In some embodiments, the Fc domain of the antibody molecule is an Fc domain from IgG, such as Fc domains from IgG1, IgG2, or IgG4, preferably an Fc domain from human IgG1.

As understood by those skilled in the art, according to the expected use of the antibody molecule of the present invention, the antibody molecule of the present invention may comprise modifications in the Fc domain that alter the effector functions. In one embodiment, one or more effector functions of the Fc domain of the present invention have been reduced or eliminated relative to a wild-type Fc domain of the same isotype. The effector function of the Fc domain may be reduced or eliminated by any method selected from the followings: altering the glycosylation of the Fc domain, using an Fc isotype that naturally has reduced or eliminated effector functions, and modifying the amino acid sequence of the Fc domain.

In one embodiment, the effector function is reduced or eliminated by reducing the glycosylation of the Fc domain. Various methods known in the art that can be used to reduce the glycosylation of the Fc domain include, but are not limited to, producing the antibody molecule of the present invention in an environment that does not allow wild-type glycosylation; removing a carbohydrate group already present on the Fc domain; and modifying the Fc domain so that wild-type glycosylation does not occur. In one embodiment, the glycosylation of the Fc domain is reduced by modifying the Fc domain, for example, a mutation is introduced at position 297 of the Fc domain, such that the wild-type asparagine residue at this position is substituted with another amino acid that interferes with the glycosylation at this position, such as an N297A mutation.

In another embodiment, the effector function is reduced or eliminated by modifying the amino acid sequence of at least one Fc domain. The modification of the Fc domain may be selected from: introducing a point mutation that damages the binding of the Fc domain to one or more Fc receptors at one or more of the following positions: 238, 239, 248, 249, 252, 254, 265, 268, 269, 270, 272, 278, 289, 292, 293, 294, 295, 296, 297, 298, 301, 303, 322, 324, 327, 329, 333, 335, 338, 340, 373, 376, 382, 388, 389, 414, 416, 419, 434, 435, 437, 438 and 439; or introducing a point mutation that damages the C1q binding at a position selected from: 270, 322, 329 and 321.

As understood by a person skilled in the art, according to the expected use of the antibody molecule of the present invention, the antibody molecule of the present invention can further comprise modifications in the Fc domain that alter binding affinity to one or more Fc receptors. In one embodiment, the Fc receptor is an Fcy receptor, in particular a human Fcy receptor. In one embodiment, the modification reduces the effector function of the antibody molecule of the present invention. In one specific embodiment, the effector function is antibody-dependent cell-mediated cytotoxicity (ADCC). In one embodiment, the Fc domain of the antibody molecule of the present invention comprises amino acid replacements at positions 234 and 235 (EU numbering). In one specific embodiment, the amino acid replacements are L234A and L235A ("LALA mutations"). In another embodiment, the Fc domain of an antibody molecule of the present invention comprises an amino acid replacement at position 329 (EU numbering). In one specific embodiment, the amino acid replacement is P329G.

As understood by a person skilled in the art, when the antibody molecule of the present invention is an asymmetric antibody molecule (e.g., the antibody molecules of Format_2 and Format_7), in order to promote the formation of a correct asymmetric antibody molecule, the antibody molecule of the present invention may comprise mutations in the first and second Fc domains that facilitate heterodimerization of the first Fc domain with the second Fc domain. For example, complementary Knob and Hole mutations can be introduced into the first and second Fc domains on the basis of the Knob-in-Hole technique.

Therefore, in some embodiments, the present invention provides a trispecific antibody molecule, wherein the antibody molecule comprises first and second heterodimerization mutations in the first and second Fc domains, respectively, that promote pairing and heterodimerization of the first and second Fc domains. In some preferred embodiments, the first heterodimerization mutation on the first Fc domain comprises a Knob mutation and the second heterodimerization mutation of the second Fc domain comprises a Hole mutation complementary to the Knob mutation, or the first heterodimerization mutation on the first Fc domain comprises a Hole mutation and the second heterodimerization mutation of the second Fc domain comprises a Knob mutation complementary to the Hole mutation. In some preferred embodiments, the Knob mutation is T366W and the complementary hole mutation is T366S/L368A/Y407V.

In one embodiment, the present invention provides a trispecific antibody molecule, which comprises:
i) a homodimeric Fc-region of the human IgG1 subtype, optionally with mutations L234A and L235A, or
ii) a homodimeric Fc-region of the human IgG4 subtype, optionally with mutations P329G, S228P, and L235E, or
iii) a heterodimeric Fc-region, wherein
   a) one Fc-region polypeptide comprises a mutation T366W, and the other Fc-region polypeptide comprises mutations T366S, L368A and Y407V, or
   b) one Fc-region polypeptide comprises mutations T366W and Y349C, and the other Fc-region polypeptide comprises mutations T366S, L368A, Y407V and S354C, or
   c) one Fc-region polypeptide comprises mutations T366W and S354C, and the other Fc-region polypeptide comprises mutations T366S, L368A, Y407V and Y349C,
      or
iv) a heterodimeric Fc-region of the human IgG1 subtype, wherein the two Fc-region polypeptides both comprise mutations L234A and L235A, and
   a) one Fc-region polypeptide comprises a mutation T366W, and the other Fc-region polypeptide comprises mutations T366S, L368A and Y407V, or
   b) one Fc-region polypeptide comprises mutations T366W and Y349C, and the other Fc-region polypeptide comprises mutations T366S, L368A, Y407V and S354C, or
   c) one Fc-region polypeptide comprises mutations T366W and S354C, and the other Fc-region polypeptide comprises mutations T366S, L368A, Y407V and Y349C,
      or
v) a heterodimeric Fc-region of the human IgG4 subtype, wherein the two Fc-region polypeptides both comprise mutations P329G, S228P and L235E, and
   a) one Fc-region polypeptide comprises a mutation T366W, and the other Fc-region polypeptide comprises mutations T366S, L368A and Y407V, or
   b) one Fc-region polypeptide comprises mutations T366W and Y349C, and the other Fc-region polypeptide comprises mutations T366S, L368A, Y407V and S354C, or
   c) one Fc-region polypeptide comprises mutations T366W and S354C, and the other Fc-region polypeptide comprises mutations T366S, L368A, Y407V and Y349C.

In some embodiments, the Fc domain of the antibody molecule of the present invention may further comprise other mutations that facilitate the purification of a heteromultimeric antibody. For example, an H435R mutation can be introduced into one of the first and second Fc domains (e.g., an Fc domain with a Hole mutation) to facilitate the purification of a target heteromultimeric antibody using protein A.

In the antibody molecule of the present invention, the stem of the antibody is linked to the antibody arm at the N-terminus of the Fc domain. As will be appreciated by a person skilled in the art, linker peptides suitable for linking the antibody arms and the Fc domain fragments of the stem in the antibody molecule of the present invention may be any flexible linker peptide known in the art. In some embodiments, the linker peptide may comprise a hinge region amino acid sequence derived from IgG1, or may comprise an amino acid sequence selected from the followings: (GS)n, (GSGGS)n, (GGGGS)n and (GGGS)n, wherein n is an integer of at least 1. Preferably, the stem of the antibody is linked to the antibody arm via a hinge region from IgG (particularly a hinge region from human IgG1). In some embodiments, with regard to the heteromultimeric antibody of the present invention comprising hinge regions, a mutation, such as C220S, may be introduced in the hinge regions to facilitate the formation of the target heteromultimeric antibody.

### Exemplary trispecific antibody molecule

Format_2

In some embodiments, when p = 0 and q = 1, the antibody molecule of the present invention has the structure: (M1:M2)-Fc::Fc-(X2)q. Therefore, there is only a conjugate component X2 conjugated to the stem of the antibody molecule. In the antibody molecule, preferably, the antibody arms M1 and M2 comprise Fab antigen-binding sites that bind to a first antigen and a second antigen, respectively, and the conjugate component X2 comprises an scFv antigen-binding site that binds to a third antigen, wherein the first, second and third antigens are different from each other and independently selected from: GPRC5D, BCMA and CD3.

Therefore, in some preferred embodiments, the present invention provides a trispecific Y-type antibody molecule having the structure:

(M1:M2)-Fc::Fc-(X2)q, (Format_2)

wherein q = 1,
M1 and M2 represent a first antibody arm and a second antibody arm of the antibody molecule, respectively, and M1 and M2 comprise Fab or scFab, preferably Fab, that bind to a first antigen and a second antigen, respectively;
Fc::Fc represents the stem of the antibody molecule, consisting of a first Fc domain and a second Fc domain which are paired and dimerized, wherein the first and second antibody arms are linked to the N-termini of the first Fc domain and the second Fc domain, either directly or via a linker peptide (preferably a hinge region), respectively;
X2 represents a conjugate component conjugated to the C-terminus of the stem, wherein the conjugate component comprises an scFv that binds to a third antigen, wherein X2 is conjugated to the C-terminus of the first Fc domain or to the C-terminus of the second Fc domain, either directly or via a linker peptide, the first, the second, and the third antigens are different from each other and are independently selected from GPRC5D, BCMA, and CD3.

In some embodiments, the present invention provides a trispecific antibody molecule having a structure of Format_2, wherein
(a): the antibody arm M1 and antibody arm M2 of the molecule comprise Fab that binds to GPRC5D and Fab that binds to CD3, respectively, the conjugate component X2 comprises scFv that binds to BCMA, and the molecule (e.g., the TS-F2-1, 3-6 molecules in the examples) has the structure: (Fab_GPRC5D:Fab_CD3)-Fc::Fc-(scFv_BCMA); or
(b): the antibody arm M1 and antibody arm M2 of the molecule comprise Fab that binds to BCMA and Fab that binds to CD3, respectively, the conjugate component X2 comprises scFv that binds to GPRC5D, and the molecule (e.g., the TS-F2-2 molecule in the examples) has the structure (Fab_BCMA:Fab_CD3)-Fc::Fc-(scFv_GPRC5D), or
(c): the antibody arm M1 and antibody arm M2 of the molecule comprise Fab that binds to GPRC5D and Fab that binds to BCMA, respectively, the conjugate component X2 comprises scFv that binds to CD3, and the molecule has the structure: (Fab_GPRC5D:Fab_BCMA)-Fc::Fc-(scFv_CD3).

In any one of the embodiments of the antibody molecule of Format_2 of the present invention described above, the Fab or scFv that binds to GPRC5D may be any suitable Fab or scFv that specifically binds to GPRC5D, such as the GPRC5D antigen-binding site of the present invention having an scFv or Fab structure described above, particularly scFv or Fab having the VH and VL amino acid sequences described above. In any one of the embodiments of the antibody molecule of Format_2 of the present invention described above, Fab or scFv that binds to BCMA may be any suitable Fab or scFv that specifically binds to BCMA, such as the BCMA antigen-binding site of the present invention having an scFv or Fab structure described above, particularly scFv or Fab having VH and VL amino acid sequences described above.

In any one of the embodiments of the antibody molecule of Format_2 of the present invention described above, the antigen-binding site that binds to CD3 may be any suitable scFv or Fab that specifically binds to CD3, such as the CD3 antigen-binding site of the present invention having an scFv or Fab structure described above, particularly scFv or Fab having VH and VL amino acid sequences described above.

In any one of the embodiments of the antibody molecule of Format_2 of the present invention described above, the antibody molecule may comprise any antibody stem structure of the present invention that is suitable for Format_2 described above. For example, in the asymmetric trispecific antibody molecule of Format_2 of the present invention, preferably, in order to promote correct pairing of the polypeptide chains of the antibody molecule, mutations that facilitate heterodimerization of the first and second Fc domains, particularly complementary "knob-in-hole" mutations, may be introduced into the first and the second Fc domains of the stem, and for example, a knob mutation is introduced into the first Fc domain and a complementary hole mutation is introduced into the second Fc domain, or vice versa, so that the two Fc domains of the antibody molecule may be paired to form a "knob-in-hole" stable association. According to the expected use, the first and/or second Fc domains of the antibody molecule of Format_2 of the present invention preferably may further comprise a mutation that affects the effector function of the antibody, such as a mutation that decreases ADCC activity, e.g., an LALA mutation.

In some embodiments, the trispecific antibody molecule of Format_2 of the present invention comprises or consists of a first heavy chain polypeptide chain, a first light chain polypeptide chain, a second heavy chain polypeptide chain and a second light chain polypeptide chain, wherein
the first heavy chain polypeptide chain comprises a heavy chain variable domain VH, an immunoglobulin CH1 domain and an Fc domain from the N-terminus to the C-terminus;
the first light chain polypeptide chain comprises a light chain variable domain VL and an immunoglobulin CL domain from the N-terminus to the C-terminus;
the second heavy chain polypeptide chain comprises a heavy chain variable domain VH, an immunoglobulin CH1 domain and an Fc domain from the N-terminus to the C-terminus; and
the second light chain polypeptide chain comprises a light chain variable domain VL and an immunoglobulin CL domain from the N-terminus to the C-terminus;
and wherein the first heavy chain polypeptide chain or the second heavy chain polypeptide chain further comprises an scFv domain conjugated to the C-terminus of an Fc domain thereof, either directly or preferably via a linker peptide (e.g., (G4S)2 or TS(G4S)2);
wherein,
VH-CH1 of the first heavy chain polypeptide chain pair and VL-CL of the first light chain polypeptide chain are paired to form a first antibody arm (M1) that binds to a first antigen;
VH-CH1 of the second heavy chain polypeptide chain and VL-CL of the second light chain polypeptide chain are paired to form a second antibody arm (M2) that binds to a second antigen;
the Fc domain of the first heavy chain polypeptide chain and the Fc domain of the second heavy chain polypeptide chain are paired and dimerized to form a stem of the antibody (Fc::Fc); and
the scFv domain conjugated to the C-terminus of the first heavy chain polypeptide chain or the second heavy chain polypeptide chain forms a conjugate component (X2) that binds to a third antigen,
wherein the first, second and third antigens are different and independently selected from: GPRC5D, BCMA, and CD3.

In one embodiment, the antibody arm M1 binds to GPRC5D, the second antibody arm M2 binds to CD3, the conjugate component X2 binds to BCMA, and therefore,
- the first heavy chain polypeptide chain and the first light chain polypeptide chain comprise VH and VL amino acid sequences that specifically bind to a Fab antigen-binding site of GPRC5D at the N-terminus, respectively, and
- the second heavy chain polypeptide chain and the second light chain polypeptide chain comprise VH and VL amino acid sequences that specifically bind to a Fab antigen-binding site of CD3 at the N-terminus, respectively,
wherein when the conjugate component X2 is conjugated to the first heavy chain polypeptide chain, the first heavy chain polypeptide chain further comprises an scFv amino acid sequence that specifically binds to BCMA at the C-terminus, or preferably, when the conjugate component X2 is conjugated to the second heavy chain polypeptide chain, the second heavy chain polypeptide chain further comprises an scFv amino acid sequence that specifically binds to BCMA at the C-terminus.

In another embodiment, the antibody arm M1 binds to BCMA, the second antibody arm M2 binds to CD3, the conjugate component X2 binds to GPRC5D, and therefore,
- the first heavy chain polypeptide chain and the first light chain polypeptide chain comprise VH and VL amino acid sequences that specifically bind to a Fab antigen-binding site of BCMA at the N-terminus, respectively, and
- the second heavy chain polypeptide chain and the second light chain polypeptide chain comprise VH and VL amino acid sequences that specifically bind to a Fab antigen-binding site of CD3 at the N-terminus, respectively,
wherein when the conjugate component X2 is conjugated to the first heavy chain polypeptide chain, the first heavy chain polypeptide chain further comprises an scFv amino acid sequence that specifically binds to GPRC5D at the C-terminus, or preferably, when the conjugate component X2 is conjugated to the second heavy chain polypeptide chain, the second heavy chain polypeptide chain further comprises an scFv amino acid sequence that specifically binds to GPRC5D at the C-terminus.

In yet another embodiment, the antibody arm M1 binds to GPRC5D, the second antibody arm M2 binds to BCMA, the conjugate component X2 binds to CD3, and therefore,
- the first heavy chain polypeptide chain and the first light chain polypeptide chain comprise VH and VL amino acid sequences that specifically bind to a Fab antigen-binding site of GPRC5D at the N-terminus, respectively, and
- the second heavy chain polypeptide chain and the second light chain polypeptide chain comprise VH and VL amino acid sequences that specifically bind to a Fab antigen-binding site of BCMA at the N-terminus, respectively,
wherein when the conjugate component X2 is conjugated to the first heavy chain polypeptide chain, the first heavy chain polypeptide chain further comprises an scFv amino acid sequence that specifically binds to CD3 at the C-terminus, or when the conjugate component X2 is conjugated to the second heavy chain polypeptide chain, the second heavy chain polypeptide chain further comprises an scFv amino acid sequence that specifically binds to CD3 at the C-terminus.

In a preferred embodiment, Fab or scFv that specifically binds to GPRC5D comprises VH and VL selected from the following groups:
(a) VH comprising HCDR1-3 sequences set forth in SEQ ID NOs: 1-3 and VL comprising LCDR1-3 sequences set forth in SEQ ID NOs: 4-6;
(b) VH comprising HCDR1-3 sequences set forth in SEQ ID NOs: 7-8 and VL comprising LCDR1-3 sequences set forth in SEQ ID NOs: 10-12;
(c) VH comprising HCDR1-3 sequences set forth in SEQ ID NOs: 13-15 and VL comprising LCDR1-3 sequences set forth in SEQ ID NOs: 16-18; or
(d) VH comprising HCDR1-3 sequences set forth in SEQ ID NOs: 19-21 and VL comprising LCDR1-3 sequences set forth in SEQ ID NOs: 22-24.

Preferably, the Fab and the scFv comprise VH and VL amino acid sequence pairs selected from the following groups: SEQ ID NOs: 25/26, SEQ ID NOs: 27/28, SEQ ID NOs: 29/30, and SEQ ID NOs: 31/32, and preferably, when the scFv is dsscFv, the antigen-binding site further comprises a cysteine replacement introduced into the VH and VL sequences, such as a cysteine replacement at position 44 in the VH sequence and position 100 in the VL sequence.

In a preferred embodiment, the Fab or scFv that specifically binds to CD3 comprises VH and VL selected from the following groups:
(a) VH comprising HCDR1-3 sequences set forth in SEQ ID NOs: 41-43 and VL comprising LCDR1-3 sequences set forth in SEQ ID NOs: 44-46; or
(b) VH comprising HCDR1-3 sequences set forth in SEQ ID NOs: 41, 47, and 43, and VL comprising LCDR1-3 sequences set forth in SEQ ID NOs: 44-46.

Preferably, the Fab and the scFv comprise VH and VL amino acid sequence pairs selected from the following groups: SEQ ID NOs: 48/49, SEQ ID NOs: 50/49, and more preferably SEQ ID NOs: 48/49, and preferably, when the scFv is dsscFv, the antigen-binding site further comprises a cysteine replacement introduced into the VH and VL sequences, such as a cysteine replacement at position 44 in the VH sequence and position 100 in the VL sequence.

In a preferred embodiment, the Fab or scFv that specifically binds to BCMA comprises the following VH and VL:
VH comprising HCDR1-3 sequences set forth in SEQ ID NOs: 33-35 and VL comprising LCDR1-3 sequences set forth in SEQ ID NOs: 36-38.

Preferably, the Fab and the scFv comprise a VH and VL amino acid sequence pair: SEQ ID NOs: 39/40, and preferably, when the scFv is dsscFv, the antigen-binding site further comprises a cysteine replacement introduced into the VH and VL sequences, such as a cysteine replacement at position 44 in the VH sequence and position 100 in the VL sequence.

In some preferred embodiments, the first light chain polypeptide chain and the second light chain polypeptide chain comprise a kappa light chain constant domain CLκ and a lamda light chain constant domain CL_{λ}, respectively. In a specific embodiment, the light chain constant domain CLx comprises an amino acid sequence set forth in SEQ ID NO: 105 or an amino acid sequence having at least 90%, 92%, 95%, 97%, 98%, 99% or higher identity thereto; and the light chain constant domain CL_{λ} comprises an amino acid sequence set forth in SEQ ID NO: 106 or an amino acid sequence having at least 90%, 92%, 95%, 97%, 98%, 99% or higher identity thereto.

In some preferred embodiments, the first heavy chain polypeptide chain and the second heavy chain polypeptide chain comprise a heavy chain constant region CH1 derived from human IgG1, and preferably comprise an amino acid sequence set forth in SEQ ID NO: 104 or an amino acid sequence having at least 90%, 92%, 95%, 97%, 98%, 99% or higher identity thereto.

In some preferred embodiments, the first heavy chain polypeptide chain and the second heavy chain polypeptide chain comprise an Fc domain containing a knob or complementary hole mutation, respectively. In one specific embodiment, the Fc domain containing a knob mutation comprises T366W; and the Fc domains containing a complementary hole mutation comprises T366S, L368A and Y407V mutations. In one specific embodiment, the Fc domain containing a hole mutation comprises amino acid sequences selected from SEQ ID NO: 102 and 107 or amino acid sequences having at least 90%, 92%, 95%, 97%, 98%, 99% or higher identity thereto; and the Fc domain containing a complementary knob mutation comprises an amino acid sequence set forth in SEQ ID NO: 103 or an amino acid sequence having at least 90%, 92%, 95%, 97%, 98%, 99% or higher identity thereto.

In some embodiments, the present invention provides a trispecific antibody molecule having a structure of Format_2, and the trispecific antibody molecule comprises or consists of a first heavy chain polypeptide chain, a first light chain polypeptide chain, a second heavy chain polypeptide chain and a second light chain polypeptide chain, wherein the first heavy chain polypeptide chain comprises an amino acid sequence set forth in SEQ ID NO: 65 or an amino acid sequence having at least 90%, 92%, 95%, 97%, 98%, 99% or higher identity thereto; the first light chain polypeptide chain comprises an amino acid sequence set forth in SEQ ID NO: 66 or an amino acid sequence having at least 90%, 92%, 95%, 97%, 98%, 99% or higher identity thereto; the second heavy chain polypeptide chain comprises an amino acid sequence set forth in SEQ ID NO: 67 or an amino acid sequence having at least 90%, 92%, 95%, 97%, 98%, 99% or higher identity thereto; and the second light chain polypeptide chain comprises an amino acid sequence set forth in SEQ ID NO: 68 or an amino acid sequence having at least 90%, 92%, 95%, 97%, 98%, 99% or higher identity thereto.

In some embodiments, the present invention provides a trispecific antibody molecule having a structure of Format_2, and the trispecific antibody molecule comprises or consists of a first heavy chain polypeptide chain, a first light chain polypeptide chain, a second heavy chain polypeptide chain and a second light chain polypeptide chain, wherein the first heavy chain polypeptide chain comprises an amino acid sequence set forth in SEQ ID NO: 69 or an amino acid sequence having at least 90%, 92%, 95%, 97%, 98%, 99% or higher identity thereto; the first light chain polypeptide chain comprises an amino acid sequence set forth in SEQ ID NO: 70 or an amino acid sequence having at least 90%, 92%, 95%, 97%, 98%, 99% or higher identity thereto; the second heavy chain polypeptide chain comprises an amino acid sequence set forth in SEQ ID NO: 71 or an amino acid sequence having at least 90%, 92%, 95%, 97%, 98%, 99% or higher identity thereto; and the second light chain polypeptide chain comprises an amino acid sequence set forth in SEQ ID NO: 68 or an amino acid sequence having at least 90%, 92%, 95%, 97%, 98%, 99% or higher identity thereto.

In some embodiments, the present invention provides a trispecific antibody molecule having a structure of Format_2, and the trispecific antibody molecule comprises or consists of a first heavy chain polypeptide chain, a first light chain polypeptide chain, a second heavy chain polypeptide chain and a second light chain polypeptide chain, wherein the first heavy chain polypeptide chain comprises an amino acid sequence set forth in SEQ ID NO: 72 or an amino acid sequence having at least 90%, 92%, 95%, 97%, 98%, 99% or higher identity thereto; the first light chain polypeptide chain comprises an amino acid sequence set forth in SEQ ID NO: 73 or an amino acid sequence having at least 90%, 92%, 95%, 97%, 98%, 99% or higher identity thereto; the second heavy chain polypeptide chain comprises an amino acid sequence set forth in SEQ ID NO: 74 or an amino acid sequence having at least 90%, 92%, 95%, 97%, 98%, 99% or higher identity thereto; and the second light chain polypeptide chain comprises an amino acid sequence set forth in SEQ ID NO: 68 or an amino acid sequence having at least 90%, 92%, 95%, 97%, 98%, 99% or higher identity thereto.

In some embodiments, the present invention provides a trispecific antibody molecule having a structure of Format_2, and the trispecific antibody molecule comprises or consists of a first heavy chain polypeptide chain, a first light chain polypeptide chain, a second heavy chain polypeptide chain and a second light chain polypeptide chain, wherein the first heavy chain polypeptide chain comprises an amino acid sequence set forth in SEQ ID NO: 75 or an amino acid sequence having at least 90%, 92%, 95%, 97%, 98%, 99% or higher identity thereto; the first light chain polypeptide chain comprises an amino acid sequence set forth in SEQ ID NO: 76 or an amino acid sequence having at least 90%, 92%, 95%, 97%, 98%, 99% or higher identity thereto; the second heavy chain polypeptide chain comprises an amino acid sequence set forth in SEQ ID NO: 77 or 78 or an amino acid sequence having at least 90%, 92%, 95%, 97%, 98%, 99% or higher identity thereto; and
the second light chain polypeptide chain comprises an amino acid sequence set forth in SEQ ID NO: 68 or an amino acid sequence having at least 90%, 92%, 95%, 97%, 98%, 99% or higher identity thereto.

In some embodiments, the present invention provides a trispecific antibody molecule having a structure of Format_2, and the trispecific antibody molecule comprises or consists of a first heavy chain polypeptide chain, a first light chain polypeptide chain, a second heavy chain polypeptide chain and a second light chain polypeptide chain, wherein the first heavy chain polypeptide chain comprises an amino acid sequence set forth in SEQ ID NO: 79 or an amino acid sequence having at least 90%, 92%, 95%, 97%, 98%, 99% or higher identity thereto; the first light chain polypeptide chain comprises an amino acid sequence set forth in SEQ ID NO: 80 or an amino acid sequence having at least 90%, 92%, 95%, 97%, 98%, 99% or higher identity thereto; the second heavy chain polypeptide chain comprises an amino acid sequence set forth in SEQ ID NO: 77 or an amino acid sequence having at least 90%, 92%, 95%, 97%, 98%, 99% or higher identity thereto; and

the second light chain polypeptide chain comprises an amino acid sequence set forth in SEQ ID NO: 68 or an amino acid sequence having at least 90%, 92%, 95%, 97%, 98%, 99% or higher identity thereto.

### Format_7

In some embodiments, when q = 0 and p = 2, the antibody molecule of the present invention has the structure: (M1:M2-(X1)p)-Fc::Fc. Therefore, there is only conjugate component X1 conjugated to the antibody arm on the antibody molecule. In the antibody molecule, the antibody arms M1 and M2 comprise Fab antigen-binding sites that bind to a first antigen and a second antigen, respectively, and the conjugate component X1 comprises an scFv antigen-binding site that binds to a third antigen, wherein the first, second and third antigens are different from each other and independently selected from: GPRC5D, BCMA and CD3.

Therefore, in some preferred embodiments, the present invention provides a trispecific Y-type antibody molecule having the structure:

(M1:M2-(X1)p)-Fc::Fc, (Format_7)

wherein p= 2,
M1 and M2 represent a first antibody arm and a second antibody arm of the antibody molecule, respectively, and M1 and M2 comprise Fab that binds to a first antigen and a second antigen, respectively;
Fc::Fc represents the stem of the antibody molecule, consisting of a first Fc domain and a second Fc domain which are paired and dimerized, wherein the first and second antibody arms are linked to the N-termini of the first Fc domain and the second Fc domain, either directly or via a linker peptide (preferably a hinge region), respectively;
X1 represents a conjugate component conjugated to the C-terminus of the antibody arm, wherein the conjugate component comprises scFv that binds to a third antigen, and X1 is conjugated to the C-termini of the Fab light chains of the first and the second antibody arms either directly or via a linker peptide,
the first, the second, and the third antigens are different from each other and are independently selected from GPRC5D, BCMA, and CD3.

In some embodiments, the present invention provides a trispecific antibody molecule having a structure of Format 7, wherein
(a): the antibody arm M1 and antibody arm M2 of the molecule comprise Fab that binds to GPRC5D and Fab that binds to CD3, respectively, the conjugate component X1 comprises scFv that binds to BCMA, and the molecule (e.g., the TS-F7-1, 2, 5 molecules in the examples) has the structure: (Fab_GPRC5D:Fab_CD3-(scFv_BCMA)₂)-Fc::Fc; or
(b): the antibody arm M1 and antibody arm M2 of the molecule comprise Fab that binds to GPRC5D and Fab that binds to BCMA, respectively, the conjugate component X1 comprises scFv that binds to CD3, and the molecule (e.g., the TS-F7-3, 4 molecules in the examples) has the structure: (Fab_GPRC5D:Fab_BCMA-(scFv_CD3)₂)-Fc::Fc; or
(c) the antibody arm M1 and antibody arm M2 of the molecule comprise Fab that binds to BCMA and Fab that binds to CD3, respectively, the conjugate component X1 comprises scFv that binds to GPRC5D, and the molecule has the structure: (Fab_BCMA:Fab_CD3-(scFv_GPRC5D)₂)-Fc::Fc.

In any one of the embodiments of the antibody molecule of Format_7 of the present invention described above, the Fab or scFv that binds to GPRC5D may be any suitable Fab or scFv that specifically binds to GPRC5D, such as the GPRC5D antigen-binding site of the present invention having an scFv or Fab structure described above, particularly scFv or Fab having the VH and VL amino acid sequences described above. In any one of the embodiments of the antibody molecule of Format_7 of the present invention described above, Fab or scFv that binds to BCMA may be any suitable Fab or scFv that specifically binds to BCMA, such as the BCMA antigen-binding site of the present invention having an scFv or Fab structure described above, particularly scFv or Fab having VH and VL amino acid sequences described above.

In any one of the embodiments of the antibody molecule of Format_7 of the present invention described above, the antigen-binding site that binds to CD3 may be any suitable scFv or Fab that specifically binds to CD3, such as the CD3 antigen-binding site of the present invention having an scFv or Fab structure described above, particularly scFv or Fab having VH and VL amino acid sequences described above.

In any one of the embodiments of the antibody molecule of Format_7 of the present invention described above, the antibody molecule may comprise any antibody stem structure of the present invention that is suitable for Format_7 described above. For example, in the asymmetric trispecific antibody molecule of Format_7 of the present invention, preferably, in order to promote correct pairing of the polypeptide chains of the antibody molecule, mutations that facilitate heterodimerization of the first and second Fc domains, particularly complementary "knob-in-hole" mutations, may be introduced into the first and the second Fc domains of the stem, and for example, a knob mutation is introduced into the first Fc domain and a complementary hole mutation is introduced into the second Fc domain, or vice versa, so that the two Fc domains of the antibody molecule may be paired to form a "knob-in-hole" stable association. According to the expected use, the first and/or second Fc domains of the antibody molecule of Format_7 of the present invention preferably may further comprise a mutation that affects the effector function of the antibody, such as a mutation that decreases ADCC activity, e.g., an LALA mutation.

In some embodiments, the trispecific antibody molecule of Format_7 of the present invention comprises or consists of a first heavy chain polypeptide chain, a first light chain polypeptide chain, a second heavy chain polypeptide chain and a second light chain polypeptide chain, wherein
the first heavy chain polypeptide chain comprises a heavy chain variable domain VH, an immunoglobulin CH1 domain and an Fc domain from the N-terminus to the C-terminus;
the first light chain polypeptide chain comprises a light chain variable domain VL and an immunoglobulin CL domain from the N-terminus to the C-terminus;
the second heavy chain polypeptide chain comprises a heavy chain variable domain VH, an immunoglobulin CH1 domain and an Fc domain from the N-terminus to the C-terminus; and
the second light chain polypeptide chain comprises a light chain variable domain VL and an immunoglobulin CL domain from the N-terminus to the C-terminus;
and wherein the first light chain polypeptide chain and the second light chain polypeptide chain further comprise scFv domains conjugated to the C-termini of the CL domains thereof, either directly or preferably via a linker peptide (e.g., (G4S)2 or TS(G4S)2);
wherein,
VH-CH1 of the first heavy chain polypeptide chain pair and VL-CL of the first light chain polypeptide chain are paired to form a first antibody arm (M1) that binds to a first antigen;
VH-CH1 of the second heavy chain polypeptide chain and VL-CL of the second light chain polypeptide chain are paired to form a second antibody arm (M2) that binds to a second antigen;
the Fc domain of the first heavy chain polypeptide chain and the Fc domain of the second heavy chain polypeptide chain are paired and dimerized to form a stem of the antibody (Fc::Fc); and
the scFv domain conjugated to the C-termini of the first light chain polypeptide chain and the second light chain polypeptide chain form a conjugate component (X1) that binds to a third antigen,
wherein the first, second and third antigens are different and independently selected from: GPRC5D, BCMA, and CD3.

In one embodiment, the antibody arm M1 binds to GPRC5D, the second antibody arm M2 binds to CD3, the conjugate component X1 binds to BCMA, and therefore,
- the first heavy chain polypeptide chain and the first light chain polypeptide chain comprise VH and VL amino acid sequences that specifically bind to a Fab antigen-binding site of GPRC5D at the N-terminus, respectively, and
- the second heavy chain polypeptide chain and the second light chain polypeptide chain comprise VH and VL amino acid sequences that specifically bind to a Fab antigen-binding site of CD3 at the N-terminus, respectively,
and the first light chain polypeptide chain and the second light chain polypeptide chain further comprise amino acid sequences of scFv that specifically binds to BCMA at the C-terminus.

In one embodiment, the antibody arm M1 binds to BCMA, the second antibody arm M2 binds to CD3, the conjugate component X1 binds to GPRC5D, and therefore,
- the first heavy chain polypeptide chain and the first light chain polypeptide chain comprise VH and VL amino acid sequences that specifically bind to a Fab antigen-binding site of BCMA at the N-terminus, respectively, and
- the second heavy chain polypeptide chain and the second light chain polypeptide chain comprise VH and VL amino acid sequences that specifically bind to a Fab antigen-binding site of CD3 at the N-terminus, respectively,
and the first light chain polypeptide chain and the second light chain polypeptide chain further comprise amino acid sequences of scFv that specifically binds to GPRC5D at the C-terminus.

In one embodiment, the antibody arm M1 binds to GPRC5D, the second antibody arm M2 binds to BCMA, the conjugate component X1 binds to CD3, and therefore,
- the first heavy chain polypeptide chain and the first light chain polypeptide chain comprise VH and VL amino acid sequences that specifically bind to a Fab antigen-binding site of GPRC5D at the N-terminus, respectively, and
- the second heavy chain polypeptide chain and the second light chain polypeptide chain comprise VH and VL amino acid sequences that specifically bind to a Fab antigen-binding site of BCMA at the N-terminus, respectively,
and the first light chain polypeptide chain and the second light chain polypeptide chain further comprise amino acid sequences of scFv that specifically binds to CD3 at the C-terminus.

In a preferred embodiment, Fab or scFv that specifically binds to GPRC5D comprises VH and VL selected from the following groups:
(a) VH comprising HCDR1-3 sequences set forth in SEQ ID NOs: 1-3 and VL comprising LCDR1-3 sequences set forth in SEQ ID NOs: 4-6;
(b) VH comprising HCDR1-3 sequences set forth in SEQ ID NOs: 7-8 and VL comprising LCDR1-3 sequences set forth in SEQ ID NOs: 10-12;
(c) VH comprising HCDR1-3 sequences set forth in SEQ ID NOs: 13-15 and VL comprising LCDR1-3 sequences set forth in SEQ ID NOs: 16-18; or
(d) VH comprising HCDR1-3 sequences set forth in SEQ ID NOs: 19-21 and VL comprising LCDR1-3 sequences set forth in SEQ ID NOs: 22-24.

Preferably, the Fab and the scFv comprise VH and VL amino acid sequence pairs selected from the following groups: SEQ ID NOs: 25/26, SEQ ID NOs: 27/28, SEQ ID NOs: 29/30, and SEQ ID NOs: 31/32, and preferably, when the scFv is dsscFv, the antigen-binding site further comprises a cysteine replacement introduced into the VH and VL sequences, such as a cysteine replacement at position 44 in the VH sequence and position 100 in the VL sequence.

In a preferred embodiment, the Fab or scFv that specifically binds to CD3 comprises VH and VL selected from the following groups:
(a) VH comprising HCDR1-3 sequences set forth in SEQ ID NOs: 41-43 and VL comprising LCDR1-3 sequences set forth in SEQ ID NOs: 44-46; or
(b) VH comprising HCDR1-3 sequences set forth in SEQ ID NOs: 41, 47, and 43, and VL comprising LCDR1-3 sequences set forth in SEQ ID NOs: 44-46.

Preferably, the Fab and the scFv comprise VH and VL amino acid sequence pairs selected from the following groups: SEQ ID NOs: 48/49, SEQ ID NOs: 50/49, and more preferably SEQ ID NOs: 48/49, and preferably, when the scFv is dsscFv, the antigen-binding site further comprises a cysteine replacement introduced into the VH and VL sequences, such as a cysteine replacement at position 44 in the VH sequence and position 100 in the VL sequence.

In a preferred embodiment, the Fab or scFv that specifically binds to BCMA comprises the following VH and VL:
VH comprising HCDR1-3 sequences set forth in SEQ ID NOs: 33-35 and VL comprising LCDR1-3 sequences set forth in SEQ ID NOs: 36-38.

Preferably, the Fab and the scFv comprise a VH and VL amino acid sequence pair: SEQ ID NOs: 39/40, and preferably, when the scFv is dsscFv, the antigen-binding site further comprises a cysteine replacement introduced into the VH and VL sequences, such as a cysteine replacement at position 44 in the VH sequence and position 100 in the VL sequence.

In some embodiments, the first light chain polypeptide chain and the second light chain polypeptide chain comprise a kappa light chain constant domain CLκ. In some embodiments, the first light chain polypeptide chain and the second light chain polypeptide chain comprise a lamda light chain constant domain CL_{λ}. In a specific embodiment, the light chain constant domain CLx comprises an amino acid sequence set forth in SEQ ID NO: 105 or an amino acid sequence having at least 90%, 92%, 95%, 97%, 98%, 99% or higher identity thereto. In a specific embodiment, the light chain constant domain CL_{λ} comprises an amino acid sequence set forth in SEQ ID NO: 106 or an amino acid sequence having at least 90%, 92%, 95%, 97%, 98%, 99% or higher identity thereto.

In some preferred embodiments, the first heavy chain polypeptide chain and the second heavy chain polypeptide chain comprise a heavy chain constant region CH1 derived from human IgG1, and preferably comprise an amino acid sequence set forth in SEQ ID NO: 104 or an amino acid sequence having at least 90%, 92%, 95%, 97%, 98%, 99% or higher identity thereto.

In some preferred embodiments, the first heavy chain polypeptide chain and the second heavy chain polypeptide chain comprise an Fc domain containing a knob or complementary hole mutation, respectively. In one specific embodiment, the Fc domain containing a knob mutation comprises T366W; and the Fc domains containing a complementary hole mutation comprises T366S, L368A and Y407V mutations. In one specific embodiment, the Fc domain comprising a knob mutation comprises an amino acid sequence selected from SEQ ID NOs: 102 and 107 or an amino acid sequence having at least 90%, 92%, 95%, 97%, 98%, 99% or higher identity thereto; and the Fc domain containing a complementary hole mutation comprises an amino acid sequence set forth in SEQ ID NO: 103 or an amino acid sequence having at least 90%, 92%, 95%, 97%, 98%, 99% or higher identity thereto.

In some embodiments, the present invention provides a trispecific antibody molecule having a structure of Format 7, and the trispecific antibody molecule comprises or consists of a first heavy chain polypeptide chain, a first light chain polypeptide chain, a second heavy chain polypeptide chain and a second light chain polypeptide chain, wherein
the first heavy chain polypeptide chain comprises an amino acid sequence set forth in SEQ ID NO: 87 or an amino acid sequence having at least 90%, 92%, 95%, 97%, 98%, 99% or higher identity thereto;
the first light chain polypeptide chain comprises an amino acid sequence set forth in SEQ ID NO: 88 or an amino acid sequence having at least 90%, 92%, 95%, 97%, 98%, 99% or higher identity thereto;
the second heavy chain polypeptide chain comprises an amino acid sequence set forth in SEQ ID NO: 77 or 78 or an amino acid sequence having at least 90%, 92%, 95%, 97%, 98%, 99% or higher identity thereto; and
the second light chain polypeptide chain comprises an amino acid sequence set forth in SEQ ID NO: 89 or an amino acid sequence having at least 90%, 92%, 95%, 97%, 98%, 99% or higher identity thereto.

In some embodiments, the present invention provides a trispecific antibody molecule having a structure of Format 7, and the trispecific antibody molecule comprises or consists of a first heavy chain polypeptide chain, a first light chain polypeptide chain, a second heavy chain polypeptide chain and a second light chain polypeptide chain, wherein
the first heavy chain polypeptide chain comprises an amino acid sequence set forth in SEQ ID NO: 90 or an amino acid sequence having at least 90%, 92%, 95%, 97%, 98%, 99% or higher identity thereto;
the first light chain polypeptide chain comprises an amino acid sequence set forth in SEQ ID NO: 91 or an amino acid sequence having at least 90%, 92%, 95%, 97%, 98%, 99% or higher identity thereto;
the second heavy chain polypeptide chain comprises an amino acid sequence set forth in SEQ ID NO: 77 or an amino acid sequence having at least 90%, 92%, 95%, 97%, 98%, 99% or higher identity thereto; and
the second light chain polypeptide chain comprises an amino acid sequence set forth in SEQ ID NO: 89 or an amino acid sequence having at least 90%, 92%, 95%, 97%, 98%, 99% or higher identity thereto.

In some embodiments, the present invention provides a trispecific antibody molecule having a structure of Format 7, and the trispecific antibody molecule comprises or consists of a first heavy chain polypeptide chain, a first light chain polypeptide chain, a second heavy chain polypeptide chain and a second light chain polypeptide chain, wherein
the first heavy chain polypeptide chain comprises an amino acid sequence set forth in SEQ ID NO: 92 or an amino acid sequence having at least 90%, 92%, 95%, 97%, 98%, 99% or higher identity thereto;
the first light chain polypeptide chain comprises an amino acid sequence set forth in SEQ ID NO: 93 or 96 or an amino acid sequence having at least 90%, 92%, 95%, 97%, 98%, 99% or higher identity thereto;
the second heavy chain polypeptide chain comprises an amino acid sequence set forth in SEQ ID NO: 94 or an amino acid sequence having at least 90%, 92%, 95%, 97%, 98%, 99% or higher identity thereto; and
the second light chain polypeptide chain comprises an amino acid sequence set forth in SEQ ID NO: 95 or 97 or an amino acid sequence having at least 90%, 92%, 95%, 97%, 98%, 99% or higher identity thereto.

### Format 1

In some embodiments, when p = 0 and q = 1, the antibody molecule of the present invention has the structure: (M1:M2)-Fc::Fc-(X2)q, wherein the antibody arms M1 and M2 and the conjugate component X2 comprise scFv antigen-binding sites that bind to a first antigen, a second antigen and a third antigen, respectively, wherein the first, the second and the third antigens are different and are independently selected from: GPRC5D, BCMA, and CD3.

Therefore, in some preferred embodiments, the present invention provides a trispecific Y-type antibody molecule having the structure:

(M1:M2)-Fc::Fc-(X2)q, (Format_1)

wherein q = 1,
M1 and M2 represent the first antibody arm and the second antibody arm of the antibody molecule, respectively, and M1 and M2 comprise scFv that binds to a first antigen and a second antigen, respectively, Fc::Fc represents the stem of the antibody molecule, consisting of a first Fc domain and a second Fc domain which are paired and dimerized, wherein the first and second antibody arms are linked to the N-termini of the first Fc domain and the second Fc domain, either directly or via a linker peptide (preferably a hinge region), respectively;
X2 represents a conjugate component conjugated to the C-terminus of the stem, wherein the conjugate component comprises an scFv that binds to a third antigen, wherein X2 is conjugated to the C-terminus of the first Fc domain or to the C-terminus of the second Fc domain, either directly or via a linker peptide, the first, the second, and the third antigens are different from each other and are independently selected from GPRC5D, BCMA, and CD3.

In some embodiments, the present invention provides a trispecific antibody molecule having a structure of Format_1, wherein
(a): the antibody arm M1 and antibody arm M2 of the molecule comprise scFv that binds to GPRC5D and scFv that binds to CD3, respectively, the conjugate component X2 comprises scFv that binds to BCMA, and the molecule (e.g., the TS-F1-1 molecule in the example) has the structure: (scFv_GPRC5D:scFv_CD3)-Fc::Fc-(scFv_BCMA); or
(b): the antibody arm M1 and antibody arm M2 of the molecule comprise scFv that binds to BCMA and scFv that binds to CD3, respectively, the conjugate component X2 comprises scFv that binds to GPRC5D, and the molecule (e.g., the TS-F1-2 molecule in the example) has the structure: (scFv_BCMA:scFv_CD3)-Fc::Fc-(scFv_GPRC5D).

In some embodiments, the trispecific antibody molecule of Format_1 of the present invention comprises or consists of a first heavy chain polypeptide chain and a second heavy chain polypeptide chain, wherein,
the first heavy chain polypeptide chain comprises a first scFv and an Fc domain from N-terminus to C-terminus;
the second heavy chain polypeptide chain comprises a second scFv and an Fc domain from N-terminus to C-terminus;
and wherein the first heavy chain polypeptide chain or the second heavy chain polypeptide chain further comprises a third scFv domain conjugated to the C-terminus of an Fc domain thereof, either directly or preferably via a linker peptide (e.g., (G4S)2 or TS(G4S)2);
wherein,
the first scFv of the first heavy chain polypeptide chain forms a first antibody arm (M1) that binds to a first antigen;
the second scFv of the second heavy chain polypeptide chain forms a second antibody arm (M2) that binds to a second antigen;
the Fc domain of the first heavy chain polypeptide chain and the Fc domain of the second heavy chain polypeptide chain are paired and dimerized to form a stem of the antibody (Fc::Fc); and
the third scFv domain conjugated to the C-terminus of the first heavy chain polypeptide chain or the second heavy chain polypeptide chain forms a conjugate component (X2) that binds to a third antigen,
wherein the first, second and third antigens are different and independently selected from: GPRC5D, BCMA, and CD3.

In some embodiments, the present invention provides a trispecific antibody molecule having a structure of Format 1, and the trispecific antibody molecule comprises or consists of a first heavy chain polypeptide chain and a second heavy chain polypeptide chain, wherein
the first heavy chain polypeptide chain comprises an amino acid sequence set forth in SEQ ID NO: 61 or an amino acid sequence having at least 90%, 92%, 95%, 97%, 98%, 99% or higher identity thereto;
the second heavy chain polypeptide chain comprises an amino acid sequence set forth in SEQ ID NO: 62 or an amino acid sequence having at least 90%, 92%, 95%, 97%, 98%, 99% or higher identity thereto.

In some embodiments, the present invention provides a trispecific antibody molecule having a structure of Format 1, and the trispecific antibody molecule comprises or consists of a first heavy chain polypeptide chain and a second heavy chain polypeptide chain, wherein
the first heavy chain polypeptide chain comprises an amino acid sequence set forth in SEQ ID NO: 63 or an amino acid sequence having at least 90%, 92%, 95%, 97%, 98%, 99% or higher identity thereto;
the second heavy chain polypeptide chain comprises an amino acid sequence set forth in SEQ ID NO: 64 or an amino acid sequence having at least 90%, 92%, 95%, 97%, 98%, 99% or higher identity thereto.

### Format 6

In some embodiments, when p = 2 and q = 2, the antibody molecule of the present invention has the structure: (M1:M2-(X2)p)-Fc::Fc-(X2)q, wherein the antibody arms M1 and M2 comprise Fab antigen-binding sites that bind to a first antigen, the conjugate components X1 and X2 comprise scFv antigen-binding sites that bind to a second antigen and a third antigen, respectively, and the first antigen, the second antigen and the third antigen are different from each other and are independently selected from: GPRC5D, BCMA, and CD3.

Therefore, in some preferred embodiments, the present invention provides a trispecific symmetric Y-type antibody molecule, which has the structure:

(M1:M2-(X2)p)-Fc::Fc-(X2)q, (Format_6)

wherein p = 2 and q = 2,
M1 and M2 represent the first antibody arm and the second antibody arm of the antibody molecule, respectively, and M1 and M2 both comprise Fab that binds to a first antigen,
Fc::Fc represents the stem of the antibody molecule, consisting of a first Fc domain and a second Fc domain which are paired and dimerized, wherein the first and second antibody arms are linked to the N-termini of the first Fc domain and the second Fc domain, either directly or via a linker peptide (preferably a hinge region), respectively;
X1 and X2 represent conjugate components conjugated to the C-termini of the Fab light chains of the antibody arms or the C-terminus of the Fc domain of the stem, respectively, wherein the conjugate component X1 comprises scFv that binds to a second antigen, the conjugate component X2 comprises scFv that binds to a third antigen, and X1 and X2 are conjugated to the arms or the stem either directly or via a linker peptide,
the first, the second, and the third antigens are different from each other and are independently selected from GPRC5D, BCMA, and CD3.

In some embodiments, the present invention provides a trispecific antibody molecule having a structure of Format_6, wherein
(a): the antibody arm M1 and antibody arm M2 of the molecule comprise Fab that binds to GPRC5D, the conjugate component X1 comprises scFv that binds to CD3, the conjugate component X2 comprises scFv that binds to BCMA, and the molecule (e.g., the TS-F6-1, 2 molecules in the examples) has the structure: (Fab_GPRC5D:Fab_GPRC5D-(scFv_CD3)₂)-Fc::Fc-(scFv_BCMA)₂; or
(b): the antibody arm M1 and antibody arm M2 of the molecule comprise Fab that binds to BCMA, the conjugate component X1 comprises scFv that binds to CD3, the conjugate component X2 comprises scFv that binds to GPRC5D, and the molecule (e.g., the TS-F6-3, 4 molecules in the examples) has the structure: (Fab_BCMA:Fab_BCMA-(scFv_CD3)₂)-Fc::Fc-(scFv_GPRC5D)₂.

In some embodiments, the trispecific antibody molecule of Format_6 of the present invention comprises or consists of two identical heavy chain polypeptide chains and two identical light chain polypeptide chains, wherein
the heavy chain polypeptide chain comprises a heavy chain variable domain VH, an immunoglobulin CH1 domain, an Fc domain and a first scFv domain from N-terminus to C-terminus;
the light chain polypeptide chain comprises a light chain variable domain VL, an immunoglobulin CL domain and a second scFv domain from N-terminus to C-terminus;
the first and the second scFv domains of the heavy chain polypeptide chain and the light chain polypeptide chain are conjugated to the C-termini thereof, respectively, either directly or preferably via a linker peptide (e.g., (G4S)2 or TS(G4S)2);
wherein,
the VH-CH1 domain at the N-terminus of one heavy chain polypeptide chain and the VL-CH1 domain at the N-terminus of one light chain polypeptide chain are paired to form a first antibody arm (M1) that binds to a first antigen;
the other VH-CH1 domain at the N-terminus of one heavy chain polypeptide chain and the VL-CH1 domain at the N-terminus of one light chain polypeptide chain are paired to form a first antibody arm (M2) that binds to a second antigen;
the Fc domains of the two heavy chain polypeptide chains are paired and dimerized to form an antibody stem (Fc::Fc); and
the scFv domains conjugated to the C-termini of the two light chain polypeptide chains form a conjugate component (X1) that binds to a second antigen;
the scFv domains conjugated to the C-termini of the two heavy chain polypeptide chains form a conjugate component (X2) that binds to a third antigen;
wherein the first, second and third antigens are different and independently selected from: GPRC5D, BCMA, and CD3.

In some embodiments, the present invention provides a trispecific antibody molecule having a structure of Format_6, and the trispecific antibody molecule comprises or consists of two identical heavy chain polypeptide chains and two identical light chain polypeptide chains, wherein
the heavy chain polypeptide chain comprises an amino acid sequence set forth in SEQ ID NO: 81 or an amino acid sequence having at least 90%, 92%, 95%, 97%, 98%, 99% or higher identity thereto; and
the light chain polypeptide chain comprises an amino acid sequence set forth in SEQ ID NO: 82 or 83 or an amino acid sequence having at least 90%, 92%, 95%, 97%, 98%, 99% or higher identity thereto.

In some embodiments, the present invention provides a trispecific antibody molecule having a structure of Format_6, and the trispecific antibody molecule comprises or consists of two identical heavy chain polypeptide chains and two identical light chain polypeptide chains, wherein
the heavy chain polypeptide chain comprises an amino acid sequence set forth in SEQ ID NO: 84 or an amino acid sequence having at least 90%, 92%, 95%, 97%, 98%, 99% or higher identity thereto; and
the light chain polypeptide chain comprises an amino acid sequence set forth in SEQ ID NO: 85 or 86 or an amino acid sequence having at least 90%, 92%, 95%, 97%, 98%, 99% or higher identity thereto.

### III. GPRC5D antibody molecule of the present invention

In one aspect, the present invention provides an antibody or an antigen-binding fragment thereof that specifically binds to GPRC5D. In some preferred embodiments, the anti-GPRC5D antibody or the antigen-binding fragment thereof of the present invention comprises:
(i) HCDR1, 2, and 3 sequences of a heavy chain variable domain set forth in SEQ ID NO: 25, and LCDR1, 2, and 3 sequences of a light chain variable domain set forth in SEQ ID NO: 26, or
(ii) HCDR1, 2, and 3 sequences of a heavy chain variable domain set forth in SEQ ID NO: 27, and LCDR1, 2, and 3 sequences of a light chain variable domain set forth in SEQ ID NO: 28, or
(iii) HCDR1, 2, and 3 sequences of a heavy chain variable domain set forth in SEQ ID NO: 29, and LCDR1, 2, and 3 sequences of a light chain variable domain set forth in SEQ ID NO: 30, or
(iv) HCDR1, 2, and 3 sequences of a heavy chain variable domain set forth in SEQ ID NO: 31, and LCDR1, 2, and 3 sequences of a light chain variable domain set forth in SEQ ID NO: 32, or
(v) HCDR1, 2, and 3 sequences of a heavy chain variable domain set forth in SEQ ID NO: 98, and LCDR1, 2, and 3 sequences of a light chain variable domain set forth in SEQ ID NO: 99, or
(vi) HCDR1, 2, and 3 sequences of a heavy chain variable domain set forth in SEQ ID NO: 100, and LCDR1, 2, and 3 sequences of a light chain variable domain set forth in SEQ ID NO: 101.

In other embodiments, the GPRC5D antibody or the antigen-binding fragment thereof of the present invention comprises a combination of CDR sequences selected from the followings:
(i) HCDR1, 2, and 3 sequences of a heavy chain variable domain and LCDR1, 2, and 3 sequences of a light chain variable domain set forth in SEQ ID NOs: 1-6, respectively, or
(ii) HCDR1, 2, and 3 sequences of a heavy chain variable domain and LCDR1, 2, and 3 sequences of a light chain variable domain set forth in SEQ ID NOs: 7-12, respectively, or
(iii) HCDR1, 2, and 3 sequences of a heavy chain variable domain and LCDR1, 2, and 3 sequences of a light chain variable domain set forth in SEQ ID NOs: 13-18, respectively, or
(iv) HCDR1, 2, and 3 sequences of a heavy chain variable domain and LCDR1, 2, and 3 sequences of a light chain variable domain set forth in SEQ ID NOs: 19-24, respectively, or
(v) heavy chain variable domain HCDR1, 2 and 3 sequences and light chain variable domain LCDR1, 2, and 3 sequences set forth in SEQ ID NOs: 13, 110 and 18, respectively.

In yet other embodiments, the GPRC5D antibody molecule or antigen-binding fragment thereof of the present invention comprises a combination of VH and VL amino acid sequences selected from the followings:
(a) a heavy chain variable domain comprising an amino acid sequence set forth in SEQ ID NO: 25 or an amino acid sequence having at least 80%, 85%, 90%, 95%, 97%, 98%, or 99% sequence identity thereto; and a light chain variable domain comprising an amino acid sequence set forth in SEQ ID NO: 26 or an amino acid sequence having at least 80%, 85%, 90%, 95%, 97%, 98%, or 99% sequence identity thereto; or
(b) a heavy chain variable domain comprising an amino acid sequence set forth in SEQ ID NO: 27 or an amino acid sequence having at least 80%, 85%, 90%, 95%, 97%, 98%, or 99% sequence identity thereto; and a light chain variable domain comprising an amino acid sequence set forth in SEQ ID NO: 28 or an amino acid sequence having at least 80%, 85%, 90%, 95%, 97%, 98%, or 99% sequence identity thereto; or
(c) a heavy chain variable domain comprising an amino acid sequence set forth in SEQ ID NO: 29 or an amino acid sequence having at least 80%, 85%, 90%, 95%, 97%, 98%, or 99% sequence identity thereto; and a light chain variable domain comprising an amino acid sequence set forth in SEQ ID NO: 30 or an amino acid sequence having at least 80%, 85%, 90%, 95%, 97%, 98%, or 99% sequence identity thereto; or
(d) a heavy chain variable domain comprising an amino acid sequence set forth in SEQ ID NO: 31 or an amino acid sequence having at least 80%, 85%, 90%, 95%, 97%, 98%, or 99% sequence identity thereto; and a light chain variable domain comprising an amino acid sequence set forth in SEQ ID NO: 32 or an amino acid sequence having at least 80%, 85%, 90%, 95%, 97%, 98%, or 99% sequence identity thereto;
(e) a heavy chain variable domain comprising an amino acid sequence set forth in SEQ ID NO: 98 or an amino acid sequence having at least 80%, 85%, 90%, 95%, 97%, 98%, or 99% sequence identity thereto; and a light chain variable domain comprising an amino acid sequence set forth in SEQ ID NO: 99 or an amino acid sequence having at least 80%, 85%, 90%, 95%, 97%, 98%, or 99% sequence identity thereto; or
(f) a heavy chain variable domain comprising an amino acid sequence set forth in SEQ ID NO: 100 or an amino acid sequence having at least 80%, 85%, 90%, 95%, 97%, 98%, or 99% sequence identity thereto; and a light chain variable domain comprising an amino acid sequence set forth in SEQ ID NO: 101 or an amino acid sequence having at least 80%, 85%, 90%, 95%, 97%, 98%, or 99% sequence identity thereto.

Preferably, the GPRC5D antibody molecule or antigen-binding fragment thereof of the present invention comprises a combination of VH and VL amino acid sequences selected from the followings:
(i) a heavy chain variable domain comprising an amino acid sequence set forth in SEQ ID NO: 25 and a light chain variable domain comprising an amino acid sequence set forth in SEQ ID NO: 26, or
(ii) a heavy chain variable domain comprising an amino acid sequence set forth in SEQ ID NO: 27 and a light chain variable domain comprising an amino acid sequence set forth in SEQ ID NO: 28, or
(iii) a heavy chain variable domain comprising an amino acid sequence set forth in SEQ ID NO: 29, and a light chain variable domain comprising an amino acid sequence set forth in SEQ ID NO: 30, or
(iv) a heavy chain variable domain comprising an amino acid sequence set forth in SEQ ID NO: 31 and a light chain variable domain comprising an amino acid sequence set forth in SEQ ID NO: 32;
(v) a heavy chain variable domain comprising an amino acid sequence set forth in SEQ ID NO: 98 and a light chain variable domain comprising an amino acid sequence set forth in SEQ ID NO: 99, or
(vi) a heavy chain variable domain comprising an amino acid sequence set forth in SEQ ID NO: 100, and a light chain variable domain comprising an amino acid sequence set forth in SEQ ID NO: 101.

In some embodiments, the GPRC5D antibody of the present invention is a monospecific antibody, a bispecific antibody or a trispecific antibody. In some preferred embodiments, the GPRC5D antibody of the present invention is a bispecific antibody, which further comprises an antigen-binding site that binds to CD3, such as the CD3 antigen-binding site of the present invention described above. In other preferred embodiments, the GPRC5D antibody of the present invention is a trispecific antibody, which further comprises an antigen-binding site that binds to BCMA (e.g., the BCMA antigen-binding site of the present invention described above) and an antigen-binding site that binds to CD3 (e.g., the CD3 antigen-binding site of the present invention described above).

In some embodiments, the GPRC5D antibody of the present invention is a bispecific antibody that binds to GPRC5D and CD3,
preferably, wherein the antibody comprises a VH and VL amino acid sequence pair that binds to CD3 and is selected from the following groups:
   (a) VH comprising HCDR1-3 sequences set forth in SEQ ID NOs: 41-43 and VL comprising LCDR1-3 sequences set forth in SEQ ID NOs: 44-46; or
   (b) VH comprising HCDR1-3 sequences set forth in SEQ ID NOs: 47, 42, and 43, and VL comprising LCDR1-3 sequences set forth in SEQ ID NOs: 44-46.
preferably, a VH and VL amino acid sequence pair selected from the following groups: SEQ ID NOs: 48/49 and SEQ ID NOs: 50/49,
optionally, the VH and VL sequences comprise cysteine replacements introduced, for example, cysteine replacements at position 44 in the VH sequence and position 100 in the VL sequence, and therefore a disulfide bond is formed between the VH and the VL.

Therefore, in a preferred embodiment, the present invention provides a bispecific antibody that binds to GPRC5D and CD3, wherein the antibody comprises a combination of VH and VL amino acid sequence pairs that bind to GPRC5D and VH and VL amino acid sequence pairs that bind to CD3, which is selected from the following groups:

| Combination | VH and VL amino acid sequence pair that binds to GPRC5D | VH and VL amino acid sequence pair that binds to CD3 |
|---|---|---|
| 1 | SEQ ID NO: 25 and SEQ ID NO: 26 | SEQ ID NO: 48 and SEQ ID NO: 49 |
| 2 | SEQ ID NO: 27 and SEQ ID NO: 28 | SEQ ID NO: 48 and SEQ ID NO: 49 |
| 3 | SEQ ID NO: 29 and SEQ ID NO: 30 | SEQ ID NO: 48 and SEQ ID NO: 49 |
| 4 | SEQ ID NO: 31 and SEQ ID NO: 32 | SEQ ID NO: 48 and SEQ ID NO: 49 |
| 5 | SEQ ID NO: 25 and SEQ ID NO: 26 | SEQ ID NO: 50 and SEQ ID NO: 49 |
| 6 | SEQ ID NO: 27 and SEQ ID NO: 28 | SEQ ID NO: 50 and SEQ ID NO: 49 |
| 7 | SEQ ID NO: 29 and SEQ ID NO: 30 | SEQ ID NO: 50 and SEQ ID NO: 49 |
| 8 | SEQ ID NO: 31 and SEQ ID NO: 32 | SEQ ID NO: 50 and SEQ ID NO: 49 |

The present invention further provides a variant of the above-mentioned bispecific antibody, wherein the variant comprises amino acid alterations, such as amino acid substitutions, deletions and/or insertions, in the VH and VL amino acid sequence pair that binds to GPRC5D and/or in the VH and VL amino acid sequence pair that binds to CD3, wherein the amino acid alterations do not affect the binding of the bispecific antibody to GPRC5D and CD3. In some embodiments, the variant comprises a combination of amino acid sequences selected from the followings,

| Combination | VH and VL amino acid sequence pair that binds to GPRC5D | VH and VL amino acid sequence pair that binds to CD3 |
|---|---|---|
| 1 | a VH amino acid sequence having at least 90%, 95%, 96%, 97%, 98%, or 99% identity to SEQ ID NO: 25, and a VL amino acid sequence having at least 90%, 95%, 96%, 97%, 98%, or 99% identity to SEQ ID NO: 26 | a VH amino acid sequence having at least 90%, 95%, 96%, 97%, 98%, or 99% identity to SEQ ID NO: 48, and a VL amino acid sequence having at least 90%, 95%, 96%, 97%, 98%, or 99% identity to SEQ ID NO: 49 |
| 2 | a VH amino acid sequence having at least 90%, 95%, 96%, 97%, 98%, or 99% identity to SEQ ID NO: 27, and a VL amino acid sequence having at least 90%, 95%, 96%, 97%, 98%, or 99% identity to SEQ ID NO: 28 | a VH amino acid sequence having at least 90%, 95%, 96%, 97%, 98%, or 99% identity to SEQ ID NO: 48, and a VL amino acid sequence having at least 90%, 95%, 96%, 97%, 98%, or 99% identity to SEQ ID NO: 49 |
| 3 | a VH amino acid sequence having at least 90%, 95%, 96%, 97%, 98%, or 99% identity to SEQ ID NO: 29, and a VL amino acid sequence having at least 90%, 95%, 96%, 97%, 98%, or 99% identity to SEQ ID NO: 30 | a VH amino acid sequence having at least 90%, 95%, 96%, 97%, 98%, or 99% identity to SEQ ID NO: 48, and a VL amino acid sequence having at least 90%, 95%, 96%, 97%, 98%, or 99% identity to SEQ ID NO: 49 |
| 4 | a VH amino acid sequence having at least 90%, 95%, 96%, 97%, 98%, or 99% identity to SEQ ID NO: 31, and a VL amino acid sequence having at least 90%, 95%, 96%, 97%, 98%, or 99% identity to SEQ ID NO: 32 | a VH amino acid sequence having at least 90%, 95%, 96%, 97%, 98%, or 99% identity to SEQ ID NO: 48, and a VL amino acid sequence having at least 90%, 95%, 96%, 97%, 98%, or 99% identity to SEQ ID NO: 49 |
| 5 | a VH amino acid sequence having at least 90%, 95%, 96%, 97%, 98%, or 99% identity to SEQ ID NO: 25, and a VL amino acid sequence having at least 90%, 95%, 96%, 97%, 98%, or 99% identity to SEQ ID NO: 26 | a VH amino acid sequence having at least 90%, 95%, 96%, 97%, 98%, or 99% identity to SEQ ID NO: 50, and a VL amino acid sequence having at least 90%, 95%, 96%, 97%, 98%, or 99% identity to SEQ ID NO: 49 |
| 6 | a VH amino acid sequence having at least 90%, 95%, 96%, 97%, 98%, or 99% identity to SEQ ID NO: 27, and a VL amino acid sequence having at least 90%, 95%, 96%, 97%, 98%, or 99% identity to SEQ ID NO: 28 | a VH amino acid sequence having at least 90%, 95%, 96%, 97%, 98%, or 99% identity to SEQ ID NO: 50, and a VL amino acid sequence having at least 90%, 95%, 96%, 97%, 98%, or 99% identity to SEQ ID NO: 49 |
| 7 | a VH amino acid sequence having at least 90%, 95%, 96%, 97%, 98%, or 99% identity to SEQ ID NO: 29, and a VL amino acid sequence having at least 90%, 95%, 96%, 97%, 98%, or 99% identity to SEQ ID NO: 30 | a VH amino acid sequence having at least 90%, 95%, 96%, 97%, 98%, or 99% identity to SEQ ID NO: 50, and a VL amino acid sequence having at least 90%, 95%, 96%, 97%, 98%, or 99% identity to SEQ ID NO: 49 |
| 8 | a VH amino acid sequence having at least 90%, 95%, 96%, 97%, 98%, or 99% identity to SEQ ID NO: 31, and a VL amino acid sequence having at least 90%, 95%, 96%, 97%, 98%, or 99% identity to SEQ ID NO: 32 | a VH amino acid sequence having at least 90%, 95%, 96%, 97%, 98%, or 99% identity to SEQ ID NO: 50, and a VL amino acid sequence having at least 90%, 95%, 96%, 97%, 98%, or 99% identity to SEQ ID NO: 49 |

In a preferred embodiment, the bispecific antibody of the present invention comprises cysteine replacements introduced into the VH and VL amino acid sequence pair that binds to GPRC5D and/or into the VH and VL amino acid sequence pair that binds to CD3, such as cysteine replacements at position 44 in the VH sequence and position 100 in the VL sequence, and therefore a disulfide bond is formed between VH and VL.

The present invention further provides a fusion protein and immunoconjugate (e.g., conjugated to a toxin or a small chemical molecule), as well as a pharmaceutical composition and a pharmaceutical combination comprising the GPRC5D antibody of the present invention. In the pharmaceutical composition or the pharmaceutical combination, the antibody of the present invention may comprise another therapeutic agent, e.g., another therapeutic agent that may be used for the expected use of the antibody of the present invention, such as a chemotherapeutic agent, a radiotherapeutic agent and an anti-tumor molecule.

### IV. Production and purification of the antibody molecule of the present invention

In yet another aspect, the present invention provides a method for producing the antibody molecule of the present invention, which comprises: culturing a host cell comprising a polynucleotide encoding polypeptide chains under conditions suitable for the expression of the polypeptide chains of the antibody; and assembling the polypeptide chains to produce the antibody under conditions suitable for the assembly of the polypeptide chains into the antibody molecule.

When the antibody molecule of the present invention is an asymmetric antibody molecule, e.g., a trispecific antibody molecule comprising a first heavy chain polypeptide chain, a first light chain polypeptide chain, a second heavy chain polypeptide chain and a second light chain polypeptide chain, such as the antibody molecules of Format_2 and Format 7, preferably, the method for producing the antibody molecule of the present invention comprises the steps of: (i) culturing a host cell comprising a polynucleotide encoding a first heavy chain and a first light chain under conditions suitable for the expression of the first heavy chain polypeptide chain and the first light chain polypeptide chain of the antibody molecule to produce a first parent protein; (ii) culturing a host cell comprising a polynucleotide encoding a second heavy chain and a second light chain under conditions suitable for the expression of the second heavy chain polypeptide chain and the second light chain polypeptide chain of the antibody molecule to produce a second parent protein; and (iii) mixing the first parent protein and the second parent protein at an equimolar ratio, and placing the mixture under suitable redox conditions *in vitro* to make the mixture assembled into the antibody. In one specific embodiment, step (iii) comprises adding glutathione (GSH) to the mixed solution of the purified first and second parent proteins, wherein preferably, the GSH/protein molar ratio is controlled to 500-700 folds. In a specific embodiment, step (iii) further comprises performing autoxidation for a period of time (e.g., 2-3 h) after the removal of GSH.

The polypeptide chains of the antibody molecule of the present invention can be obtained, for example, by solid-state peptide synthesis (e.g., Merrifield solid-phase synthesis) or recombinant production. For recombinant production, polynucleotides encoding any one polypeptide chain and/or more polypeptide chains of the antibody molecule are isolated and inserted into one or more vectors for further cloning and/or expression in host cells. The polynucleotides can be easily isolated and sequenced using conventional methods. In one embodiment, provided is a vector, preferably an expression vector, comprising one or more polynucleotides of the present invention.

Methods known to those skilled in the art can be used to construct expression vectors. The expression vector includes, but is not limited to, a virus, a plasmid, a cosmid, a λ phage or a yeast artificial chromosome (YAC). Once the expression vector comprising one or more polynucleotides of the present invention has been prepared for expression, the expression vector can be transfected or introduced into suitable host cells. Various techniques can be used for this purpose, for example, protoplast fusion, calcium phosphate precipitation, electroporation, retroviral transduction, viral transfection, biolistics, liposome-based transfection, or other conventional techniques.

In one embodiment, provided is a host cell comprising one or more polynucleotides of the present invention. In some embodiments, provided is a host cell comprising the expression vector of the present invention. As used herein, the term "host cell" refers to any kind of cell system that can be engineered to produce the antibody molecule of the present invention. Host cells suitable for replicating and supporting the expression of the antibody molecule of the present invention are well-known in the art. Such cells can be transfected or transduced with a specific expression vector as needed, and a large number of cells comprising vectors can be cultivated and then seeded in a large-scale fermentor, so as to obtain sufficient antibody molecules of the present invention for clinical application. Suitable host cells include prokaryotic microorganisms such as E. *coli,* eukaryotic microorganisms such as filamentous fungi or yeast, or various eukaryotic cells such as Chinese hamster ovary cells (CHO) and insect cells. A mammalian cell line suitable for suspension growth may be used. Examples of useful mammalian host cell lines include monkey kidney CV1 line (COS-7) transformed by SV40; human embryonic kidney line (HEK 293 or 293F cells), baby hamster kidney cells (BHK), monkey kidney cells (CV1), African green monkey kidney cells (VERO-76), human cervical cancer cells (HELA), canine kidney cells (MDCK), buffalo rat liver cells (BRL 3A), human lung cells (W138), human liver cells (Hep G2), CHO cells, NSO cells, and myeloma cell lines such as YO, NS0, P3X63 and Sp2/0. For reviews of mammalian host cell lines suitable for protein production, see, e.g., Yazaki and Wu, Methods in Molecular Biology, vol. 248 (edited by B. K. C. Lo, Humana Press, Totowa, NJ), pp. 255-268 (2003). In a preferred embodiment, the host cell is a CHO, HEK293 or NSO cell.

The antibody molecule prepared as described herein can be purified by known prior art such as high performance liquid chromatography, ion exchange chromatography, gel electrophoresis, affinity chromatography, and size exclusion chromatography. The actual conditions used to purify a particular protein further depend on factors such as net charge, hydrophobicity and hydrophilicity, which would have been apparent to those skilled in the art.

The purity of the antibody molecule of the present invention can be determined by any one of a variety of well-known analytical methods including size exclusion chromatography, gel electrophoresis, high performance liquid chromatography, and the like. The antibody molecule provided herein can be identified, screened, or characterized for its physical/chemical properties and/or bioactivity through a variety of assays known in the art.

### V. Pharmaceutical composition, pharmaceutical combination and kit

In one aspect, the present invention provides a composition, e.g., a pharmaceutical composition comprising the antibody molecule described herein formulated together with a pharmaceutically acceptable carrier. As used herein, the "pharmaceutically acceptable carrier" includes any and all solvents, dispersion media, isotonic agents and absorption delaying agents, and the like that are physiologically compatible. The pharmaceutical composition of the present invention is suitable for intravenous, intramuscular, subcutaneous, parenteral, rectal, spinal or epidermal administration (e.g., by injection or infusion). In some embodiments, the antibody molecule of the present invention is the only active ingredient in the pharmaceutical composition. In other embodiments, the pharmaceutical composition may comprise the antibody molecule described herein and more than one therapeutic agents.

In another aspect, the present invention further provides a pharmaceutical combination comprising the antibody molecule described herein and more than one therapeutic agents.

The therapeutic agent suitable for use in the pharmaceutical composition and the pharmaceutical combination of the present invention may be selected from any one of the following categories (i)-(iii): (i) drugs that enhance antigen presentation (e.g., tumor antigen presentation); (ii) drugs that enhance effector cell responses (e.g., activation and/or mobilization of B cells and/or T cells); (iii) drugs that reduce immunosuppression; and (iv) drugs that have anti-tumor effects.

The compositions of the present invention may be in a variety of forms. These forms include, for example, liquid, semi-solid and solid dosage forms, such as liquid solutions (e.g., injectable solutions and infusible solutions), dispersions or suspensions, liposomes, and suppositories. The preferred form depends on the intended mode of administration and therapeutic use. Commonly preferred compositions are in the form of injectable solutions or infusible solutions. The preferred mode of administration is parenteral (e.g., intravenous, subcutaneous, intraperitoneal (i.p.) and intramuscular) injection. In one preferred embodiment, the antibody molecule is administered by intravenous infusion or injection. In another preferred embodiment, the antibody molecule is administered by intramuscular, intraperitoneal or subcutaneous injection.

As used herein, the phrases "parenteral administration" and "administered parenterally" mean modes of administration other than enteral and topical administration, typically by injection, and include, but are not limited to, intravenous, intramuscular, intra-arterial, intradermal, intraperitoneal, transtracheal, subcutaneous injection and infusion.

Therapeutic compositions generally should be sterile and stable under the conditions of manufacture and storage. The compositions can be prepared into solutions, microemulsions, dispersions, liposomes, or lyophilized forms. Sterile injectable solutions can be prepared by adding a required amount of an active compound (i.e., antibody molecule) to a suitable solvent, and then filtering and disinfecting the resulting mixture. Generally, dispersions are prepared by incorporating the active compound into a sterile vehicle, which comprises a basic dispersion medium and other ingredients. Coating agents such as lecithin can be used. In the case of dispersions, the proper fluidity of a solution can be maintained by using a surfactant. Prolonged absorption of injectable compositions can be caused by including in the compositions a substance that delays absorption such as monostearate and gelatin.

In certain embodiments, the antibody molecule of the present invention can be administered orally, such as administered orally with an inert diluent or an edible carrier. The antibody molecule of the present invention can also be encapsulated in gelatin capsules with hard or soft shells, compressed into tablets, or incorporated directly into diets of a subject. For oral therapeutic administration, the compound can be incorporated with excipients and used in the form of ingestible tablets, buccal tablets, troches, capsules, elixirs, suspensions, syrups, wafers, and the like. In order to administer the antibody molecule of the present invention by a method other than parenteral administration, it may be necessary to coat the antibody molecule with, or administer the antibody molecule in combination with, a material preventing inactivation. Therapeutic compositions can also be administered using medical devices known in the art.

The pharmaceutical composition of the present invention may comprise a "therapeutically effective amount" or a "prophylactically effective amount" of the antibody molecule of the present invention. "Therapeutically effective amount" refers to an amount effective to achieve a desired therapeutic result at a necessary dose for a necessary period of time. The therapeutically effective amount can be varied according to a variety of factors such as disease state, age, gender, and weight of the individual. The therapeutically effective amount is an amount in which any toxic or harmful effect is outweighed by the therapeutically beneficial effect. The "therapeutically effective amount" preferably inhibits a measurable parameter (e.g., tumor growth rate) by at least about 20%, more preferably at least about 40%, even more preferably at least about 60%, and still more preferably at least about 80%, relative to untreated subjects. The ability of the antibody molecule of the present invention to inhibit a measurable parameter (e.g., tumor volume) can be evaluated in an animal model system that predicts efficacy in human tumors.

"Prophylactically effective amount" refers to an amount effective to achieve a desired prophylactic result at a necessary dose for a necessary period of time. Generally, since a prophylactic dose is used in subjects before or at an earlier stage of a disease, the prophylactically effective amount will be less than the therapeutically effective amount.

A kit comprising the antibody molecule described herein is also within the scope of the present invention. The kit may include one or more other elements, including, for example, a package insert; other reagents, such as a label or a reagent for coupling; a pharmaceutically acceptable carrier; and a device or other materials for administration to a subject.

### V. Use and method of the molecule of the present invention

In one aspect, the present invention provides *in vivo* and *in vitro* use and method for the use of the antibody molecule of the present invention.

In some embodiments, the use and the method of the present invention involve the application of the antibody molecule of the present invention *in vivo* and/or *in vitro* in:
- binding to a GPRC5D antigen, including a GPRC5D antigen expressed on the surfaces of cells, with high affinity, e.g., with KD less than 10 nM;
- targeting T cells (e.g., CD4+ and/or CD8+ T cells) to cells that express GPRC5D on the surfaces, in particular GPRC5D-positive tumor cells;
- targeting T cells (e.g., CD4+ and/or CD8+ T cells) to cells that express BCMA on the surfaces, in particular BCMA-positive tumor cells;
- activating the CD3 downstream signaling pathways of T cells;
- mediating the killing effects of T cells on GPRC5D-positive and/or BCMA-positive tumor cells;
- inducing T cells to release cytokines such as TNF-α, IFN-γ and IL-2;
- inhibiting or killing GPRC5D-positive and/or BCMA-positive tumor cells, or
- treating GPRC5D-positive and/or BCMA-positive multiple myeloma, e.g., treating a BCMA-positive patient population, a GPRC5D-positive patient population, and a GPRC5D-positive patient population in which BCMA is lost after the binding of an anti-BCMA molecule; and
- avoiding tumor recurrence mediated by BCMA escape, such as recurrence of multiple myeloma.

In some embodiments, the antibody molecule of the present invention or the pharmaceutical composition comprising the antibody molecule of the present invention is used as a drug for the treatment and/or prevention of a disease in an individual or as a diagnostic tool for a disease; preferably, the individual is a mammal, and more preferably a human.

In some embodiments, the present invention provides a method for treating cancer using the antibody molecule of the present invention, in particular the trispecific antibody molecule, and use thereof, wherein the cancer may, for example, be selected from multiple myeloma, melanoma and B-cell lymphoma. Preferably, the cancer is multiple myeloma. By having antigen-binding specificity against BCMA and GPRC5D, the trispecific antibody molecule of the present invention can have a broader patient population for cancer treatment compared to BCMA/CD3-targeting bispecific antibodies and GPRC5D/CD3-targeting bispecific antibodies. In some embodiments, the present invention provides a method for treating BCMA-negative cancer or cancer with low BCMA expression using the trispecific antibody molecule of the present invention. In some embodiments, the present invention provides use of the trispecific antibody molecule of the present invention in the treatment of a GPRC5D/CD3-double-positive cancer.

In one aspect, the present invention provides a diagnostic method for detecting the presence of related antigens in a biological sample, such as serum, semen, urine or tissue biopsy samples (e.g., from a hyperproliferative or cancerous lesion), *in vitro* or *in vivo.* The diagnostic method comprises: (i) contacting a sample (and optionally a control sample) with the antibody molecule as described herein or administering the antibody molecule to a subject under conditions that allow interactions, and (ii) detecting the formation of a complex between the antibody molecule and the sample (and optionally the control sample). The formation of a complex indicates the presence of the related antigen and may show the suitability or need for the treatment and/or prevention described herein.

In some embodiments, the related antigen is detected prior to the treatment, e.g., prior to the initial treatment or prior to a certain treatment after a treatment interval. Detection methods that can be used include immunohistochemistry, immunocytochemistry, FACS, ELISA assays, PCR techniques (e.g., RT-PCR), or *in vivo* imaging techniques. Generally, antibody molecules used in *in vivo* and *in vitro* detection methods are directly or indirectly labeled with a detectable substance to facilitate the detection of bound or unbound conjugates. Suitable detectable substances include a variety of biologically active enzymes, prosthetic groups, fluorescent substances, luminescent substances, paramagnetic (e.g., nuclear magnetic resonance active) substances, and radioactive substances.

In some embodiments, the level and/or distribution of related antigens are/is determined *in vivo.* For example, the antibody molecule of the present invention labeled with a detectable substance is detected in a non-invasive manner, e.g., by using appropriate imaging techniques such as positron emission tomography (PET) scanning. In one embodiment, for example, the level and/or distribution of the related antigens are/is determined *in vivo* by detecting the antibody molecule of the present invention that is detectably labeled with a PET reagent (e.g., 18F-fluorodeoxyglucose (FDG)).

In one embodiment, the present invention provides a diagnostic kit comprising the antibody molecule described herein and a package insert.

The following examples are described to assist in understanding the present invention. The examples are not intended to be and should not be interpreted in any way as limiting the protection scope of the present invention.

### EXAMPLE

### Example 1. Preparation of Hybridoma Cells

### Construction of an overexpression cell line

Full-length sequences of human, monkey and mouse GPRC5D were inserted into PEE17.4 plasmids (Lonza, GS Xceed Expression System), respectively, the plasmids were transformed into host cells GS-CHO by an electrotransformation method, and a stable cell line overexpressing the GPRC5D protein was constructed by pressurized screening. Moreover, the full-length sequence of human GPRC5D was inserted into a pCHO1.0 vector, and the vector was transformed into host cells 293F to construct an overexpression cell strain 293F-huGPRC5D.

### Immunization

The full-length sequence of human GPRC5D was constructed into a pcDNA3.1 vector, and Balb\c and Harbour mice (purchased from Beijing Charles River Laboratory Animal Technology Co., Ltd.) were immunized with the plasmid, once every two weeks by intramuscular injection (50 µg plasmid per mouse), for a total of three times. Then, immunization was boosted with a GS-CHO cell strain overexpressing human GPRC5D once every two weeks by intraperitoneal injection (1 × 10⁷ per mouse), for a total of two times.

### Cell fusion

When the serum titer met the requirement, the spleen of a mouse was taken to prepare a B lymphocyte suspension. The B lymphocyte suspension was mixed with SP2/0 myeloma cells (ATCC) in a ratio of 1:2 to 1:1, and then electrofusion was performed. The fused cells were transferred from an electrode dish into a 50-mL centrifuge tube and diluted with an HAT medium to 1-2 × 10⁴ cells/mL, and 100 µL of cell suspension was added into each well of a 96-well plate. The screening medium was replaced on day 7 after fusion, the resulting product was detected by a flow cytometer (FACS) after 10 days (or longer, depending on the cell growth state) of culture, and positive clones were screened out.

### High-throughput screening of hybridoma cells

Hybridoma cells that specifically express an anti-GPRC5D antibody were screened out by a flow cytometer (FACS). The cells to be detected (293F-huGPRC5D/GS-CHO-huGPRC5D) were counted, diluted to 1 × 10⁶ cells/mL, and added to a U-shaped-bottom 96-well plate at 100 µL/well. The cells were centrifuged at 500 g for 5 min to remove the cell culture medium. The hybridoma culture supernatant in the 96-well plate and a positive control antibody were added to a U-shaped plate at 100 µL/well, the cells were resuspended, and the suspension was left to stand on ice for 30 min. The suspension was centrifuged at 500 g for 5 min to remove the supernatant, and the cells were washed once with PBS. The mixture was centrifuged at 500 g for 5 min to remove PBS. 100 µL of an FITC-labeled secondary antibody (Jackson Immunoresear, Cat# 115-545-006, diluted in 1:500 in PBS) of goat anti-mouse IgG was added to each well where the hybridoma supernatant was added; and 100 µL of a PE-labeled secondary antibody (BioLegend, Cat# 409304) of anti-human Fc was added to the wells where the positive control antibody (Roche-5E11) was contained. The resulting mixtures were incubated on ice in the dark for 30 min. The mixture was centrifuged at 500 g for 5 min to remove the supernatant, and the cells were washed once with PBS. The cells were resuspended with 50 µL of 1× PBS and determined on the computer by FACS.

The screened positive clones were re-screened for GS-cynoGPRC5D/GS-huGPRC5A cells in the same manner as described above to obtain 82 hybridoma cells binding to both human GPRC5D and monkey GPRC5D and not binding to human GPRC5A.

### Subcloning of positive hybridoma cells

According to the results of cell binding experiments, the candidate clones were subcloned by conventional methods. Then, detection was performed using the above-mentioned high-throughput screening method, and target positive wells were picked out for cell cryopreservation.

### Example 2. Preparation of Chimeric Antibody

Antibody light and heavy chain gene sequences of the hybridoma candidate clones obtained in Example 1 were extracted and used to construct a human-mouse chimeric antibody.

About 5 × 10⁶ freshly-cultured cells were taken, and RNA (Macherey-Nagel, Cat# 740984.250) was extracted. Reverse transcription was performed by using PrimeScript II 1st Strand cDNA Synthesis Kit (Takara) to obtain cDNA. Gene fragments of antibody light and heavy chain variable regions were amplified by designing an upstream primer using a base sequence located in FR1 region at the 5' end and designing a downstream primer using a base located in the antibody constant region or FR4 region. The gene fragments were ligated into a T vector (Mighty TA-cloning Kit, Takara), monoclones were picked out for sequencing, and the sequencing results were analyzed and compared using MEGA7 software.

By comparison, the clones with correct and paired antibody light and heavy chain variable region sequences were selected, and the gene fragments of the light and heavy chain variable regions thereof were respectively ligated to a pcDNA3.1 vector by homologous recombinases from Nanjing Vazyme Biotech Co., Ltd. (Exnase^{®}II, Cat# C112-01), wherein an IgG1 subtype was selected as the constant region, and expression plasmids of light chain and heavy chain antibodies were obtained.

The light chain plasmid and heavy chain plasmid of the same antibody were then mixed in a molar ratio of 1:1, and transfected into 293F cells by polyethyleneimine (PEI) (Polysciences, Cat# 23966). After 5-7 days of culture, the cell culture supernatant was collected and purified by a Protein A affinity column when the cell viability was below 60% to obtain a monoclonal antibody.

### Example 3. In vitro Screening of Chimeric Antibodies

The affinity of the anti-GPRC5D antibody was detected by flow cytometry. Antibodies at various concentrations were co-incubated with cells expressing human GPRC5D, including GS-CHO cells overexpressing human GPRC5D (huGPRC5D GS-CHO), multiple myeloma cells MM1.R, H929 and AMO-1, for 30 min. A fluorescently-labeled secondary antibody (APC-mouse anti-human IgG Fc antibody, Biolegend, Cat# 409306) was then added, the cell fluorescence intensity was detected using a Live/Dead yellow dead cell stain (Thermo Fisher, Cat# L34967) by flow cytometry, a curve was fitted using GraphPad Prism 8.0, and the EC50 value was calculated to show the binding affinity of the antibody to human GPRC5D.

Among the antibodies detected, HB15H1G1 (the heavy chain variable region of SEQ ID NO: 25, and the light chain variable region of SEQ ID NO: 26) was a fully human antibody, and ch5E12C4 (the heavy chain variable region of SEQ ID NO: 98, and the light chain variable region of SEQ ID NO: 99), ch11B7 (the heavy chain variable region of SEQ ID NO: 100, and the light chain variable region of SEQ ID NO: 101) and ch7F5D4 antibodies (the heavy chain variable region of SEQ ID NO: 27, and the light chain variable region of SEQ ID NO: 28) were chimeric antibodies. The results are shown in Table 1 below.

**Table 1. Detection of affinity of exemplary antibodies to human GPRC5D**

| Exemplary antibodies | EC₅₀ (nM) | | | |
|---|---|---|---|---|
| | H929 | MM1.R | AMO-1 | huGPRC5D-GS-CHO |
| HB15H1G1 | 3.70 | 0.70 | 9.40 | 1.89 |
| ch5E12C4* | 21.70 | 3.70 | 100.10 | 9.18 |
| ch11B7* | 5.99 | -- | -- | 1.14 |
| ch7F5D4 | 7.30 | 0.50 | 6.30 | 2.59 |

| | | | | |
|---|---|---|---|---|
| *ch5E12C4 was the sequence before the humanization ofhz5E12.1.P1; and ch11B7 was the sequence before the humanization of hz11B7.5. | | | | |

The screened ch5E12C4 and the chimeric ch11B7 antibody were humanized to form the antibodies hz5E12.1.P1 and hz11B7.5.

### Example 4. Molecular Structure Design and Construction of Trispecific Antibody (Ts)

A T-cell engager type multispecific antibody simultaneously targeting GPRC5D, BCMA and CD3 can simultaneously bind to two types of tumor-associated antigens GPRC5D and BCMA on the surface of multiple myeloma (MM) cells and CD3 receptors on the surface of T cells. On the basis of four anti-GPRC5D antibodies (HB15H1B1, ch7F5D4, hz5E12.1.P1 and hz11B7.5), one anti-BCMA antibody (ADI-38456) and two anti-CD3 antibodies with different affinities (low-affinity CD3 antibody hzsp34.87 and high-affinity CD3 antibody hzsp34.24), a multispecific antibody simultaneously targeting GPRC5D, BCMA and CD3 was designed.

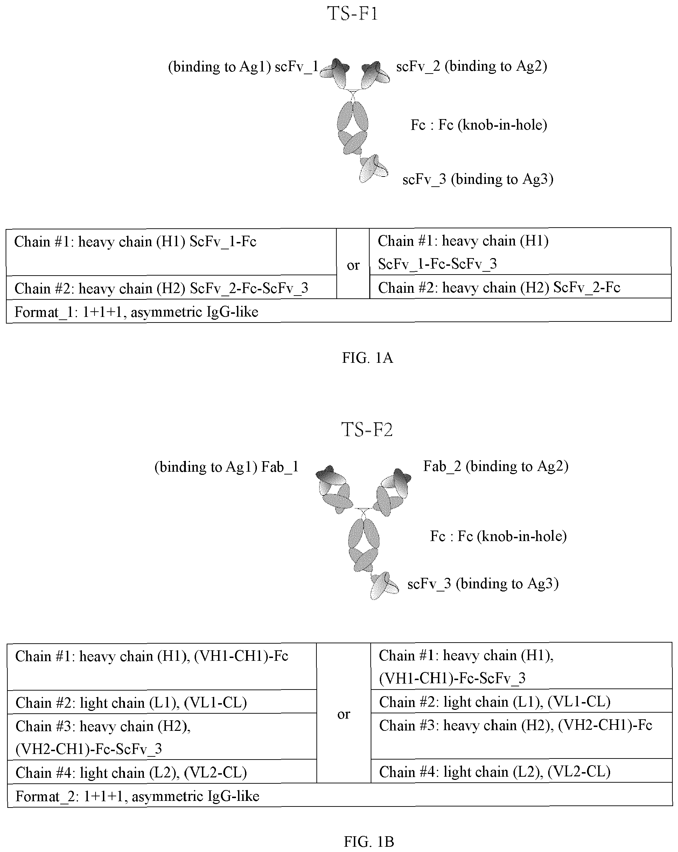

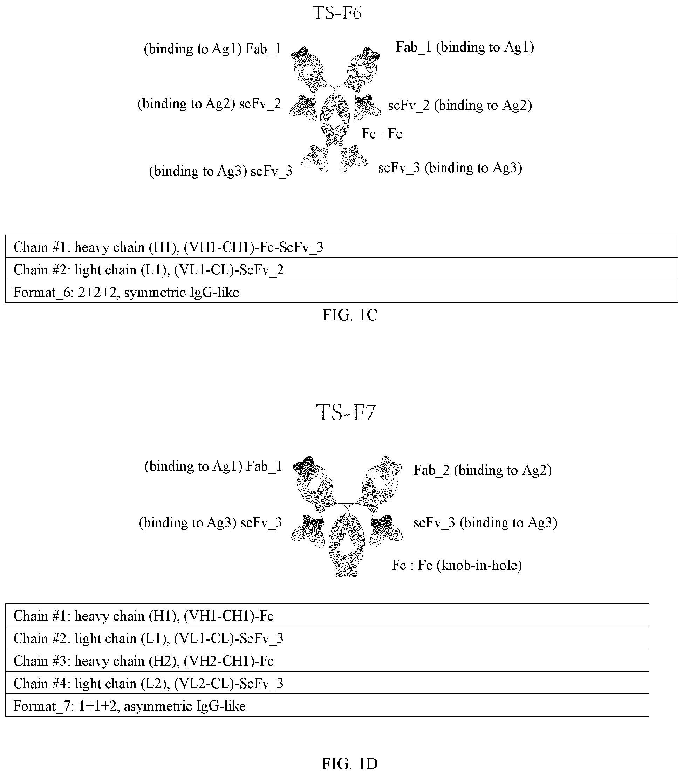

As shown in FIG. 1, four multispecific antibody molecules of different formats were designed, including two exemplary antibodies of 1+1+1 format 1 (TS-F1), six exemplary antibodies of 1+1+1 format 2 (TS-F2), four exemplary antibodies of 2+2+2 format 6 (TS-F6) and five exemplary antibodies of 1+1+2 format 7 (TS-F7) (see Table 4 below). The exemplary antibody design solves heavy chain mispairing of asymmetric IgG-like bispecific antibodies using the Fc "knob-in-hole" technology, wherein Fc was the heavy chain constant region of IgG1; and moreover, amino acid mutations L234A and L235A (according to "EU" numbering of Kabat) that weaken the effector function were introduced.

TS-F2 and TS-F7 were left-right asymmetric IgG-like tetramers that each consisted of 4 polypeptide chains, and TS-F6 was a left-right symmetric IgG-like tetramer that consisted of 2 polypeptide chains.

TS-F2 consisted of peptide chain 1# comprising a heavy chain variable domain, an immunoglobulin CH1 domain and an Fc domain, peptide chain 2# comprising a light chain variable domain and an immunoglobulin CL domain, peptide chain 3# comprising a heavy chain variable domain, an immunoglobulin CH1 domain, an Fc domain and a single-chain antibody scFv linked by an artificially synthesized linker peptide, and peptide chain 4# comprising a light chain variable domain and an immunoglobulin CL domain. The heavy chain variable domain of peptide chain 1# and the light chain variable domain of peptide chain 2# were paired to form a first antigen recognition site, the heavy chain variable domain of peptide chain 3# and the light chain variable domain of peptide chain 4# were paired to form a second antigen recognition site, and scFv formed a third antigen recognition site.

TS-F7 consisted of peptide chain 1# comprising a heavy chain variable domain, an immunoglobulin CH1 domain and an Fc domain, peptide chain 2# comprising a light chain variable domain, an immunoglobulin CL domain, and a single-chain antibody scFv linked by an artificially synthesized linker peptide, peptide chain 3# comprising a heavy chain variable domain, an immunoglobulin CH1 domain and an Fc domain, and peptide chain 4# comprising a light chain variable domain, an immunoglobulin CL domain, and a single-chain antibody scFv linked by an artificially synthesized linker peptide. The heavy chain variable domain of peptide chain 1# and the light chain variable domain of peptide chain 2# were paired to form a first antigen recognition site, the heavy chain variable domain of peptide chain 3# and the light chain variable domain of peptide chain 4# were paired to form a second antigen recognition site, and the two scFv in peptide chain 2# and peptide chain 4# were two identical third antigen recognition sites.

In TS-F2 and TS-F7, the Fc domains of peptide chain 1# and peptide chain 3# contained corresponding stable association mutations of "knob-in-hole", respectively.

TS-F6 consisted of two identical peptide chains 1 and two identical peptide chains 2, wherein peptide chain 1# contained a heavy chain variable domain, an immunoglobulin CH1 domain, an Fc domain and a single-chain antibody scFv linked by an artificially synthesized linker peptide; and peptide chain 2# contained a light chain variable domain, an immunoglobulin CL domain and a single-chain antibody scFv linked by an artificially synthesized linker peptide. The heavy chain variable domain of peptide chain 1# and the light chain variable domain of peptide chain 2# were paired to form a first antigen recognition site, the scFv in peptide chain 1# was a second antigen recognition site, and the scFv in peptide chain 2# was a third antigen recognition site.

TS-F1 consisted of peptide chain 1 and peptide chain 2, wherein peptide chain 1# contained a first single-chain antibody scFv and an Fc domain; and peptide chain 2# contained a second single-chain antibody scFv and an Fc domain. The first scFv in peptide chain 1# formed a first antigen recognition site, and the scFv in peptide chain 2# formed a second antigen recognition site.

### Example 5. Expression and Purification of TS Antibody

The amino acid sequences of the CDRs, light chain variable regions and heavy chain variable regions of four anti-GPRC5D antibodies (HB15H1B1, ch7F5D4, hz5E12.1.P1, and hz11B7.5), one anti-BCMA antibody (ADI-38456) and two anti-CD3 antibodies with different affinities (hzsp34.87 and hzsp34.24) were listed in the section "Sequence Listing" of the present application, wherein HB15H1B1 was a fully human antibody, ch7F5D4 was a chimeric antibody, and hz5E12.1.P1 and hz11B7.5 were humanized antibodies, and the sequence numbers of the amino acid sequences of the CDRs, light chain variable regions and heavy chain variable regions of the above-mentioned antibodies were listed in Table 2.

**Table 2. Amino acid sequence numbers of CDRs, light chain variable regions and heavy chain variable regions of anti-GPRC5D antibodies, anti-BCMA antibody and anti-CD3 antibodies.**

| **Target** | **GPRC5D** | | | | **BCMA** | **CD3** | |
|---|---|---|---|---|---|---|---|
| **ID** | **HB15H1B1** | **ch7F5D4** | **hz5E12.1.P1** | **hz11B7.5** | **38456** | **hzsp34.24** | **hzsp34.87** |
| HCDR1 | Seq 1 | Seq 7 | Seq 13 | Seq 19 | Seq 33 | Seq 41 | Seq 47 |
| HCDR2 | Seq 2 | Seq 8 | Seq 14 | Seq 20 | Seq 34 | Seq 42 | Seq 42 |
| HCDR3 | Seq 3 | Seq 9 | Seq 15 | Seq 21 | Seq 35 | Seq 43 | Seq 43 |
| LCDR1 | Seq 4 | Seq 10 | Seq 16 | Seq 22 | Seq 36 | Seq 44 | Seq 44 |
| LCDR2 | Seq 5 | Seq 11 | Seq 17 | Seq 23 | Seq 37 | Seq 45 | Seq 45 |
| LCDR3 | Seq 6 | Seq 12 | Seq 18 | Seq 24 | Seq 38 | Seq 46 | Seq 46 |
| VH | Seq 25 | Seq 27 | Seq 29 | Seq 31 | Seq 39 | Seq 48 | Seq 50 |
| VL | Seq 26 | Seq 28 | Seq 30 | Seq 32 | Seq 40 | Seq 49 | Seq 49 |

**Table 3A. Amino acid sequence numbers and sequence descriptions of linkers in exemplary antibodies**

| **Name** | **Seq ID** | **Sequence description** |
|---|---|---|
| linker 1 | seq 51 | (G4S)4 |
| linker 2 | seq 52 | (G4S)2 |
| linker 3 | seq 53 | (G4S)3 |
| linker 4 | seq 54 | TS(G4S)3 |

**Table 3B. Amino acid sequence numbers and sequence descriptions of single-chain antibody fragments in exemplary antibodies**

| Name | Sequence | Description |
|---|---|---|
| HB15H1B1-scFv_linker1 | seq 55 | The scFv form of GPRC5D antibody HB15H1B1 contained VL sequence-linker 1-VH sequence from the N-terminus to the C-terminus |
| ch7F5D4-scFv | seq 60 | The scFv form of GPRC5D antibody ch7F5D4 contained VL sequence-linker 1-VH sequence from the N-terminus to the C-terminus |
| 38456 -scFv_linker1 | seq 56 | The scFv form of BCMA antibody 38456 contained VL sequence-linker 1-VH sequence from the N-terminus to the C-terminus |
| 38456 -scFv_linker3 | seq 57 | The scFv form of BCMA antibody 38456 contained VL sequence-linker 3-VH sequence from the N-terminus to the C-terminus |
| Hzsp34.24 -scFv-VL-VH | seq 58 | The scFv form of high-affinity CD3 antibody Hzsp34.24 contained VL sequence-linker 1-VH sequence from the N-terminus to the C-terminus |
| Hzsp34.24 -scFv-VH-VL | seq 59 | The scFv form of high-affinity CD3 antibody Hzsp34.24 contained VH sequence-linker 1-VL sequence from the N-terminus to the C-terminus |

**Table 4. Amino acid sequence numbers of exemplary TS antibodies**

| **Exemplary antibodies** | **Peptide chain 1#** | **Peptide chain 2#** | **Peptide chain 3#** | **Peptide chain 4#** |
|---|---|---|---|---|
| TS-F1-1 | seq 61 | seq 62 | - | - |
| TS-F1-2 | seq 63 | seq 64 | - | - |
| TS-F2-1 | seq 65 | seq 66 | seq 67 | seq 68 |
| TS-F2-2 | seq 69 | seq 70 | seq 71 | seq 68 |
| TS-F2-3 | seq 72 | seq 73 | seq 74 | seq 68 |
| TS-F2-4 | seq 75 | seq 76 | seq 77 | seq 68 |
| TS-F2-5 | seq 75 | seq 76 | seq 78 | seq 68 |
| TS-F2-6 | seq 79 | seq 80 | seq 77 | seq 68 |
| TS-F6-1 | seq 81 | seq 82 | - | - |
| TS-F6-2 | seq 81 | seq 83 | - | - |
| TS-F6-3 | seq 84 | seq 85 | - | - |
| TS-F6-4 | seq 84 | seq 86 | - | - |
| TS-F7-1 | seq 87 | seq 88 | seq 77 | seq 89 |
| TS-F7-2 | seq 90 | seq 91 | seq 77 | seq 89 |
| TS-F7-3 | seq 92 | seq 93 | seq 94 | seq 95 |
| TS-F7-4 | seq 92 | seq 96 | seq 94 | seq 97 |
| TS-F7-5 | seq 87 | seq 88 | seq 78 | seq 89 |

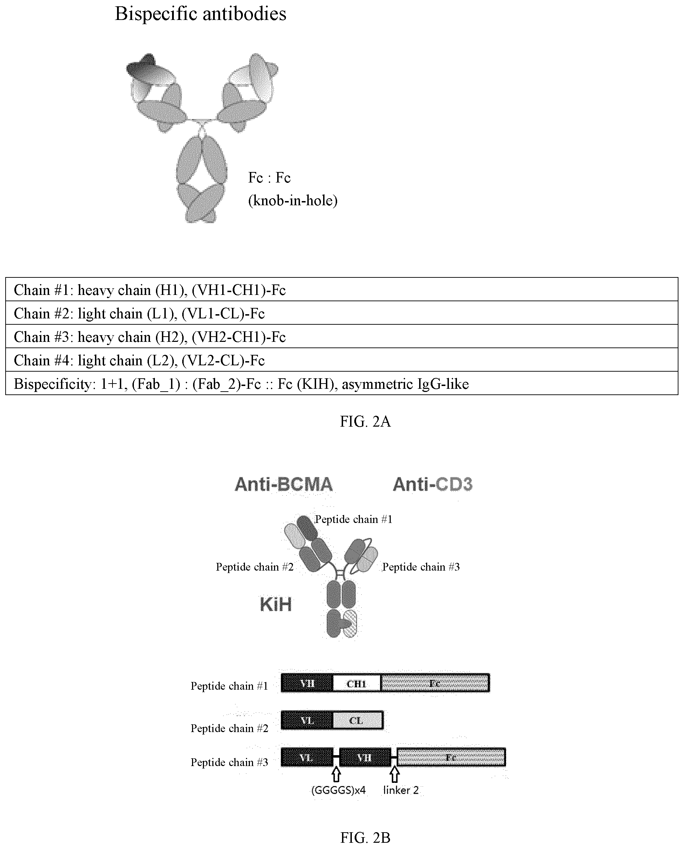

GPRC5D × CD3 and BCMA × CD3 bispecific antibodies were designed simultaneously. As shown in FIG. 2A, as a rational design, the two ends of each bispecific antibody were both Fab forms, and an asymmetric Ig-like structure of 1+1 format was formed. Heavy chain mispairing of asymmetric IgG-like bispecific antibodies was solved using the Fc "knob-in-hole" technology, wherein Fc was the heavy chain constant region of IgG1; and moreover, amino acid mutations L234A and L235A (according to "EU" numbering of Kabat) that weaken the effector function were introduced.

Moreover, BCMA × CD3 bispecific antibody 46758 was further expressed. As shown in FIG. 2B, one antibody arm of the bispecific antibody was a Fab binding to BCMA, and the other antibody arm was an scFv binding to CD3, wherein the Fab was derived from the anti-BCMA parent antibody 38456, and the scFv was derived from an anti-CD3 parent antibody different from the previous one. The three polypeptide chains constituting the bispecific antibody were as set forth in SEQ ID NOs: 111-113.

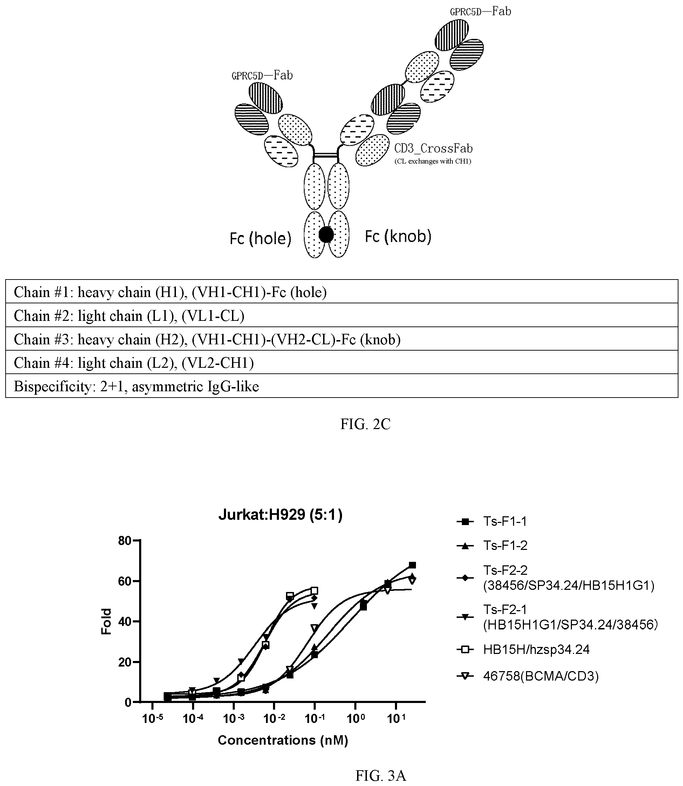

Moreover, the Roche-5E11 bispecific antibody (GPRC5D × CD3, 2:1 format, derived from the patent WO 2019/154890 A1) was further expressed and purified. As shown in FIG. 2C, the bispecific antibody Roche_5E11 consisted of the sequences set forth in SEQ ID NOs: 17, 18, 19 and 20 of WO 2019/154890 A1, and contained two GPRC5D-binding sites and one CD3-binding site.

For the recombinant production, Suzhou Genewiz Biological Technology Co., Ltd. was entrusted to synthesize the nucleotide sequences encoding the heavy chain and the light chain and insert the nucleotide sequences into a vector pcDNA3.1 respectively to facilitate further cloning and/or expression in host cells.

### Expression and purification in HEK293 cells:

Expi293F cells (purchased from Thermo Fisher scientific company) were subcultured in an Expi293F cell culture medium (purchased from Thermo Fisher scientific company). The cell density was detected the day before transfection and was adjusted to 2 × 10⁶ cells/mL with a fresh Expi293 cell culture medium, culture was continued, and the cell density was adjusted to 3 × 10⁶ cells/mL on the day of transfection.

An Opti-MEM medium (purchased from Gibco company) with a volume of 1/10 of the final volume of transfected Expi293F cells was taken as a transfection buffer, 10 µg of recombinant plasmids comprising paired heavy chain and light chain nucleotide sequences respectively prepared above in a molar ratio of 1:1 was added to each milliliter of the transfection buffer, and then the resulting mixture was mixed uniformly.

30 µg of polyethyleneimine (PEI) (Polysciences) was then added to each milliliter of the transfection buffer, and the resulting mixture was mixed uniformly and incubated at room temperature for 20 min. Then, the PEI/DNA mixture was gently poured into the Expi293F cell suspension, and the resulting mixture was mixed uniformly and cultured in a shaker under the conditions of 8% CO₂, 36.5 °C and 120 rpm.

After culturing for 16-18 h, the culture flasks were supplemented with 200 g/L FEED (100 g/L Phyton Peptone + 100 g/L Difco Select Phytone) with a volume of 1/50 of the volume of the cultures obtained after transfection, a glucose solution with a final concentration of 4 g/L and VPA (Gibco) with a final concentration of 2 mM/L, and the resulting mixture was mixed gently and continuously cultured in a shaker under the conditions of 8% CO₂, 36.5 °C and 120 rpm. Culture was continued for 6 days, the cultures were collected and centrifuged at 4000 rpm for 30 min, the cell supernatant was taken and filtered with a 0.45-µM filter membrane, and the parent protein was purified by affinity chromatography and ion exchange chromatography. For the IgG-like antibody of a symmetric structure, a final molecule was purified.

For the above-mentioned TS antibodies and bispecific antibodies of different Formats, the parent proteins produced by recombination were as shown below:

| | Parent protein 1 | | Parent protein 2 | |
|---|---|---|---|---|
| Format | Heavy chain H1 | Light chain L 1 | Heavy chain H2 | Light chain L2 |
| TS-Format_1 | Peptide chain 1# | - | Peptide chain 2# | - |
| TS-Format_2 | Peptide chain 1# | Peptide chain 2# | Peptide chain 3# | Peptide chain 4# |
| TS-Format_6 | Peptide chain 1# | Peptide chain 2# | - | - |
| TS-Format_7 | Peptide chain 1# | Peptide chain 2# | Peptide chain 3# | Peptide chain 4# |
| Bs_1+1_format | Peptide chain 1# | Peptide chain 2# | Peptide chain 3# | Peptide chain 4# |

For the asymmetric IgG-like antibody, two purified parent proteins did not need medium change, with the protein concentrations > 1 mg/mL respectively, and were mixed according to a molar ratio of 1:1, wherein the protein concentration in the mixed solution was 1-10 mg/mL. Then, a proper amount of GSH was added, with the GSH/protein molar ratio controlled to 500-700 folds and the GSH final concentration > 5 mM.

Finally, the pH of the above-mentioned mixture was adjusted to 8.0-8.5 with 1 M Arg at pH 10.0. The final concentration of Arg was >= 50 mM. The above-mentioned reaction solution was kept at room temperature overnight for no more than 24 h. The GSH was removed from the reaction solution obtained after the overnight reaction by medium change the next day, wherein the buffer for medium change could be the above-mentioned 0.2 M GSH-free PB solution (pH 6.0) of the recombination reaction. The resulting product was oxidized naturally for 2-3 h, and purified by monoS (GE Cat. 17516801).

The Roche-5E11 bispecific antibody was prepared according to the method disclosed in WO 2019/154890 A1.

The symmetric antibody and the asymmetric antibody prepared according to the above-mentioned method were harvested and purified by affinity chromatography and ion exchange chromatography.

The purity of the samples collected by chromatography in each fraction tube was detected by size exclusion chromatography (SEC). According to the SEC results, the samples with a purity greater than 95% in the fraction tubes were combined.

The purified bispecific antibody solution was centrifuged in a 15-mL ultrafiltration centrifuge tube at 4500 rpm for 30 min. The protein was diluted with PBS and then centrifuged at 4500 rpm for another 30 min, and this operation was repeated several times to exchange the buffer. The antibodies obtained after buffer exchange were combined, and the antibody concentration was measured. Further, the composition and content of the multispecific antibody were qualitatively and quantitatively determined by capillary electrophoresis (CE-SDS) in combination with liquid chromatography-mass spectrometry (LC-MS).

### Example 6. Determination of affinities of Trispecific Antibodies

In the exemplary trispecific antibodies, the binding affinities of the antigen-binding sites from the humanized antibodies hz5E12.1.P1 and hz11B7.5 to GPRC5D were detected. In the same way as that in Example 3, trispecific antibodies at different concentrations were co-incubated with huGPRC5D GS-CHO cells for 30 min, then fluorescently-labeled secondary antibodies were added, the fluorescence intensity of the cells was detected by a flow cytometer (BD), curves were fitted using GraphPad Prism 8.0, and EC50 values were calculated to show the binding affinities of the antibodies to human GPRC5D. The results are shown in Table 5 below.

**Table 5. Affinities of exemplary antibodies to human GPRC5D**

| | Exemplary antibodies | | | |
|---|---|---|---|---|
| | TS-F2-4 (hz5E12.1.P1/SP34.2 4/38456) | TS-F2-5 (hz5E12.1.P1/SP34.8 7/38456) | TS-F7-1 (hz5E12.1.P1/SP34.2 4/38456) | TS-F7-2 (hz11B7.5/SP34.24 /38456) |
| EC₅₀ (nM) | 40.04 | 45.04 | 53.24 | 49.15 |

In the exemplary trispecific antibodies, the affinities of the anti-BCMA end and the anti-CD3 end were detected by bio-layer interferometry (BLI). The binding kinetics of the exemplary antibodies to human BCMA, monkey BCMA, human CD3E and monkey CD3E were determined.

Sensors (Fortebio, Cat# 18-5019) coupled to streptavidin protein (SA) or anti-human-Fc (AHC) were prewetted by immersion in 200 µL of SD buffer (1× PBS, 0.1% BSA, 0.05% tween-20). Exemplary antibodies and biotin-labeled or Fc-tagged BCMA and CD3D&E heterodimer antigens were diluted with SD buffer, respectively. 200 µL of SD buffer and diluted samples (100 nM) were taken and added to a 96-well black plate (Greiner, Cat# 655209), respectively. The sensors and the samples were placed in Octet (Fortebio, Red96e). Data Acquisition 10.0 was executed and "New Kinetic Experiment" was selected. The sensors were arranged according to the positions of the samples, and the operation procedures and times were set as: Baseline 60 s, Loading 250 s, Baseline 100 s, Association 600 s and Dissociation 600 s. The experiment was conducted at a rotation speed of 1000 rpm and 30 °C.

The results were analyzed in "Data Analysis 10.0" software, with the buffer reference channel being subtracted. 1:1 binding was selected to fit the data, and kₒₙ, k_{off}, and K_{D} values for the exemplary antibodies were calculated.

In the experiments performed according to the above-mentioned assay, the exemplary antibodies were detected using a human BCMA antigen (ACRO Biosystems) and a monkey BCMA antigen (ACRO Biosystems). The detection results of the affinities to human BCMA are shown in Table 6; and the detection results of the affinities to monkey BCMA are shown in Table 7.

**Table 6. Detection of affinities of exemplary antibodies to human BCMA**

| Name | Antigen | Ka(1/Ms) | kd(1/s) | KD (M) |
|---|---|---|---|---|
| TS-F2-4 | Human | 1.31E+05 | 2.97E-04 | 2.27E-09 |
| TS-F2-5 | BCMA-Bio | 1.33E+05 | 2.79E-04 | 2.10E-09 |
| TS-F7-2 | | 2.70E+05 | 2.00E-04 | 7.41E-10 |

As can be seen from the results in the above-mentioned table, the above-mentioned 3 exemplary antibodies of the present invention all showed extremely high affinities to BCMA.

**Table 7. Detection of affinities of exemplary antibodies to monkey BCMA**

| Name | Antigen | Ka(1/Ms) | kd(1/s) | KD (M) |
|---|---|---|---|---|
| TS-F2-4 | Cyno | 9.80E+05 | 2.00E-04 | 2.04E-10 |
| TS-F2-5 | BCMA-Fc | 9.50E+05 | 2.00E-04 | 2.10E-10 |
| TS-F7-2 | | 6.77E+05 | 3.73E-04 | 5.52E-10 |

In experiments performed according to the above-mentioned assay, the affinities of the exemplary antibodies were detected using a human CD3D&E heterodimer antigen (ACRO Biosystems) and a monkey CD3D&E heterodimer antigen (ACRO Biosystems). The results are shown in Tables 8 and 9. The binding affinities of GPRC5D × CD3 bispecific antibodies hz5E12.1-P1/SP34.24 and hz5E12.1-P1/SP34.87 to human/monkey CD3D&E were also detected.

**Table 8. Detection of affinities of exemplary antibodies to human CD3D&E**

| Name | Antigen | Ka(1/Ms) | kd(1/s) | KD (M) |
|---|---|---|---|---|
| TS-F2-4 | Human | 1.47E+05 | 3.26E-03 | 2.21E-08 |
| TS-F2-5 | CD3D&E-Bio | 7.75E+04 | 1.88E-02 | 2.43E-07 |
| hz5E12.1-P1/SP34.24 | | 1.56E+05 | 3.35E-03 | 2.14E-08 |
| hz5E12.1-P1/SP34.87 | | 6.19E+04 | 1.76E-02 | 2.85E-07 |

**Table 9. Detection of affinities of exemplary antibodies to monkey CD3D&E**

| Name | Antigen | Ka(1/Ms) | kd(1/s) | KD (M) |
|---|---|---|---|---|
| TS-F2-4 | Cyno | 1.32E+05 | 3.01E-03 | 2.27E-08 |
| TS-F2-5 | CD3D&E-Bio | 7.86E+04 | 1.73E-02 | 2.20E-07 |
| hz5E12.1-P1/SP34.24 | | 1.23E+05 | 3.19E-03 | 2.60E-08 |
| hz5E12.1-P1/SP34.87 | | 5.83E+04 | 1.69E-02 | 2.90E-07 |

### Example 7. Anti-GPRCSD × BCMA × CD3 Antibody-Mediated Jurkat Cell Activation Experiments

Activation activities of anti-GPRC5D × BCMA × CD3 antibodies on the downstream signaling pathway of CD3E were detected by a Jurkat-NFAT-Luc reporter system. When the anti-GPRC5D × BCMA × CD3 polyclonal antibody binds to GPRC5D and/or BCMA on the surface of the multiple myeloma (MM) cell strain and simultaneously binds to CD3E on the surface of Jurkat-NFAT-Luc cells (Jurkat), downstream signaling of CD3E in the Jurkat-NFAT-Luc cells can be stimulated by tumor-associated antigen (GPRC5D and/or BCMA)-dependent CD3 cross-linking. Therefore, the level of activation of T cells mediated by exemplary antibodies was evaluated by the Luciferase reporter system.

Exemplary antibodies at different concentrations were added to Jurkat NFAT luciferase cells and MM tumor cells, and the expression of luciferase in the Jurkat cells was observed.

### Reagents and materials

| Name | Cat# | Manufacturer |
|---|---|---|
| RPMI medium 1640 (1x) | 11835-030 | Gibco |
| FBS | SH30406.05 | Hyclone |
| Bio-Glo^{™} Luciferase Assay system | G7940 | Promega |

Briefly, the sample was first diluted, wherein the antibody was diluted with RPMI medium 1640 (5% FBS) to a concentration of 100 nM and then diluted in a 5-fold gradient. Then, the cells were prepared, wherein the Jurkat cells and tumor cells were centrifuged at 300 g for 5 min, the supernatant was discarded, the resulting product was resuspended in an RPMI medium 1640 (5% FBS) and counted (Countstar), then the cell density was adjusted to 2 × 10⁶ cells/mL for Jurkat NFAT, and the tumor cells were adjusted according to the ratio of effector cells to target cells. 45 µL of the tumor cells were added to each well of a 96-well white cell culture plate, 30 µL of the samples subjected to gradient dilution were added, and 45 µL of the Jurkat NFAT cells were added. The 96-well plate was then placed in a 37 °C, 5% CO₂ incubator for further culture. After culture for a period of time, the cell culture plate was taken and left to stand at room temperature for 5 min, 80 µL of Bio-Glo (Promega, G7940) was added to each well, the resulting mixture was incubated in the dark for 10 min, and the fluorescence value was read on SpectraMax i3 (MOLECULAR DEVICES). The curves were fitted using GraphPad Prism 8.0 and the EC50 values were calculated to compare the activation activities of exemplary antibodies on T cells. The results were shown as a ratio of the reading values of the experimental group to the reading values of the control hIgG1 group.

### Results:

### TS-F2-1 and TS-F2-2

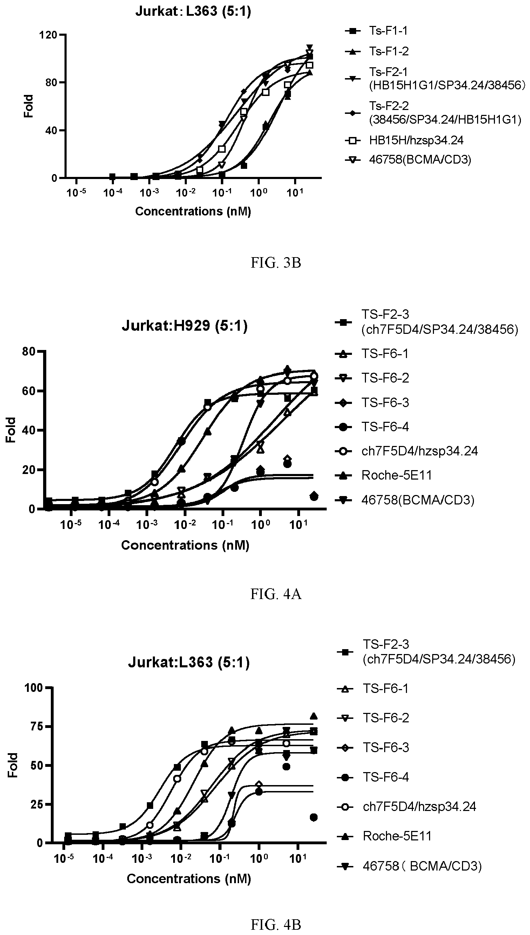

Under the conditions that H929 cells and Jurkat cells (the ratio of effector cells to tumor cells was 5:1) were co-cultured for 16 h, as well as L363 cells and Jurkat cells (the ratio of effector cells to tumor cells was 5:1) were co-cultured for 16 h, TS-F2-1 and TS-F2-2 can both significantly improve the expression of luciferase in the Jurkat cells with the increase of the antibody concentration (FIGs. 3A and 3B); and the agonistic activities thereof were higher than those of TS-F1 and the control groups, i.e., GPRC5D × CD3 bispecific antibodies and BCMA × CD3 bispecific antibodies. Specific EC50 for the antibodies detected is shown in Table 10. Exemplary antibodies TS-F2-1 and TS-F2-2 both consisted of three binding specificities from HB15H1G1 (the GPRC5D antibody), hzsp34.24 (the CD3 antibody) and 38456 (the BCMA antibody), but the positions of the HB15H1G1 and 38456 binding specificities were different.

**Table 10. Activation of T cells mediated by exemplary antibodies**

| Exemplary antibodies | EC₅₀ (nM) | |
|---|---|---|
| | H929 | L363 |
| TS-F1-1 (HB15H1G1/SP34.24/38456) | 0.80 | 3.55 |
| TS-F1-2 (38456/SP34.24/HB15H1G1) | 0.21 | 1.92 |
| TS-F2-1 (HB15H1G1/SP34.24/38456) | 0.00 | 0.24 |
| TS-F2-2 (38456/SP34.24/HB15H1G1) | 0.01 | 0.13 |
| GPRC5DxCD3 (HB15H/hzsp34.24) | 0.01 | 0.28 |
| BCMAxCD3 (46758) | 0.07 | 0.43 |

### TS-F2-3

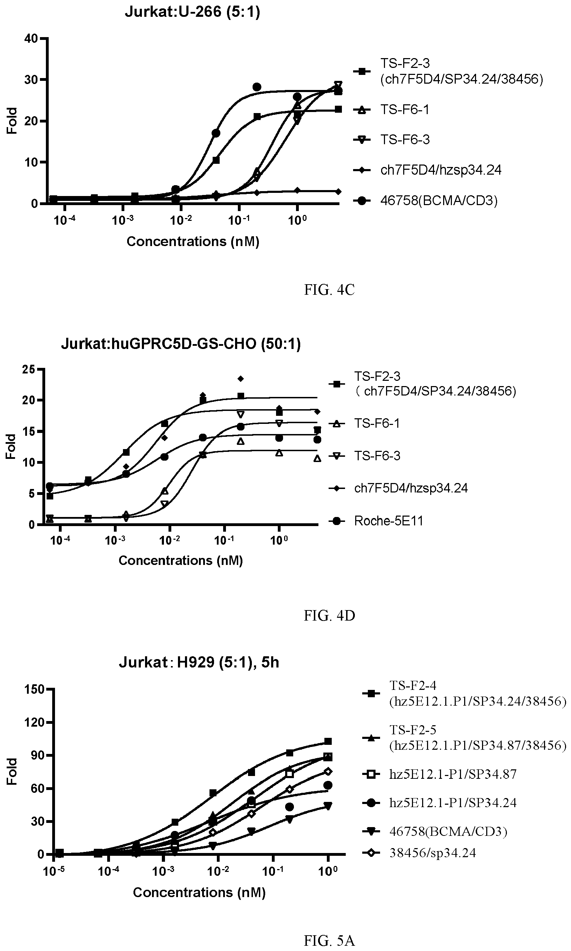

Under the conditions that H929 cells and Jurkat cells (the ratio of effector cells to tumor cells was 5:1) were co-cultured for 16 h, L363 cells and Jurkat cells (the ratio of effector cells to tumor cells was 5:1) were co-cultured for 16 h, as well as U266 cells and Jurkat cells (the ratio of effector cells to tumor cells was 5:1) were co-cultured for 16 h, TS-F2-3 can significantly improve the expression of luciferase in the Jurkat cells with the increase of the antibody concentration (FIGs. 4A, 4B and 4C); and the agonistic activities thereof were significantly higher than that of the TS-F6 antibody and higher than those of the control groups, i.e., the GPRC5D × CD3 bispecific antibody (ch7F5D4/hzsp34.24) and the BCMA × CD3 (46758) bispecific antibody. Specific EC50 for the antibodies detected is shown in Table 11. Exemplary antibodies TS-F2-3 and TS-F6 (TS-F6-1 to TS-F6-4) consisted of three binding specificities from ch7F5D4 (the GPRC5D antibody), hzsp34.24 (the CD3 antibody) and 38456 (the BCMA antibody).

The constructed GS-CHO cells overexpressing huGPRC5D (huGPRC5D-GS-CHO) and Jurkat cells were co-cultured for 16 h, and the exemplary antibodies were added to simulate the activation of T cells mediated by multiple myeloma cells expressing only GPRC5D. The ratio of effector cells (Jurkat cells) to target cells (huGPRC5D-GS-CHO cells) was 50:1. The experimental results showed that TS-F2-3 can significantly improve the expression of luciferase in the Jurkat cells with the increase of the antibody concentration (FIG. 4D); and the agonistic activity thereof was higher than those of the TS-F6 antibody and the control groups, i.e., the GPRC5D × CD3 bispecific antibody and the BCMA × CD3 bispecific antibody (46758). Specific EC50 is shown in Table 11.

**Table 11. Activation of T cells mediated by exemplary antibodies**

| Exemplary antibodies | EC₅₀ (pM) | | | |
|---|---|---|---|---|
| | H929 | L363 | U266 | huGPRC5D-GS-CHO |
| TS-F2-3 (ch7F5D4/SP34.24/38456) | 5.473 | 2.856 | 46.95 | 1.447 |
| TS-F6-1 | 3788 | 77.14 | 366 | 9.66 |
| TS-F6-2 | 2203 | 61.71 | -- | -- |
| TS-F6-3 | 107.7 | 220.6 | 633.6 | 25.46 |
| TS-F6-4 | 77.09 | 247.1 | -- | -- |
| ch7F5D4/hzsp34.24 | 7.716 | 5.624 | 36.14 | 5.662 |
| Roche-5E11 | 30.15 | 20.82 | -- | -- |
| 46758 | 343.8 | 192.4 | 31.13 | 5.902 |

### TS-F2-4 and TS-F2-5

Under the condition that H929 cells and Jurkat cells (the ratio of effector cells to tumor cells was 5:1) were co-cultured for 5 h,

Under the condition that L363 cells and Jurkat cells (the ratio of effector cells to tumor cells was 5:1) were co-cultured for 5 h,

Under the condition that 8226 cells and Jurkat cells (the ratio of effector cells to tumor cells was 5:1) were co-cultured for 5 h, and

Under the condition that U266 cells and Jurkat cells (the ratio of effector cells to tumor cells was 5:1) were co-cultured for 5 h,

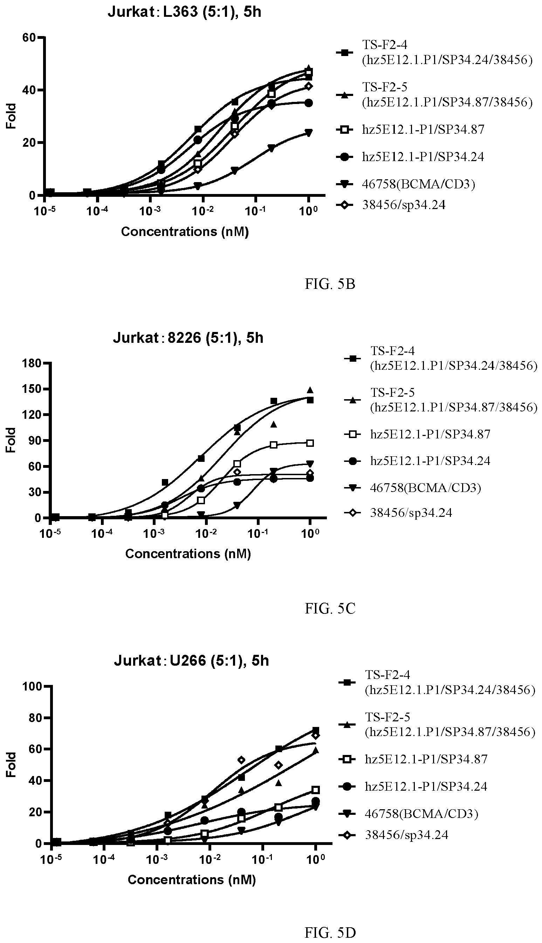

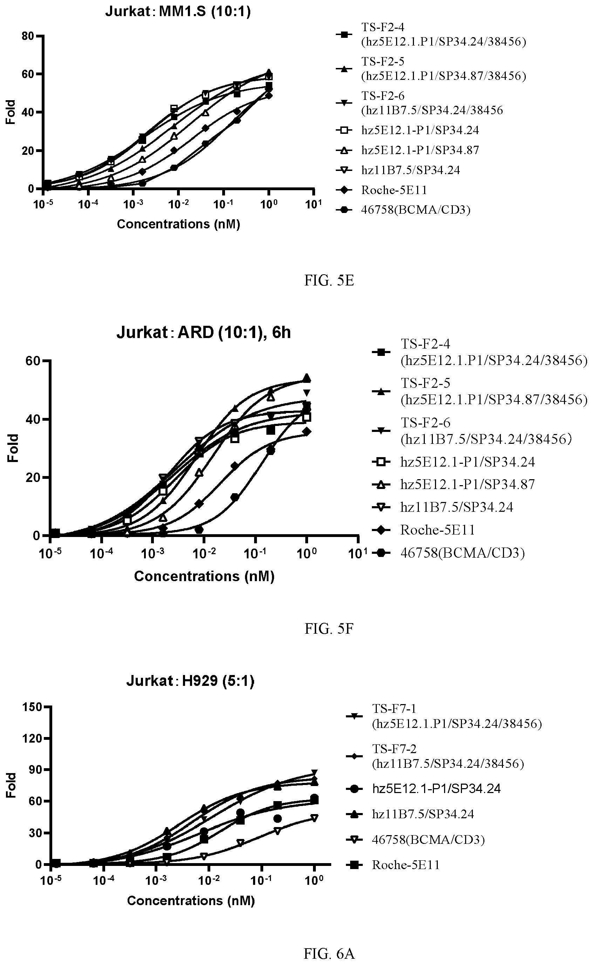

TS-F2-4 and TS-F2-5 can both significantly improve the expression of luciferase in the Jurkat cells with the increase of the antibody concentration (FIGs. 5A, 5B and 5C); and the agonistic activities thereof were higher than those of the control groups, i.e., the GPRC5D × CD3 bispecific antibody, the BCMA × CD3 bispecific antibody (38456/hzsp34.24) and the 46758 bispecific antibody. Specific EC50 is shown in Table 12. The exemplary antibody TS-F2-4 consisted of hz5E12.1.P1 (the GPRC5D antibody), hzsp34.24 (the high-affinity CD3 antibody) and 38456 (the BCMA antibody), and the exemplary antibody TS-F2-5 consisted of hz5E12.1.P1 (the GPRC5D antibody), hzsp34.87 (the low-affinity CD3 antibody) and 38456 (the BCMA antibody).

**Table 12. Activation of T cells mediated by exemplary antibodies**

| Exemplary antibodies | EC₅₀ (pM) | | | |
|---|---|---|---|---|
| | H929 | L363 | 8226 | U266 |
| TS-F2-4 (hz5E12.1.P1/SP34.24/38456) | 7.38 | 6.29 | 7.88 | 50.33 |
| TS-F2-5 (hz5E12.1.P1/SP34.87/38456) | 17.80 | 21.26 | 21.09 | 858.40 |
| hz5E12.1-P1/SP34.24 | 6.56 | 5.59 | 3.18 | 5.70 |
| hz5E12.1-P1/SP34.87 | 45.33 | 38.86 | 20.10 | 145.20 |
| 46758 | 83.49 | 90.09 | 79.78 | 603.30 |
| 38456/SP34.24 | 59.62 | 36.59 | 4.82 | 10.97 |

Under the conditions that MM1.S cells and Jurkat cells (the ratio of effector cells to tumor cells was 10:1) were co-cultured for 5 h, as well as ARD cells and Jurkat cells (the ratio of effector cells to tumor cells was 10:1) were co-cultured for 5 h, TS-F2-4 and TS-F2-5 can significantly improve the expression of luciferase in the Jurkat cells with the increase of the antibody concentration (FIG. 5E); and the agonistic activities thereof were higher than those of the control groups, i.e., the GPRC5D × CD3 bispecific antibody and the BCMA × CD3 bispecific antibody 46758. Specific EC50 is shown in Table 13.

**Table 13. Activation of T cells mediated by exemplary antibodies**

| Exemplary antibodies | EC₅₀ (pM) | |
|---|---|---|
| | MM.1S | ARD |
| TS-F2-4 (hz5E12.1.P1/SP34.24/38456) | 1.79 | 2.38 |
| TS-F2-5 (hz5E12.1.P1/SP34.87/38456) | 7.34 | 6.96 |
| TS-F2-6 (hz11B7.5/SP34.24/38456) | 2.02 | 2.78 |
| hz5E12.1-P1/SP34.24 | 2.25 | 2.73 |
| hz5E12.1-P1/SP34.87 | 18.27 | 14.88 |
| hz11B7.5/SP34.24 | 1.98 | 2.04 |
| Roche-SE11 | 20.06 | 20.79 |
| 46758 | 158.90 | 132.90 |

### TS-F7-1 and TS-F7-2

Under the condition that H929 cells and Jurkat cells (the ratio of effector cells to tumor cells was 5:1) were co-cultured for 5 h,

Under the condition that L363 cells and Jurkat cells (the ratio of effector cells to tumor cells was 5:1) were co-cultured for 5 h,

Under the condition that 8226 cells and Jurkat cells (the ratio of effector cells to tumor cells was 5:1) were co-cultured for 5 h, and

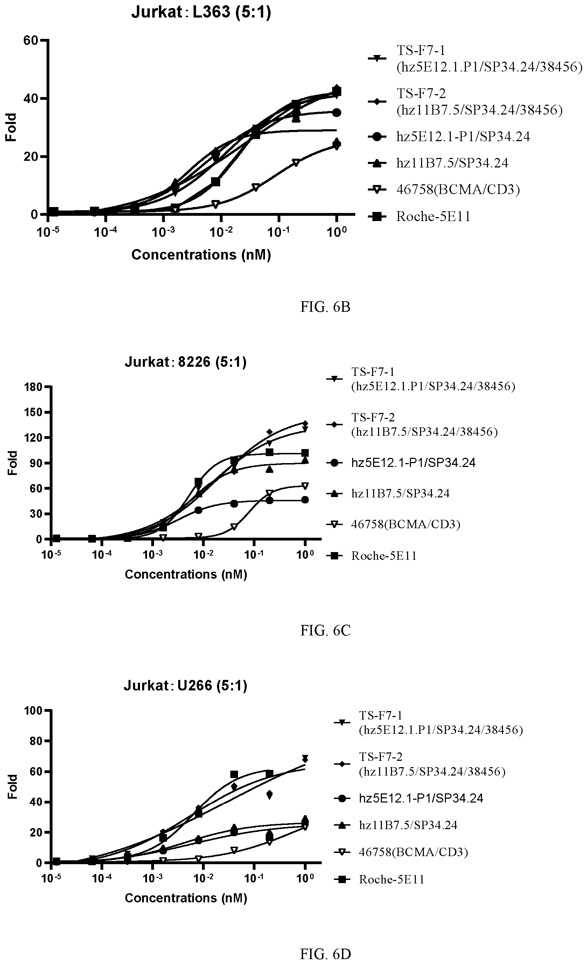

Under the condition that U266 cells and Jurkat cells (the ratio of effector cells to tumor cells was 5:1) were co-cultured for 5 h,
TS-F7-1 and TS-F7-2 can significantly improve the expression of luciferase in the Jurkat cells with the increase of the antibody concentration (FIGs. 6A, 6B, 6C and 6D); and the agonistic activities thereof were higher than those of the control groups, i.e., the GPRC5D × CD3 bispecific antibody and the BCMA × CD3 bispecific antibody 46758. Specific EC50 is shown in Table 14. The exemplary antibody TS-F7-1 consisted of hz5E12.1.P1 (the GPRC5D antibody), hzsp34.24 (the CD3 antibody) and 38456 (the BCMA antibody), and the exemplary antibody TS-F7-2 consisted of hz11B7.5 (the GPRC5D antibody), hzsp34.87 (the CD3 antibody) and 38456 (the BCMA antibody).

**Table 14. Activation of T cells mediated by exemplary antibodies**

| Exemplary antibodies | EC₅₀ (pM) | | | |
|---|---|---|---|---|
| | H929 | L363 | 8226 | U266 |
| TS-F7-1 (hz5E12.1.P1/SP34.24/38456) | 11.91 | 12.10 | 14.04 | 26.83 |
| TS-F7-2 (hz11B7.5/SP34.24/38456) | 4.91 | 24.77 | 19.79 | 5.68 |
| hz5E12.1-P1/SP34.24 | 6.56 | 5.59 | 3.18 | 5.70 |
| hz11B7.5/SP34.24 | 2.72 | 2.79 | 5.39 | 4.09 |
| Roche-5E11 | 16.46 | 21.92 | 5.13 | 6.37 |
| 46758 | 83.49 | 90.09 | 79.78 | 603.30 |

### TS-F7-3 and TS-F7-4

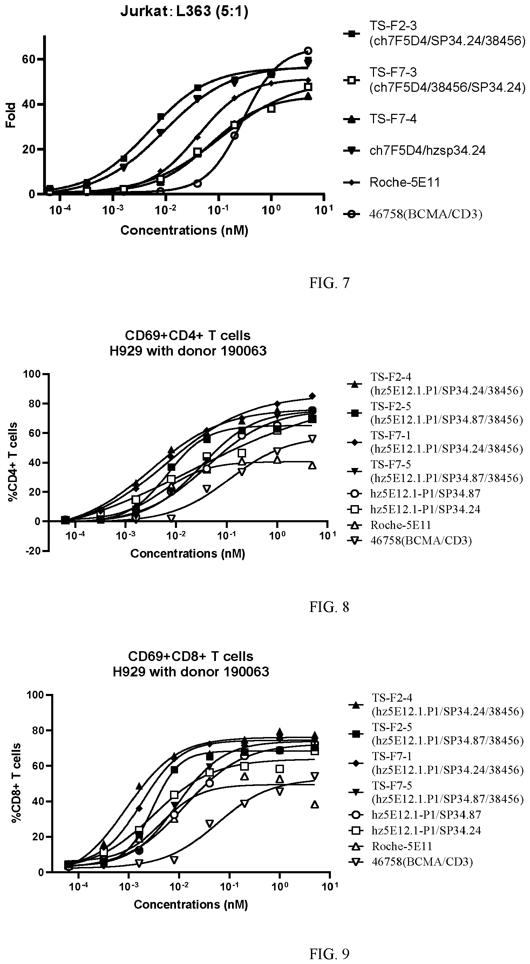

Under the condition that L363 cells and Jurkat cells (the ratio of effector cells to tumor cells was 5:1) were co-cultured for 16 h, TS-F7-3 and TS-F7-4 can significantly improve the expression of luciferase in the Jurkat cells with the increase of the antibody concentration (FIG. 7), but the agonistic activities thereof were less than that of TS-F2-3. The exemplary antibodies TS-F7-3 and TS-F7-4 consisted of ch7F5D4 (the GPRC5D antibody), hzsp34.24 (the CD3 antibody) and 38456 (the BCMA antibody); when the hzsp34.24 antibody formed a single-chain antibody, VL of TS-F7-3 was located forward and VH of TS-F7-3 was located afterward, while VH of TS-F7-4 was located forward and VL of TS-F7-4 was located afterward.

### Example 8. Experiments on Killing Effects of Anti-GPRCSD × BCMA × CD3 Antibodies on Human Multiple Myeloma Cells

The abilities of exemplary antibody-mediated T cells to kill human multiple myeloma cells were detected by the lactate dehydrogenase (LDH) method under the condition that PBMC and GPRC5D-positive and/or BCMA-positive MM cells were co-cultured. When anti-GPRC5D × BCMA × CD3 polyclonal antibodies bind to GPRC5D and/or BCMA on the surface of MM cells and also to CD3E on the surface of primary T cells, activation of T cells can be stimulated by tumor-associated antigen (GPRC5D and/or BCMA)-dependent cross-linking to T cells, thereby mediating the killing of tumor cells. The levels of various cytokines were simultaneously detected by using a multiple cytokine detection kit (Human Th1/Th2/Th17, BD); moreover, the percentage of CD69-positive cells among the T cells was detected by a flow cytometer (BD).

The following reagents and materials were used.

| Name | Cat# | Manufacturer |
|---|---|---|
| RPMI medium 1640 (1x) | 11835-030 | Gibco |
| FBS | SH30406.05 | Hyclone |
| Human Th1/Th2/Th17 Kit | 560484 | BD Biosciences |
| CytoTox 96 Non-Radio. Cytotoxicity Assay | G1781/G1782 | Promega |

### Experimental procedures:

The PBMC cells were taken out from liquid nitrogen, rapidly dissolved in a 37 °C water bath kettle, and added into 9 mL of a serum-free culture medium. The mixture was centrifuged at 300 g for 4 min, the supernatant was discarded, the resulting product was resuspended in a 10% FBS phenol red-free 1640 culture medium, and the cell density was adjusted to 4 × 10⁶ cells/mL. A certain amount of H929 cells in a logarithmic phase was collected and centrifuged, and the tumor cell density was adjusted to 2 × 10⁵ cells/mL with a 10% FBS phenol red-free 1640 culture medium. The antibodies were diluted from 5 µM to 100 nM with a 10% FBS phenol red-free 1640 culture medium according to the above-mentioned table, in a 5-fold gradient. 100 µL of the tumor cells, 50 µL of the samples subjected to gradient dilution and 50 µL of the PBMC cells were added to a 96-well cell culture plate (U-shaped). Moreover, control wells were set and supplemented to 200 µL/well by using a culture medium. The resulting mixture was cultured in a 37 °C, CO₂ incubator for 16 h. After culturing for 16 h, the cell culture plate was taken out and placed at room temperature for 5 min, 10% lysis solution was added to each TM well, and lysis was performed in the dark for 15 min. The cell culture plate was centrifuged at 400 g for 5 min, and 50 µL of the supernatant was taken and transferred to a new flat-bottom 96-well plate. 50 µL of LDH color development reagent was added, the color development was performed at room temperature in the dark for 30 min, and 50 µL of stop buffer was added. The absorbance value was read using a microplate reader, and the killing was calculated by the absorbance value. Another 90 µL of the supernatant was taken and transferred to a new 96-well plate (V-shaped) for subsequent cytokine detection. The cell culture plate was washed once by adding 200 µL of FACS buffer per well, centrifugation was performed and then the liquid was removed. 45 µL of a stain solution (prepared with FACS buffer) was added to each well, the plate was incubated at 4 °C in the dark for 20 min, and the resulting product was washed once with 200 µL of FACS buffer, resuspended with 150 µL of FACS buffer and then tested on the computer.

### CBA detection of cytokines:

A standard substance was dissolved with 2 mL of an assay diluent to obtain standard 0 (std 0), followed by 2-fold gradient dilution of eight wells std 1-std 8, wherein std 9 was an assay diluent blank control. Each bead was vortexed for 5-10 s, and 322 µL of each bead was sucked out. 2254 µL of the assay diluent was then added, and the resulting mixture was vortexed and mixed uniformly. A 96-well V-shaped plate was prepared, 50 µL of uniformly-mixed beads were added per well, 30 µL of the assay diluent was added per well, and 30 µL of samples were then added per well, that is, all samples were diluted 2-fold. A PE detection reagent was used after being diluted with the assay diluent at 1:1. 50 µL of std or the sample and the PE detection reagent were added to the wells, and the plate was sealed with a sealing film and covered with aluminum foil. The plate was oscillated on a plate oscillator at a rotation speed of 1100 rpm at room temperature for 5 min, and then incubated at room temperature for 3 h. After the incubation was completed, 120 µL of a wash buffer was added to each well, the resulting mixture was centrifuged at 300 g for 5 min, and the supernatant was discarded. The resulting product was resuspended by adding 120 µL of the wash buffer and detected by a flow cytometer.

### Experimental results:

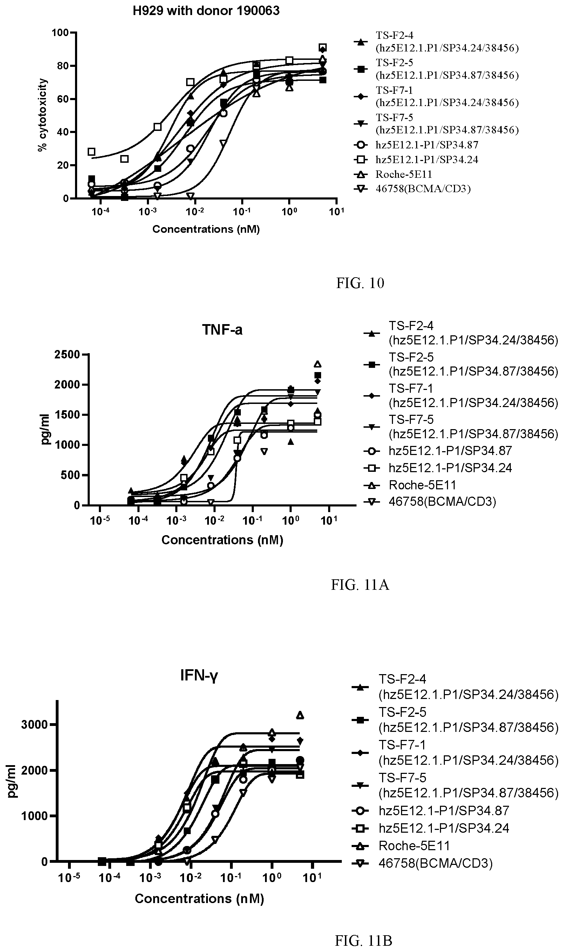

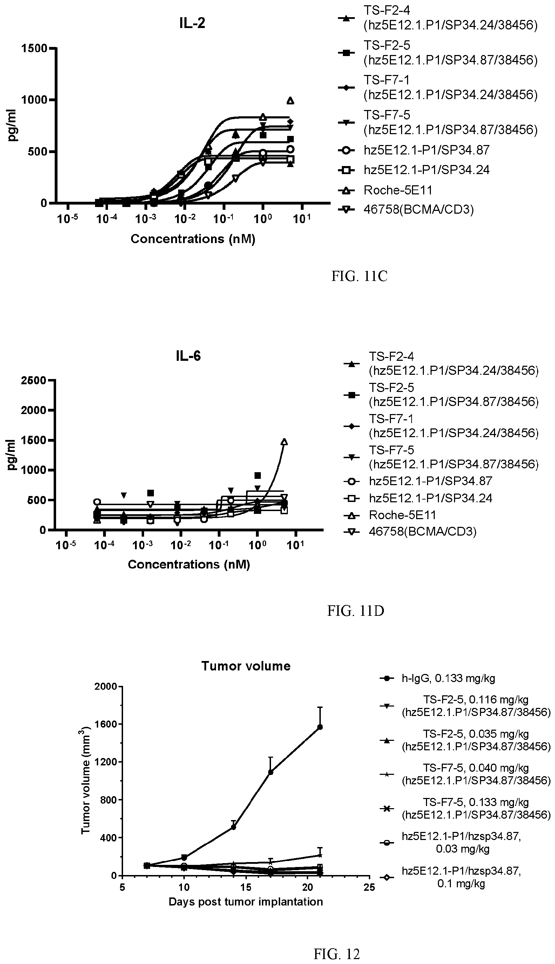

As detected by flow cytometry, it was found that primary T cells can be dose-dependently activated by the exemplary antibodies (FIGs. 8A and 8B), and there was a certain correlation between the activation degree of T cells and the affinity to CD3: the higher the anti-CD3 affinity was, the stronger the abilities to activate T cells were, thereby mediating the killing effect of PBMC on H929 cells (FIG. 9). The release levels of various cytokines accompanied during the killing process are shown in FIGs. 11A-D.

### Example 9. Anti-Tumor Effects of Exemplary Antibodies in H929 Tumor-Bearing Humanized Mouse Model

Female NOG mice (aged 35-41 days) were purchased from Beijing Vitalstar Biotechnology Co., Ltd. and were in an SPF grade. The study started after the mice were acclimated and quarantined for 7 days upon arrival.

H929 cells were subcultured conventionally for subsequent *in vivo* study. The H929 cells were collected by centrifugation and dispersed in PBS. The NOG mice were subjected to shaving on the right side of the back and abdomen and inoculated with H929 cells at 5 × 10⁶ cells/mouse with an inoculation volume of 200 µL/mouse. On day 5 after inoculation with H929 cells, the mice were intravenously injected with PBMC cells at 5 × 10⁶ cells/mouse with an inoculation volume of 200 µL/mouse.

Administration: On day 3 after inoculation with PBMC cells, the mice were divided into groups (7 mice per group) and administered according to the tumor volume of the mice. Administration was performed once every 7 days for a total of 3 times. The tumor volume and body weight of the mice were monitored twice a week. Tumor volume measurement: The maximum length of major axis (L) and maximum length of minor axis (W) of tumors were measured with a vernier caliper, and tumor volume was calculated using the following formula: V = L × W²/2. The mice were weighted using an electronic balance. Throughout the study, the mice were euthanized when the tumors reached an endpoint or when the mice lost more than 20% of body weight. The tumor size was counted.

### Experimental results

The tumor growth curves are shown in FIG. 12, and the exemplary antibodies can significantly inhibit the growth of H929 cells. Moreover, no body weight loss was found in the administered mouse groups.

### Example 10. Expression Levels of GPRC5D and BCMA on Surface of Different Multiple Myelomas

MM cells were stained with the BCMA antibody and the GPRC5D antibody with saturated concentrations by using a qifikit kit, and BCMA and GPRC5D molecules on the surface of different multiple myeloma cell strains were quantified by flow cytometry.

### Experimental reagents

| Name | Company or source | Cat# |
|---|---|---|
| BCMA (CD269) | Biolegend | 357502 |
| Mgprc5d-17G1B8 | Innoventbio | NA |
| Qifikit kit | Dako | K0078 |

1 × 10⁵ different MM cells were added to a 96-well plate, wherein GS-CHO-huGPRC5D and GS-CHO-huBCMA cells were used as positive controls and were negative controls for each other. The plate was centrifuged at 400 g for 5 min, and the supernatant was removed. 10 µL of mouse-derived primary antibodies (BCMA and Mgprc5d-17G1B8) were added, and the resulting mixture was incubated at 4 °C for 1 h. IgG1 was used as a negative control. Preparation of QIFIKIT/Beads: 100 µL of uniformly-mixed beads (Vial 1 and Vial 2) were added respectively. The resulting mixture was washed three times by adding 200 µL of FACS buffer, and the supernatant was then removed. 100 µL of secondary antibody (FITC Conjugate, Vial 3 (200 µL in total), diluted at 1:50 in 0.01 mol/L PBS) was added, and the resulting mixture was mixed uniformly and incubated at 4 °C in the dark for 45 min. The resulting product was washed three times by adding 200 µL of FACS buffer, 100 µL of PBS was then added, and the resulting mixture was mixed uniformly and detected by flow cytometry.

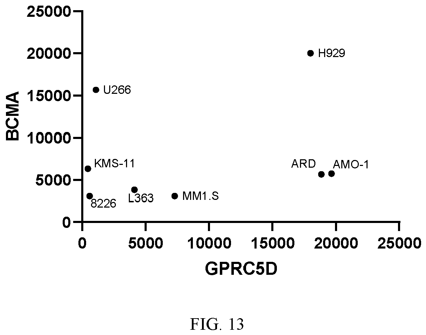

Experimental results: The expression levels of GPRC5D and BCMA were different on different surfaces of different MM cells tested, as shown in FIG. 13. As shown in FIGs. 5A-F, FIGs. 6A-D and FIG. 7, the evaluated trispecific antibodies of Format 2 and Format 7 induced advantageous biological activities on various MM cells with different GPRC5D and BCMA expression levels (H929, L363, 8226, U266, MM1.S and ARD) compared to the controls, i.e., the GPRC5D/CD3 bispecific antibody and the BCMA/CD3 bispecific antibody. This demonstrated that the trispecific antibody of the present invention can cover both patients with GPRC5D-positive tumors and patients with BCMA-positive tumors by the combined targeting against GPRC5D and BCMA, thereby improving the coverage of patients with multiple tumors. Moreover, the tumor recurrence caused by the loss of a single antigen such as BCMA can also be avoided, and the treatment effect was improved.

## Claims

1. A trispecific Y-type antibody molecule, wherein the antibody molecule has a structure of the following formula (I):
(M1:M2-(X1)p)-Fc::Fc-(X2)q, (I)
wherein,
M1 and M2 represent a first antibody arm and a second antibody arm of the antibody molecule, respectively, and M1 and M2 comprise antigen-binding sites that bind to a first antigen and a second antigen, respectively, the antigen-binding site being Fab or scFab, preferably Fab;
Fc::Fc represents the stem of the antibody molecule, consisting of a first Fc domain and a second Fc domain which are paired and dimerized, wherein the first and second antibody arms are linked to the N-termini of the first Fc domain and the second Fc domain, either directly or via a linker peptide (preferably a hinge region), respectively;
X1 represents a conjugate component conjugated to the C-terminus of each antibody arm, and X2 represents a conjugate component conjugated to the C-terminus of the stem, wherein the conjugate component comprises an antigen-binding site that binds to a third antigen; p and q each represent an integer of 0, 1, or 2, and one of p and q is 0;
preferably, when p = 0, q = 1, the antigen-binding sites of M1 and M2 are each Fab or scFab, and the conjugate component X2 is conjugated to one of the two Fc domains of the stem of the antibody molecule, or
when q = 0, p = 2, the antigen-binding sites of M1 and M2 are each Fab, and the conjugate component X1 is conjugated to Fab light chains of both the antibody arms M1 and M2 of the antibody molecule; and
the first, the second, and the third antigens are different from each other and are independently selected from GPRC5D, BCMA, and CD3.

2. The antibody molecule according to claim 1, wherein the conjugate component comprises an antigen-binding site selected from scFv, Fv, dsFv, and dsAb, preferably scFv.

3. The antibody molecule according to claim 1, wherein the antibody molecule has a structure:
(M1:M2)-Fc::Fc-(X2)q, (Format_2)
wherein,
q = 1,
M1 and M2 comprise an antigen-binding site Fab or scFab, preferably Fab, that binds to the first antigen and the second antigen, respectively;
X2 comprises an antigen-binding site scFv, preferably dsscFv, that binds to the third antigen; and
the first, the second, and the third antigens are different from each other and are independently selected from GPRC5D, BCMA, and CD3.

4. The antibody molecule according to claim 1, wherein the antibody molecule has a structure:
(M1:M2-(X1)p)-Fc::Fc, (Format_7)
wherein,
P = 2,
M1 and M2 comprise an antigen-binding site Fab that binds to the first antigen and the second antigen, respectively;
X1 comprises an antigen-binding site scFv, preferably dsscFv, that binds to the third antigen; and
the first, the second, and the third antigens are different from each other and are independently selected from GPRC5D, BCMA, and CD3.

5. The antibody molecule according to any one of claims 1-4, wherein the Fab antigen-binding site of the antibody arm comprises two chains, wherein one chain comprises, from N-terminus to C-terminus, VH-CH1, and the other chain comprises, from N-terminus to C-terminus, VL-CL; or one chain comprises, from the N-terminus to the C-terminus, VH-CL, and the other chain comprises, from the N-terminus to the C-terminus, VL-CH1.

6. The antibody molecule according to any one of claims 1-5, wherein the scFv contained in the conjugate component comprises, from N-terminus to C-terminus, VH-linker-VL or VL-linker-CH.

7. The antibody molecule according to any one of claims 1-6, wherein the first Fc domain and the second Fc domain are Fc domains derived from IgG1, IgG2, or IgG4 immunoglobulin, preferably Fc domains derived from IgG1 immunoglobulin, and more preferably Fc domains derived from human IgG1 immunoglobulin.

8. The antibody molecule according to any one of claims 1-7, wherein the antigen-binding site that binds to GPRC5D comprises VH and VL selected from the following groups:
(a) VH comprising HCDR1-3 sequences set forth in SEQ ID NOs: 1-3 and VL comprising LCDR1-3 sequences set forth in SEQ ID NOs: 4-6;
(b) VH comprising HCDR1-3 sequences set forth in SEQ ID NOs: 7-8 and VL comprising LCDR1-3 sequences set forth in SEQ ID NOs: 10-12;
(c) VH comprising HCDR1-3 sequences set forth in SEQ ID NOs: 13-15 and VL comprising LCDR1-3 sequences set forth in SEQ ID NOs: 16-18; or
(d) VH comprising HCDR1-3 sequences set forth in SEQ ID NOs: 19-21 and VL comprising LCDR1-3 sequences set forth in SEQ ID NOs: 22-24;
preferably, the antigen-binding site comprises a VH and VL amino acid sequence pair selected from the following groups: SEQ ID NOs: 25/26, SEQ ID NOs: 27/28, SEQ ID NOs: 29/30, and SEQ ID NOs: 31/32, and preferably, when the antigen-binding site is dsscFv, the antigen-binding site further comprises cysteine replacements introduced into the VH and VL sequences, such as cysteine replacements at position 44 in the VH sequence and position 100 in the VL sequence.

9. The antibody molecule according to any one of claims 1-8, wherein the antigen-binding site that binds to CD3 comprises VH and VL selected from the following groups:
(a) VH comprising HCDR1-3 sequences set forth in SEQ ID NOs: 41-43 and VL comprising LCDR1-3 sequences set forth in SEQ ID NOs: 44-46; or
(b) VH comprising HCDR1-3 sequences set forth in SEQ ID NOs: 47, 42, and 43, and VL comprising LCDR1-3 sequences set forth in SEQ ID NOs: 44-46;
preferably, the antigen-binding site comprises a VH and VL amino acid sequence pair selected from the following groups: SEQ ID NOs: 48/49 and SEQ ID NOs: 50/49, and more preferably, SEQ ID NOs: 48/49, and preferably, when the antigen-binding site is dsscFv, the antigen-binding site further comprises cysteine replacements introduced into the VH and VL sequences, such as cysteine replacements at position 44 in the VH sequence and position 100 in the VL sequence.

10. The antibody molecule according to any one of claims 1-9, wherein the antigen-binding site that binds to BCMA comprises the following VH and VL:
VH comprising HCDR1-3 sequences set forth in SEQ ID NOs: 33-35 and VL comprising LCDR1-3 sequences set forth in SEQ ID NOs: 36-38;
preferably, the antigen-binding site comprises a VH and VL amino acid sequence pair: SEQ ID NOs: 39/40, and preferably, when the antigen-binding site is dsscFv, the antigen-binding site further comprises cysteine replacements introduced into the VH and VL sequences, such as cysteine replacements at position 44 in the VH sequence and position 100 in the VL sequence.

11. The antibody molecule according to claim 3, wherein the antibody molecule comprises or consists of a first heavy chain polypeptide chain, a first light chain polypeptide chain, a second heavy chain polypeptide chain, and a second light chain polypeptide chain, wherein
the first heavy chain polypeptide chain comprises a heavy chain variable domain VH, an immunoglobulin CH1 domain and an Fc domain from the N-terminus to the C-terminus;
the first light chain polypeptide chain comprises a light chain variable domain VL and an immunoglobulin CL domain from the N-terminus to the C-terminus;
the second heavy chain polypeptide chain comprises a heavy chain variable domain VH, an immunoglobulin CH1 domain and an Fc domain from the N-terminus to the C-terminus; and
the second light chain polypeptide chain comprises a light chain variable domain VL and an immunoglobulin CL domain from the N-terminus to the C-terminus;
and wherein the first heavy chain polypeptide chain or the second heavy chain polypeptide chain further comprises an scFv domain conjugated to the C-terminus of an Fc domain thereof, either directly or preferably via a linker peptide (e.g., (G4S)2 or TS(G4S)2);
wherein,
VH-CH1 of the first heavy chain polypeptide chain pair and VL-CL of the first light chain polypeptide chain are paired to form a first antibody arm (M1) that binds to a first antigen;
VH-CH1 of the second heavy chain polypeptide chain and VL-CL of the second light chain polypeptide chain are paired to form a second antibody arm (M2) that binds to a second antigen;
the Fc domain of the first heavy chain polypeptide chain and the Fc domain of the second heavy chain polypeptide chain are paired and dimerized to form a stem of the antibody (Fc::Fc); and
the scFv domain conjugated to the C-terminus of the first heavy chain polypeptide chain or the second heavy chain polypeptide chain forms a conjugate component (X2) that binds to a third antigen,
wherein the first, second and third antigens are different and independently selected from: GPRC5D, BCMA, and CD3.

12. The antibody molecule according to claim 4, wherein the antibody molecule comprises or consists of a first heavy chain polypeptide chain, a first light chain polypeptide chain, a second heavy chain polypeptide chain, and a second light chain polypeptide chain, wherein
the first heavy chain polypeptide chain comprises a heavy chain variable domain VH, an immunoglobulin CH1 domain and an Fc domain from the N-terminus to the C-terminus;
the first light chain polypeptide chain comprises a light chain variable domain VL and an immunoglobulin CL domain from the N-terminus to the C-terminus;
the second heavy chain polypeptide chain comprises a heavy chain variable domain VH, an immunoglobulin CH1 domain and an Fc domain from the N-terminus to the C-terminus; and
the second light chain polypeptide chain comprises a light chain variable domain VL and an immunoglobulin CL domain from the N-terminus to the C-terminus;
and wherein the first light chain polypeptide chain and the second light chain polypeptide chain further comprise scFv domains conjugated to the C-termini of the CL domains thereof, either directly or preferably via a linker peptide (e.g., (G4S)2 or TS(G4S)2);
wherein,
VH-CH1 of the first heavy chain polypeptide chain pair and VL-CL of the first light chain polypeptide chain are paired to form a first antibody arm (M1) that binds to a first antigen;
VH-CH1 of the second heavy chain polypeptide chain and VL-CL of the second light chain polypeptide chain are paired to form a second antibody arm (M2) that binds to a second antigen;
the Fc domain of the first heavy chain polypeptide chain and the Fc domain of the second heavy chain polypeptide chain are paired and dimerized to form a stem of the antibody (Fc::Fc); and
the scFv domain conjugated to the C-termini of the first light chain polypeptide chain and the second light chain polypeptide chain form a conjugate component (X1) that binds to a third antigen,
wherein the first, second and third antigens are different and independently selected from: GPRC5D, BCMA, and CD3.

13. The antibody molecule according to claim 11 or 12, wherein
(a) the antibody arm M1 and the antibody arm M2 of the antibody molecule bind to GPRC5D and CD3, respectively, and the conjugate component binds to BCMA; or
(b) the antibody arm M1 and the antibody arm M2 of the antibody molecule bind to BCMA and CD3, respectively, and the conjugate component binds to GPRC5D; or
(c) the antibody arm M1 and the antibody arm M2 of the antibody molecule bind to GPRC5D and BCMA, respectively, and the conjugate component binds to CD3.

14. The antibody molecule according to claim 11, wherein the antibody molecule comprises or consists of a first heavy chain polypeptide chain, a first light chain polypeptide chain, a second heavy chain polypeptide chain, and a second light chain polypeptide chain, wherein
(a) the first heavy chain polypeptide chain comprises an amino acid sequence set forth in SEQ ID NO: 65 or an amino acid sequence having at least 90%, 92%, 95%, 97%, 98%, 99% or higher identity thereto; the first light chain polypeptide chain comprises an amino acid sequence set forth in SEQ ID NO: 66 or an amino acid sequence having at least 90%, 92%, 95%, 97%, 98%, 99% or higher identity thereto; the second heavy chain polypeptide chain comprises an amino acid sequence set forth in SEQ ID NO: 67 or an amino acid sequence having at least 90%, 92%, 95%, 97%, 98%, 99% or higher identity thereto; and the second light chain polypeptide chain comprises an amino acid sequence set forth in SEQ ID NO: 68 or an amino acid sequence having at least 90%, 92%, 95%, 97%, 98%, 99% or higher identity thereto; or
(b) the first heavy chain polypeptide chain comprises an amino acid sequence set forth in SEQ ID NO: 69 or an amino acid sequence having at least 90%, 92%, 95%, 97%, 98%, 99% or higher identity thereto; the first light chain polypeptide chain comprises an amino acid sequence set forth in SEQ ID NO: 70 or an amino acid sequence having at least 90%, 92%, 95%, 97%, 98%, 99% or higher identity thereto; the second heavy chain polypeptide chain comprises an amino acid sequence set forth in SEQ ID NO: 71 or an amino acid sequence having at least 90%, 92%, 95%, 97%, 98%, 99% or higher identity thereto; and the second light chain polypeptide chain comprises an amino acid sequence set forth in SEQ ID NO: 68 or an amino acid sequence having at least 90%, 92%, 95%, 97%, 98%, 99% or higher identity thereto; or
(c) the first heavy chain polypeptide chain comprises an amino acid sequence set forth in SEQ ID NO: 72 or an amino acid sequence having at least 90%, 92%, 95%, 97%, 98%, 99% or higher identity thereto; the first light chain polypeptide chain comprises an amino acid sequence set forth in SEQ ID NO: 73 or an amino acid sequence having at least 90%, 92%, 95%, 97%, 98%, 99% or higher identity thereto; the second heavy chain polypeptide chain comprises an amino acid sequence set forth in SEQ ID NO: 74 or an amino acid sequence having at least 90%, 92%, 95%, 97%, 98%, 99% or higher identity thereto; and the second light chain polypeptide chain comprises an amino acid sequence set forth in SEQ ID NO: 68 or an amino acid sequence having at least 90%, 92%, 95%, 97%, 98%, 99% or higher identity thereto; or
(d) the first heavy chain polypeptide chain comprises an amino acid sequence set forth in SEQ ID NO: 75 or an amino acid sequence having at least 90%, 92%, 95%, 97%, 98%, 99% or higher identity thereto; the first light chain polypeptide chain comprises an amino acid sequence set forth in SEQ ID NO: 76 or an amino acid sequence having at least 90%, 92%, 95%, 97%, 98%, 99% or higher identity thereto; the second heavy chain polypeptide chain comprises an amino acid sequence set forth in SEQ ID NO: 77 or 78 or an amino acid sequence having at least 90%, 92%, 95%, 97%, 98%, 99% or higher identity thereto; and the second light chain polypeptide chain comprises an amino acid sequence set forth in SEQ ID NO: 68 or an amino acid sequence having at least 90%, 92%, 95%, 97%, 98%, 99% or higher identity thereto; or
(e) the first heavy chain polypeptide chain comprises an amino acid sequence set forth in SEQ ID NO: 79 or an amino acid sequence having at least 90%, 92%, 95%, 97%, 98%, 99% or higher identity thereto; the first light chain polypeptide chain comprises an amino acid sequence set forth in SEQ ID NO: 80 or an amino acid sequence having at least 90%, 92%, 95%, 97%, 98%, 99% or higher identity thereto; the second heavy chain polypeptide chain comprises an amino acid sequence set forth in SEQ ID NO: 77 or an amino acid sequence having at least 90%, 92%, 95%, 97%, 98%, 99% or higher identity thereto; and the second light chain polypeptide chain comprises an amino acid sequence set forth in SEQ ID NO: 68 or an amino acid sequence having at least 90%, 92%, 95%, 97%, 98%, 99% or higher identity thereto.

15. The antibody molecule according to claim 12, wherein the antibody molecule comprises or consists of a first heavy chain polypeptide chain, a first light chain polypeptide chain, a second heavy chain polypeptide chain, and a second light chain polypeptide chain, wherein
(a) the first heavy chain polypeptide chain comprises an amino acid sequence set forth in SEQ ID NO: 87 or an amino acid sequence having at least 90%, 92%, 95%, 97%, 98%, 99% or higher identity thereto; the first light chain polypeptide chain comprises an amino acid sequence set forth in SEQ ID NO: 88 or an amino acid sequence having at least 90%, 92%, 95%, 97%, 98%, 99% or higher identity thereto; the second heavy chain polypeptide chain comprises an amino acid sequence set forth in SEQ ID NO: 77 or 78 or an amino acid sequence having at least 90%, 92%, 95%, 97%, 98%, 99% or higher identity thereto; and the second light chain polypeptide chain comprises an amino acid sequence set forth in SEQ ID NO: 89 or an amino acid sequence having at least 90%, 92%, 95%, 97%, 98%, 99% or higher identity thereto; or
(b) the first heavy chain polypeptide chain comprises an amino acid sequence set forth in SEQ ID NO: 90 or an amino acid sequence having at least 90%, 92%, 95%, 97%, 98%, 99% or higher identity thereto; the first light chain polypeptide chain comprises an amino acid sequence set forth in SEQ ID NO: 91 or an amino acid sequence having at least 90%, 92%, 95%, 97%, 98%, 99% or higher identity thereto; the second heavy chain polypeptide chain comprises an amino acid sequence set forth in SEQ ID NO: 77 or an amino acid sequence having at least 90%, 92%, 95%, 97%, 98%, 99% or higher identity thereto; and the second light chain polypeptide chain comprises an amino acid sequence set forth in SEQ ID NO: 89 or an amino acid sequence having at least 90%, 92%, 95%, 97%, 98%, 99% or higher identity thereto; or
(c) the first heavy chain polypeptide chain comprises an amino acid sequence set forth in SEQ ID NO: 92 or an amino acid sequence having at least 90%, 92%, 95%, 97%, 98%, 99% or higher identity thereto; the first light chain polypeptide chain comprises an amino acid sequence set forth in SEQ ID NO: 93 or 96 or an amino acid sequence having at least 90%, 92%, 95%, 97%, 98%, 99% or higher identity thereto; the second heavy chain polypeptide chain comprises an amino acid sequence set forth in SEQ ID NO: 94 or an amino acid sequence having at least 90%, 92%, 95%, 97%, 98%, 99% or higher identity thereto; and the second light chain polypeptide chain comprises an amino acid sequence set forth in SEQ ID NO: 95 or 97 or an amino acid sequence having at least 90%, 92%, 95%, 97%, 98%, 99% or higher identity thereto.

16. An antibody or an antigen-binding fragment thereof that specifically binds to GPRC5D, wherein the antibody or the antigen-binding fragment thereof comprises a combination of CDR sequences selected from the followings:
(i) HCDR1, 2, and 3 sequences of a heavy chain variable domain and LCDR1, 2, and 3 sequences of a light chain variable domain set forth in SEQ ID NOs: 1-6, respectively, or
(ii) HCDR1, 2, and 3 sequences of a heavy chain variable domain and LCDR1, 2, and 3 sequences of a light chain variable domain set forth in SEQ ID NOs: 7-12, respectively, or
(iii) HCDR1, 2, and 3 sequences of a heavy chain variable domain and LCDR1, 2, and 3 sequences of a light chain variable domain set forth in SEQ ID NOs: 13-18, respectively, or
(iv) HCDR1, 2, and 3 sequences of a heavy chain variable domain and LCDR1, 2, and 3 sequences of a light chain variable domain set forth in SEQ ID NOs: 19-24, respectively, or
(v) HCDR1, 2, and 3 sequences of a heavy chain variable domain and LCDR1, 2, and 3 sequences of a light chain variable domain set forth in SEQ ID NOs: 13, 110, and 15-18, respectively.

17. The antibody according to claim 16, wherein the antibody comprises a combination of VH and VL amino acid sequences selected from the followings:
(a) a heavy chain variable domain comprising an amino acid sequence set forth in SEQ ID NO: 25 or an amino acid sequence having at least 80%, 85%, 90%, 95%, 97%, 98%, or 99% sequence identity thereto; and a light chain variable domain comprising an amino acid sequence set forth in SEQ ID NO: 26 or an amino acid sequence having at least 80%, 85%, 90%, 95%, 97%, 98%, or 99% sequence identity thereto; or
(b) a heavy chain variable domain comprising an amino acid sequence set forth in SEQ ID NO: 27 or an amino acid sequence having at least 80%, 85%, 90%, 95%, 97%, 98%, or 99% sequence identity thereto; and a light chain variable domain comprising an amino acid sequence set forth in SEQ ID NO: 28 or an amino acid sequence having at least 80%, 85%, 90%, 95%, 97%, 98%, or 99% sequence identity thereto; or
(c) a heavy chain variable domain comprising an amino acid sequence set forth in SEQ ID NO: 29 or an amino acid sequence having at least 80%, 85%, 90%, 95%, 97%, 98%, or 99% sequence identity thereto; and a light chain variable domain comprising an amino acid sequence set forth in SEQ ID NO: 30 or an amino acid sequence having at least 80%, 85%, 90%, 95%, 97%, 98%, or 99% sequence identity thereto; or
(d) a heavy chain variable domain comprising an amino acid sequence set forth in SEQ ID NO: 31 or an amino acid sequence having at least 80%, 85%, 90%, 95%, 97%, 98%, or 99% sequence identity thereto; and a light chain variable domain comprising an amino acid sequence set forth in SEQ ID NO: 32 or an amino acid sequence having at least 80%, 85%, 90%, 95%, 97%, 98%, or 99% sequence identity thereto;
(e) a heavy chain variable domain comprising an amino acid sequence set forth in SEQ ID NO: 98 or an amino acid sequence having at least 80%, 85%, 90%, 95%, 97%, 98%, or 99% sequence identity thereto; and a light chain variable domain comprising an amino acid sequence set forth in SEQ ID NO: 99 or an amino acid sequence having at least 80%, 85%, 90%, 95%, 97%, 98%, or 99% sequence identity thereto; or
(f) a heavy chain variable domain comprising an amino acid sequence set forth in SEQ ID NO: 100 or an amino acid sequence having at least 80%, 85%, 90%, 95%, 97%, 98% or 99% sequence identity thereto; and a light chain variable domain comprising an amino acid sequence set forth in SEQ ID NO: 101 or an amino acid sequence having at least 80%, 85%, 90%, 95%, 97%, 98% or 99% sequence identity thereto; optionally, the VH and VL sequences comprise cysteine replacements introduced, for example, cysteine replacements at position 44 in the VH sequence and position 100 in the VL sequence, and therefore a disulfide bond is formed between the VH and the VL.

18. The antibody according to claim 16 or 17, wherein the antibody is a monospecific, bispecific, or trispecific antibody; preferably, the antibody further comprises an antigen-binding site that binds to CD3, or further comprises an antigen-binding site that binds to BCMA and an antigen-binding site that binds to CD3.

19. The antibody according to claim 18, wherein the antibody is a bispecific antibody that binds to GPRC5D and CD3;
preferably, the antibody comprises a VH and VL pair that binds to CD3 and is selected from the following groups:
(a) VH comprising HCDR1-3 sequences set forth in SEQ ID NOs: 41-43 and VL comprising LCDR1-3 sequences set forth in SEQ ID NOs: 44-46; or
(b) VH comprising HCDR1-3 sequences set forth in SEQ ID NOs: 47, 42, and 43, and VL comprising LCDR1-3 sequences set forth in SEQ ID NOs: 44-46;
preferably, a VH and VL amino acid sequence pair selected from the following groups: SEQ ID NOs: 48/49 and SEQ ID NOs: 50/49,
optionally, the VH and VL sequences comprise cysteine replacements introduced, for example, cysteine replacements at position 44 in the VH sequence and position 100 in the VL sequence, and therefore a disulfide bond is formed between the VH and the VL.

20. A polynucleotide, encoding the antibody molecule according to any one of claims 1-19.

21. A vector, preferably an expression vector, comprising the polynucleotide according to claim 20.

22. A host cell, comprising the polynucleotide according to claim 20 or the vector according to claim 21, wherein, for example, the host cell is a mammalian cell.

23. A method for producing the antibody molecule according to any one of claims 1-19, wherein the method comprises culturing a host cell comprising a polynucleotide encoding a polypeptide chain under conditions suitable for the expression of the polypeptide chain of the antibody; and assembling the polypeptide chain to produce the antibody under conditions suitable for the assembly of the polypeptide chain into the antibody molecule; preferably,
the method comprises the steps of: (i) culturing a host cell comprising a polynucleotide encoding a first heavy chain and a first light chain under conditions suitable for the expression of the first heavy chain polypeptide chain and first light chain polypeptide chain of the antibody molecule to produce a first parent protein; (ii) culturing a host cell comprising a polynucleotide encoding a second heavy chain and a second light chain under conditions suitable for the expression of the second heavy chain polypeptide chain and the second light chain polypeptide chain of the antibody molecule to produce a second parent protein; and (iii) mixing the first parent protein and the second parent protein at an equimolar ratio, and placing the mixture under suitable redox conditions *in vitro* to make the mixture assembled into the antibody.

24. A pharmaceutical composition, comprising the antibody molecule according to any one of claims 1-19 and a pharmaceutically acceptable carrier.

25. Use of the antibody molecule according to any one of claims 1-19 and the pharmaceutical composition according to claim 24 for, *in vivo* and/or *in vitro,*
- binding to a GPRC5D antigen, including a GPRC5D antigen expressed on the surfaces of cells, with high affinity, e.g., with KD less than 10 nM;
- targeting T cells (e.g., CD4+ and/or CD8+ T cells) to cells that express GPRC5D on the surfaces, in particular GPRC5D-positive tumor cells;
- targeting T cells (e.g., CD4+ and/or CD8+ T cells) to cells that express BCMA on the surfaces, in particular BCMA-positive tumor cells;
- activating the CD3 downstream signaling pathways of T cells;
- mediating the killing effects of T cells on GPRC5D-positive and/or BCMA-positive tumor cells;
- inducing T cells to release cytokines such as TNF-α, IFN-γ and IL-2;
- inhibiting or killing GPRC5D-positive and/or BCMA-positive tumor cells, or
- treating GPRC5D-positive and/or BCMA-positive multiple myeloma, e.g., treating a BCMA-positive patient population, a GPRC5D-positive patient population, or a GPRC5D-positive patient population in which BCMA is lost after the administration of an anti-BCMA molecule.

26. The use according to claim 25, wherein the antibody molecule or the pharmaceutical composition is used as a drug for treating and/or preventing a disease in an individual or as a diagnostic tool for a disease; preferably, the individual is a mammal, and more preferably a human.
